# FASCICULE DE BREVET EUROPEEN

(11) **EP 1 851 331 B1**
(45) Date de publication et mention de la délivrance du brevet: **24.02.2016**
(21) Numéro de dépôt: 06709377.3
(22) Date de dépôt: 24.02.2006
(51) Int. Cl.: C12Q 1/68, B01J 19/00, G01N 33/543

(54) **PROCEDE ET DISPOSITIF POUR SEPARER DES CIBLES MOLECULAIRES DANS UN MELANGE COMPLEXE**
VERFAHREN UND VORRICHTUNG ZUR TRENNUNG MOLEKULARER ZIELE IN EINEM KOMPLEXEN GEMISCH
METHOD AND DEVICE FOR SEPARATING MOLECULAR TARGETS IN A COMPLEX MIXTURE

(30) Priorité: 25.02.2005 FR 0501962; 28.02.2005 FR 0502027
(43) Date de publication de la demande: 07.11.2007
(73) Titulaire: Commissariat à l'Énergie Atomique et aux Énergies Alternatives, 75015 Paris (FR)
(72) Inventeur: UGOLIN, Nicolas, F-75002 Paris (FR); CHEVILLARD, Sylvie, F-94270 Le Kremlin-Bicetre (FR); COUTANT, Alexandre, F-92130 Issy-les-Moulineaux (FR); LEBEAU, Jérôme, F-75001 Paris (FR)
(74) Mandataire: Desaix, Anne
(86) Numéro de dépôt international: PCT/FR2006/000428
(87) Numéro de publication internationale: WO 2006/090073

(56) Documents cités:
- US-A- 5 665 539
- US-A- 5 846 708
- US-A1- 2003 036 064
- US-A1- 2003 148 335
- US-A1- 2005 003 360
- CUZIN M: "DNA chips: a new tool for genetic analysis and diagnostics." TRANSFUSION CLINIQUE ET BIOLOGIQUE : JOURNAL DE LA SOCIETE FRANCAISE DE TRANSFUSION SANGUINE. JUN 2001, vol. 8, no. 3, juin 2001 (2001-06), pages 291-296, XP002356228 ISSN: 1246-7820

## Description

L'invention porte sur un procédé et un dispositif permettant d'analyser des cibles moléculaires dans un mélange complexe.

La demande de brevet US-A1-2003/0036064 décrit un procédé d'analyse d'acides nucléiques qui sont immobilisés sur un support. Le nombre d'acides nucléiques pouvant être identifiés par ce procédé correspond au nombre d'acides nucléiques pouvant être immobilisés sur le support, qui est relativement faible.

L'invention permet en particulier la séparation et l'analyse quantitative des cibles moléculaires dans un mélange complexe.

L'invention a notamment pour objet des moyens permettant de remédier, au moins en partie, aux difficultés rencontrées jusqu'à présent pour réaliser la séparation et l'analyse de cibles moléculaires au moyen de matrices organisées de sondes de bio-polymères, connus dans l'art antérieur.

Les matrices organisées de sondes de bio-polymères (puces à ADN, puces à protéines, etc.) permettent de séparer de manière qualitative et quantitative les biopolymères (cibles moléculaires) présents dans un mélange et cela théoriquement, quels que soient leur nombre, leur séquence et leur complexité. Cependant, les réseaux d'acides nucléiques ne permettent pas de compter de manière absolue et précise le nombre de molécules cibles hybridées aux sondes. Avec la technologie disponible actuellement, la détection des bio-polymères sur biopuces (« micro-arrays ») est indirecte, nécessitant une étape de marquage de ceux-ci (fluorescence, radioactivité...). A titre d'exemple, la méthode par « marqueurs fluorescents » mesure l'intensité de fluorescence apportée par les marqueurs fluorescents accrochés aux molécules à analyser.

Lorsqu'une molécule s'hybride, elle engendre une augmentation de la fluorescence qui est proportionnelle au nombre de complexes sondes-cibles formés sur une bio-puce. Les mesures indirectes par le biais de marqueurs sont toutefois d'une fiabilité relative en particulier lorsque les cibles moléculaires forment une quantité faible de matériel biologique à analyser ou ces mesures présentent des inconvénients en vue d'une utilisation dans des analyses en routine.

Si les rendements d'incorporation dans les biopolymères de résidus radiomarqués et de résidus froids sont quasiment les mêmes, il n'en va pas de même avec les marqueurs fluorescents (Martinez *et al. -* Nucl. Acids. Res. 2003 31: p.18 ; Hoen *et al. -* Nucleic Acids Res. 2003 Mar 1;31 (5) ; p. 20.). Ces problèmes de marquages sont notamment rencontrés pour la synthèse des molécules d'ADNc incorporant les marqueurs fluorescents CY3 et CY5. L'encombrement stérique résultant de ce dernier type de marquage peut en outre fortement modifier les cinétiques et les équilibres stoechiométriques des réactions (hybridation, réaction anticorps-antigène, réaction cible-ligand en général...).

Ces problèmes d'encombrements stériques sont éliminés avec l'emploi d'isotopes radioactifs; toutefois les isotopes radioactifs impliquent une gestion des déchets radioactifs,en plus des contraintes liées aux matériels utilisés et de la protection des personnes. En outre, les technologies de détection de molécules marquées radioactivement, c'est-à-dire essentiellement de type « Phosphoimager » pour la radioactivité (Bertucci F et al. - Hum Mol Genet. 1999 Sep; 8(9): 1715-22. Erratum in: Hum Mol Genet 1999 Oct;8(11) ; p. 2129.), et les différents types de scanner pour la détection de la fluorescence présentent un certain nombre de limites quant à la quantité de matériel biologique à hybrider sur une puce pour atteindre les seuils de détection et de reproductibilité des mesures effectuées. De fait, il n'est pas possible de détecter des molécules présentes à quelques copies par cellule à partir d'échantillons présentant un faible nombre de cellules (∼1 000 cellules), ce qui correspond néanmoins à une situation fréquente pour les prélèvements cliniques.

Pour surmonter les difficultés tenant à la nécessité de recourir au marquage actif tel qu'évoqué ci-dessus, des sondes bio-polymères pour, indirectement, détecter lesdites sondes, d'autres modes de détection des complexes sonde-cible formés ont été mis au point.

Ainsi, on a proposé d'utiliser les propriétés de conductance électrique des biopolymères. Une molécule d'ADN simple brin d'une séquence donnée n'a pas, en effet, la même impédance que le complexe apparié double brin correspondant. Cette propriété est utilisée sur les puces à ADN pour évaluer la proportion d'hybridation, donc le nombre de complexes sonde-cible formés sur une bio-puce. D'une manière générale, les variations d'impédance peuvent êtres utilisées pour étudier les interactions inter-moléculaires, telles que la fixation d'un ligand sur son récepteur, mais aussi les interactions entre des molécules d'ADN ou de protéines et une drogue, un ion... Toutefois pour les bio-puces, cette méthode de détection est limitée :
1) Par la difficulté à réaliser des puces à haute densité de plus de 2000 spots. En effet, à cause de la taille des électrodes et de la géométrie de la connectique utilisée pour réaliser les puces à impédance, la surface d'hybridation devient très grande dès que le nombre de spots dépasse 800. Or une grande surface d'hybridation implique un volume d'hybridation important, d'où la nécessité d'une grande quantité de matériel biologique pour atteindre la concentration minimale pour la détection. Ceci est incompatible avec les expériences où peu de matériel est disponible par exemple pour les diagnostics.
2) Par les changements de conformation des molécules étudiées (sonde et/ou cible) qui entraînent des artéfacts de mesure rendant ininterprétables les variations d'impédance mesurées. Par exemple, les déformations de l'ADN dues à la séquence où les hybridations intra-moléculaires provoquent des variations d'impédance du même ordre de grandeur que pour l'hybridation inter-moléculaire.
3) Par les variations d'impédance dues à la taille des molécules à analyser. Par exemple, les molécules d'acides nucléiques, représentant un transcriptome, ont des tailles différentes, en raison :
   - De l'avancée hétérogène de la transcription d'un gène à l'autre à un instant donné,
   - De la longueur différente des gènes,
   - Des épissages différents que subissent les transcrits d'un même gène.

Le signal électrique mesuré au niveau d'un spot d'une puce résulte donc de l'hybridation d'un mélange hétérogène en taille des transcrits d'un gène. Ce signal n'est comparable, ni à celui obtenu pour le même gène en hybridant un autre extrait cellulaire, ni à celui obtenu pour un autre gène sur la même puce.

Dans ces conditions, la mesure de l'impédance est également rendue difficile par les contraintes suivantes. Des transistors à effet de champ sont utilisés comme amplificateur de courant et / ou de tension pour mesurer la variation d'impédance liée à l'hybridation de la molécule d'ADN. Le greffage des sondes est réalisé au niveau de la grille du transistor. Lorsque les cibles viennent s'y hybrider, elles modifient l'impédance de la grille ce qui entraîne une modification du courant et de la tension entre la source (entrée du transistor) et le drain (sortie) du transistor. Aucune organisation en réseau n'a été décrite pour ce mode de détection. Le fait d'utiliser le transistor à effet de champ comme amplificateur de courant en déposant les sondes au niveau de la grille, limite l'utilisation de celui-ci.

En effet, la grille d'un transistor à effet de champ ne peut être soumise à un courant électrique. Seule une tension électrique peut lui être appliquée qui permet de contrôler l'ouverture du passage source/drain. Or pour mesurer directement efficacement l'impédance d'un oligonucléotide il est nécessaire de le soumettre directement à des tensions et/ou des courants alternatifs de différentes fréquences. De plus, la fragilité de la grille d'un transistor à effet de champ, rend très difficile sa protection vis à vis de l'électricité statique produite lors du dépôt des sondes.

La présence des sondes sur la grille interdit également de pouvoir utiliser les transistors comme des interrupteurs dans un multiplexeur, pour contrôler le passage des courants et les tensions au niveau de chaque spot. De même dans cette configuration, il n'est pas possible d'utiliser les sources et les drains des transistors comme électrode d'électrophorèse pour contrôler le déplacement des molécules cibles sur le support d'hybridation, afin de déplacer et concentrer les cibles au niveau de chaque spot.

En l'état actuel de la technique, la mesure de l'impédance électrique des acides nucléiques ne permet pas de quantifier ou d'analyser les concentrations ou les proportions d'une population hétérogène de molécules constituant un mélange complexe d'acides nucléiques.

Une autre méthode de détection utilisée dans le domaine est la spectrométrie de masse. On sait déterminer la masse des macromolécules telles que l'ADN, l'ARN ou les protéines, à partir d'une analyse en spectrométrie de masse. Si l'analyse par ce procédé s'accompagne d'une décomposition ménagée des molécules, on peut également déterminer leur séquence. Cependant, dans le cadre d'un mélange complexe, notamment un mélange comprenant plus de 100 cibles moléculaires à analyser, dont on ignore si elles peuvent être distinguées entre elles par la taille ou la masse, il devient difficile, voire impossible d'analyser les cibles.

Une autre méthode possible de détection fait appel à la résonance plasmonique de surface (SPR) qui permet de déterminer la densité de matière accumulée à une petite distance (moins de 200nm) de la surface d'une lame de faible épaisseur (xnm) en métal à électron libre comme l'or ou le platine. La réflexion sur une des faces de la lame de métal est modifiée proportionnellement à la densité et à la quantité de matière se trouvant à proximité de l'autre face.

La résonance plasmonique de surface (SPR) mesure des variations de masse. Lorsqu'une molécule s'hybride, elle apporte une certaine masse qui est proportionnelle au nombre de complexes sondes-cibles formés sur une bio-puce.

Cette méthode est donc susceptible de présenter des aléas similaires à ceux décrits ci-dessus pour l'analyse quantitative des cibles moléculaires contenues dans un mélange complexe.

L'utilisation de ces méthodes de séparation et/ou d'analyse de cibles moléculaires est limitée par la complexité des mélanges à analyser dans lesquels les molécules à étudier ont des formes, des séquences et des tailles différentes, ou par la quantité de matériel disponible, faisant obstacle à la détection des cibles retenues au moyen de sondes.

La présente invention a pour objet de proposer une alternative aux procédés connus d'analyse quantitative de cibles moléculaires contenues dans un mélange complexe, qui surmonte les limitations dues à l'hétérogénéité ou la complexité de la population desdites cibles moléculaires (notamment lorsqu'il s'agit d'un transcriptome ou d'un protéome) et en conséquence permet de recourir à différentes techniques de détection des cibles moléculaires séparées, pour réaliser une mesure quantitative fiable et reproductible desdites cibles moléculaires séparées à partir du mélange.

La présente invention fournit également des moyens compatibles avec l'analyse de cibles moléculaires d'un échantillon biologique, lorsque la quantité de matériel biologique contenant ces cibles est limitée, conformément aux quantités généralement disponibles pour réaliser un diagnostic clinique.

Pour ce faire, les inventeurs ont défini des moyens, comprenant des sondes (comprenant une partie polynucléotidique, y compris sous forme d'oligonucléotides) définies pour constituer ce qui sera dans la suite désigné comme étant un «jeu de sondes primaires ». Lorsqu'il est mis au contact du mélange complexe des cibles à analyser, en particulier en solution, le jeu de sondes primaires est apte à réaliser une empreinte moléculaire desdites cibles, reproductible d'une analyse à l'autre et pouvant être détectée quantitativement, y compris par les moyens de détection connus à ce jour.

L'empreinte des cibles moléculaires à détecter se substitue donc aux cibles, pour la phase de détection.

L'expression « empreinte » utilisée ici signifie que le groupe des sondes primaires du jeu de sondes considéré, qui ont effectivement formé une liaison spécifique avec les cibles moléculaires, représente de façon significative, qualitativement et quantitativement, les cibles moléculaires contenues dans le mélange analysé, sans que chaque sonde soit nécessairement identique par exemple dans sa composition, sa taille et sa forme, à la cible avec laquelle elle se lie. Le procédé selon l'invention permet de s'affranchir de la détermination ou de la prise en compte des séquences particulières des cibles d'acide nucléique ou de la composition particulière des cibles polypeptidiques.

Les sondes du jeu de sondes primaires sont caractérisées en ce qu'elles comprennent ou, selon les modes de réalisation de l'invention consistent en une molécule d'acide nucléique simple brin (polynucléotide), ou en un polynucléotide simple brin modifié, par exemple dont l'une des parties terminales hybride avec une séquence complémentaire de taille plus courte que la séquence simple brin ou par exemple en ce qu'il s'agit d'un PNA (PeptideNucleic Acid). Le terme polynucléotide couvre donc ces formes modifiées qui peuvent cependant être aussi expressément citées dans la suite quand elles sont visées en particulier. Le PNA dont question ci-dessus apporte à la sonde une meilleure résolution en spectrométrie de masse. Il est donc particulièrement intéressant pour la réalisation des modes de réalisation de l'invention qui utilisent ce mode de détection.

Le jeu de sondes primaires est formé par l'association, pour être utilisées en mélange, en particulier en solution, de plusieurs types de sondes, chaque type de sonde se distinguant de tout autre type de sonde du jeu de sondes et étant repérable (en particulier quantifiable) individuellement. Les sondes du jeu de sondes primaires défini dans le cadre de l'invention sont aptes et destinées à reconnaître et à lier spécifiquement, un type de cible lorsqu'il est présent dans le mélange complexe analysé. En résumé, à un type de sonde primaire correspond un unique type de cible dans un mélange complexe. Ce sont les cibles qui, dans le mélange, se lient spécifiquement aux sondes primaires dont on réalise la détection et la quantification. L'ensemble des sondes entrant dans la composition des complexes sonde-cible, forme l'empreinte des cibles analysées.

En outre, les différents types de sondes primaires sont caractérisés par l'absence de marquage de la partie polynucléotidique au moyen de marqueurs de type, coloré ou luminescent, d'affinité, enzymatique, magnétique, thermique ou électrique, (notamment par l'absence de marqueur fluorescent ou radioactif) qui serait destiné à permettre de les détecter et/ou de les quantifier. Dans ces conditions, la détection individuelle des sondes du jeu de sondes primaires ayant formé un complexe spécifique sonde-cible met en oeuvre l'une au moins des trois caractéristiques suivantes de la partie polynucléotidique de la sonde, caractéristiques que l'on qualifie de « marqueurs passifs » associant à une sonde une caractéristique autodéterminante unique et ne modifiant pas son affinité pour sa cible,: (i) la composition et l'enchaînement (la séquence) des nucléotides compris dans le polynucléotide dans la sonde ; (ii) la taille (ou la longueur) du polynucléotide de la sonde ; (iii) la masse du polynucléotide de la sonde.

Dans un mode de réalisation particulier, comme on le verra dans la suite de la description des sondes primaires, la constitution des sondes primaires est telle que soit la taille de leurs séquences d'acides nucléiques est connue et homogène voire identique, soit, lorsque ces sondes comprennent une partie polynucléotidique associée à une partie polypeptidique, leurs séquences polynucléotidiques sont de taille identique.

Dans un autre mode de réalisation de l'invention, les sondes primaires sont formées par des polynucléotides dont la taille et / ou la masse diffèrent des autres polynucléotides du jeu de sondes primaires.

Dans une variante, lorsque les sondes ont à la fois une partie polypeptidique et une partie polynucléotidique, leur partie polynucléotidique peut être calibrée par exemple par sa masse et / ou sa taille, chaque partie polynucléotidique ayant une masse et / ou une taille différentes de celles des autres parties polynucléotiques dans le jeu de sondes primaires.

L'invention a donc pour objet, un jeu de sondes primaires convenant pour l'analyse des cibles moléculaires dans un mélange complexe, comprenant une population de sondes primaires de types différents, en solution, dans laquelle :
- chaque type de sonde primaire est différent des autres types de sondes primaires du jeu de sondes,
- chaque type de sonde primaire est susceptible de se lier, par liaison spécifique, à un unique type de cible moléculaire à analyser, lorsque lesdites sondes primaires et lesdites cibles moléculaires sont mises en contact,
- chaque type de sonde primaire est un polynucléotide ou alternativement comprend un polynucléotide associé à une partie polypeptidique, et est capable de lier spécifiquement un unique type de cible moléculaire du mélange complexe, chaque polynucléotide ayant une séquence nucléotidique connue et différente de celle des polynucléotides de toutes les autres sondes primaires du jeu de sondes, soit par l'enchaînement (séquence) des nucléotides qui le compose, soit par sa taille, soit par sa masse, chacun des polynucléotides étant connus respectivement par sa séquence, sa taille ou par sa masse, au sein du jeu de sondes.

Ces propriétés des sondes du jeu de sondes primaires sont illustrées et complétées par les caractéristiques énoncées ci-dessous dans l'exposé de différents modes de réalisation, caractéristiques qui définissent des sondes particulières.

Dans un mode de réalisation particulier de l'invention, dans le jeu de sondes ainsi défini, chacun des polynucléotides est identifié par rapport aux autres et en particulier est connu, respectivement par sa séquence ou par une partie de sa séquence lorsque ledit polynucléotide est destiné à être mis en oeuvre dans une réaction spécifique d'hybridation soit pour former un complexe polynucléotide-sonde secondaire (décrit plus loin) soit pour former le complexe sonde-cible. Dans ce cas, la succession spécifique des nucléotides qui composent le polynucléotide, est déterminée ou déterminable et connue comme étant différente de celle des séquences des polynucléotides des autres types de sondes.

Si la détection du polynucléotide de la sonde primaire ne fait pas intervenir de réaction d'hybridation spécifique avec une séquence d'acide nucléique complémentaire, alors le polynucléotide de chaque type de sonde primaire peut être différent des polynucléotides des autres types de sondes primaires par sa taille et/ou par sa masse seulement. La taille d'une sonde primaire est déterminée par le nombre total de nucléotides de la formule brute, donnant la longueur du polynucléotide de la sonde, et sa masse est déterminée à partir de la formule brute de la séquence, en tenant compte du nombre de chacun des nucléotides qui la composent et de la masse de chacun au sein du jeu de sondes, et en prenant en compte les modifications éventuelles apportées dans ladite séquence pour faire varier sa masse par rapport à la masse de la séquence initialement identifiée pour constituer un polynucléotide approprié dans un complexe sonde-cible.

Ces trois critères (séquence, taille et masse) permettent de produire un nombre considérable de sondes primaires différentes. En considérant la formule brute : (Aₙ, Cₘ, Tᵢ, Gⱼ) dans lequel A est Adénosine, C est Cytosine, T est Thymine, G est Guanine, et n, m, i, j sont leurs nombres respectifs au sein de la formule (la taille du polymère d'acide nucléique étant égale à (n + m + i + j), il est possible de générer un nombre égal à (n + m + i + j) ! / (n! . m! . i! . j!) sondes différentes sans compter les possibilités de modifications notamment pour faire varier la masse (x! signifiant factorielle de x).

Dans le cas où la masse du polynucléotide de chaque type de sonde primaire est connue et est différente pour chaque type de sonde, l'analyse des sondes primaires, c'est-à-dire l'identification des sondes primaires qui se sont liées spécifiquement à des cibles moléculaires, est effectuée après une étape de détection des masses de leurs polynucléotides. Cette détection peut être réalisée par spectrométrie de masse.

La masse du polynucléotide d'un type de sonde primaire doit être facilement différenciable des masses des polynucléotides de tous les autres types de sondes primaires.

Lorsque les masses de deux polynucléotides correspondant à deux types de sondes primaires sont proches, c'est-à-dire susceptibles d'être confondues dans une détection, par exemple par spectrométrie de masse, la masse d'au moins l'un d'entre eux peut être modifiée par exemple par substitution de certains de ses atomes par des atomes lourds ou par la méthylation ou l'éthylation de certaines de ses bases (A, T, C, G) telles que la cytosine, afin de faciliter leur différenciation.

Dans le cas d'un jeu de sondes primaires contenant plusieurs centaines voire plusieurs milliers ou plusieurs dizaines ou centaines de milliers de types différents de sondes primaires, il est avantageux de modifier ainsi les masses de leurs polynucléotides de façon raisonnée afin de pouvoir aisément identifier les polynucléotides des différents types lors de l'étape d'analyse des sondes primaires.

L'étape d'analyse des sondes primaires qui se sont liées spécifiquement à des cibles moléculaires d'un échantillon testé et dont les polynucléotides ont des masses différentes peut consister à :
- Réaliser un mélange étalon de sondes constitué du même jeu de sondes que le jeu de sondes primaires destinées à l'analyse de l'échantillon en modifiant toutefois la masse de chacune des sondes d'une quantité par exemple égale à une unité de résolution du spectromètre de masse ,
- analyser par spectrométrie de masse les polynucléotides (présents en quantité connue) des sondes primaires qui se sont liées spécifiquement aux cibles moléculaires de manière à obtenir un spectre de masse, en présence d'une quantité connue de sondes du mélange étalon mis en oeuvre à plusieurs dilutions, et le cas échéant vérifier que la masse d'aucune sonde étalon modifiée de la susdite quantité d'unité de résolution ne chevauche pas la masse d'une sonde du jeu de sondes testées, cette étape constituant la préparation d'un spectre de référence

- ajouter à l'empreinte réalisée de l'échantillon analysé, au moyen des sondes du jeu de sondes primaires, une quantité connue du mélange étalon et obtenir un spectre de masse pour ce mélange ;
- comparer le spectre de masse obtenu à l'étape ci-dessus avec le spectre de masse de référence de façon à quantifier les molécules cibles à travers la quantification des sondes du jeu de sondes primaires formant la susdite empreinte..

Dans le cas où la taille du polynucléotide de chaque type de sonde primaire est connue et diffère de celle des autres types de sondes primaires, ou dans le cas de modifications stériques l'analyse des sondes primaires est effectuée par détection des tailles de leurs polynucléotides. La détection peut alors être réalisée par un système de séparation par électrophorèse, par filtration ou par chromatographie, en phase gazeuse ou liquide, couplé à un système de détection par UV ou par effet LIBS (Laser Induced Breackdown Spectroscopy).

Comme décrit précédemment, il peut être avantageux de modifier les polynucléotides choisis pour entrer dans la constitution des sondes primaires pour leur conférer des propriétés physico-chimiques différentes (par un nombre déterminé de méthylation, éthylation, etc.) afin de pouvoir aisément les identifier lors de l'étape d'analyse des sondes primaires, notamment lorsque différents polynucléotides appelés à constituer des sondes ont une même masse.

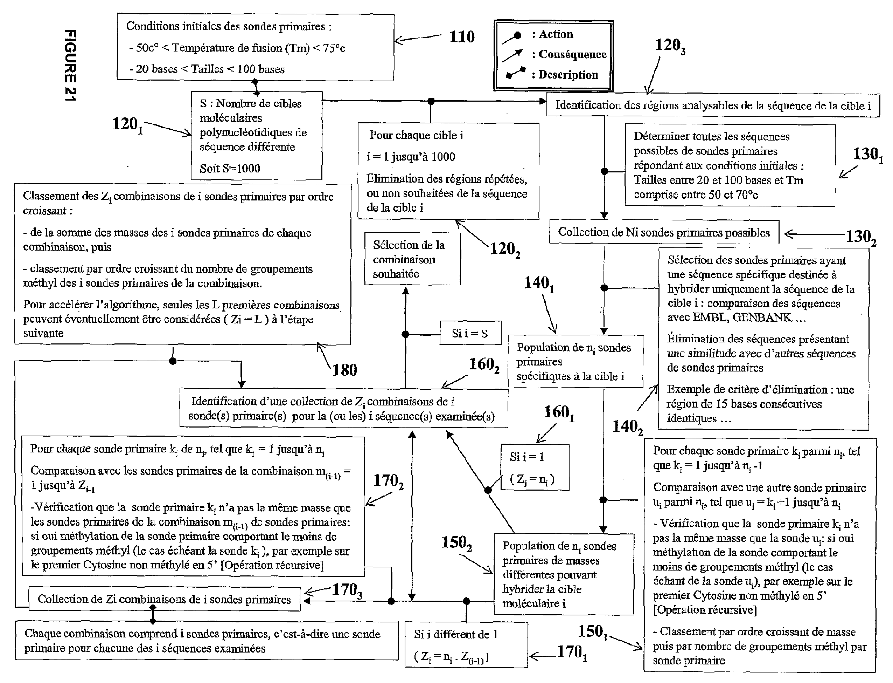

Dans ce cas, et lorsque les cibles sont des acides nucléiques, la préparation d'un jeu de sondes primaires dont la partie polynucléotidique est ainsi constituée peut être effectuée, notamment lorsqu'il y a lieu d'obtenir un grand nombre de sondes, suivant les étapes représentées en figure 21 et décrites ci-dessous, ces étapes étant mises en oeuvre à l'aide d'un programme ordinateur lorsque le nombre de sondes s'y prête.
- Etape 110 : la définition d'un intervalle de taille des sondes primaires, par exemple constituées de polynucléotides comprenant de 20 à 150, par exemple de 20 à 100 nucléotides et la définition de conditions d'hybridation des sondes aux cibles et en particulier de la température de fusion Tm (température à laquelle 50% d'une sonde donnée hybride à sa séquence complémentaire), comprise par exemple entre 50 et 75°C pour l'ensemble des sondes.
- Etapes 120₁, 120₂ et 120₃: la détermination du nombre S de cibles moléculaires polynucléotidiques de séquences différentes, le cas échéant après avoir écarté les régions de la cible non désirées pour l'établissement de sondes. Ce nombre peut être égal ou supérieur à 10, 50, 100, 500, 1000, 10 000, 20 000 voire plus. Chacune de ces séquences cibles porte un nombre i, ce nombre étant compris entre 1 et S.
- Etapes 130₁, 130₂ : l'identification, pour chaque séquence cible i potentiellement contenue dans l'échantillon, d'une collection de Nᵢ sondes possibles parfaitement hybridables avec la cible considérée et dont la taille est comprise dans l'intervalle de taille choisi et le Tm est compris dans les valeurs choisies.
- Etapes 140₁ et 140₂ : la sélection au sein de la susdite collection de Nᵢ séquences, d'une population de nᵢ séquences polynucléotidiques pouvant se lier par hybridation à la cible moléculaire constituée par la séquence i considérée et dont la liaison ainsi formée est spécifique, excluant dès lors une hybridation avec les autres séquences cibles possibles du mélange. Pour cela, chacune des sondes de la collection de Ni sondes est comparée à toutes les séquences i susceptibles d'être présentes dans l'échantillon, par exemple à l'aide des bases de données de type EMBL, Genebank... Chaque population de nᵢ sondes est spécifique d'une unique séquence i de cible moléculaire. Les séquences des sondes primaires qui présentent une similarité au-delà d'un certain seuil avec d'autres séquences de la collection de Ni sondes, par exemple un seuil de 15 bases consécutives identiques, peuvent être éliminées.
- Etapes 150₁ et 150₂ : au sein de cette population de nᵢ polynucleotides putatifs pour la constitution de sondes, l'attribution à chaque polynucléotide, d'une masse différente de la masse des autres polynucléotides de la population, le cas échéant en modifiant des nucléotides qu'il contient, par exemple par méthylation ou éthylation d'un ou plusieurs résidus cytosine, pour obtenir une collection de polynucléotides ayant tous une masse différente et connue pour chaque séquence. A chaque modification de séquence la comparaison est répétée de façon récursive. La population de nᵢ polynucléotides est de préférence classée par ordre croissant de masses puis par ordre croissant de nombre de méthylations ou autres modifications.
- Etapes 160₁ et 160₂ : Pour i = 1, c'est-à-dire pour la séquence de la première cible moléculaire considérée, il existe Zᵢ combinaisons de 1 sonde primaire (Zᵢ étant égal à nᵢ).
- Etapes 170₁, 170₂ et 170₃ : Pour la cible i, i différent de 1 et par exemple égal à 2, les étapes précitées sont répétées pour l'identification d'une population de nᵢ sondes primaires ayant toutes une masse différente et étant capable d'hybrider avec la séquence cible i. On identifie une collection de Zᵢ combinaisons de i sondes primaires, chacune de ces combinaisons comprenant une unique sonde primaire de chaque population de nᵢ sondes examinée, ou autrement dit chacune des i sondes primaires est déduite d'une et d'une seule des i séquences examinées., l'ensemble des sondes primaires de chacune des Zᵢ combinaisons étant tel que tous les polynucléotides ont une masse différente. Pour cela, chacune des sondes primaires de la population de nᵢ sondes est comparée aux sondes primaires des combinaisons précédemment établies, c'est-à-dire de chaque combinaison m₍ᵢ₋₁₎, m₍ᵢ₋₁₎ allant de 1 jusqu'à Zᵢ₋₁. Lorsque les masses de ces sondes primaires sont trop proches l'une de l'autre, la sonde primaire comprenant le moins de modifications, par exemple le moins de groupements méthyl ou éthyl peut être méthylée, par exemple sur le premier cytosine non méthylé ou non éthylé en 5'. A chaque modification de séquence la comparaison est répétée de façon récursive.
- Etape 180 : Les Zᵢ combinaisons de i sondes primaires sont classées par ordre croissant, d'une part par la somme des masses des i sondes primaires qui composent la combinaison, puis par classement par ordre croissant du nombre de groupements de modification par exemple de groupements méthyl ou éthyl des i sondes primaires de la combinaison, de manière à ce que les sondes primaires ayant un même nombre de modifications dans une combinaison soient classées par ordre croissant de leur masse. Une partie seulement des combinaisons peut être considérée, par exemple les L premières, pour accélérer l'algorithme à l'étape suivante.
- Etape 160₂ : Identification d'une collection de Zᵢ combinaisons de i sondes primaires pour les i séquences examinées.
- Les étapes précitées sont répétées pour chaque i, i allant de 3 à S. Lorsque i = S et que l'on a identifié une collection de Zₛ combinaisons de S sondes primaires pour les S séquences cibles, on peut choisir l'une de ces combinaisons qui constituera le jeu de sondes primaires. Cette combinaison est de préférence celle qui est soit la plus facile à réaliser en termes de temps et de coûts, soit celle qui apparaît en premier dans le classement des Zₛ combinaisons effectué à l'étape 180.

Les sondes primaires du jeu de sondes ainsi défini sont synthétisées par toute méthode en soi connue de préparation de polynucléotides. De façon plus générale, le jeu de sondes primaires est préparé par le procédé comprenant les étapes consistant en :
- la définition d'un intervalle de taille des sondes primaires et la définition de conditions d'hybridation des sondes aux cibles et en particulier de la température de fusion Tm pour l'ensemble des sondes,
- la détermination du nombre i de cibles moléculaires polynucléotidiques de séquences différentes du mélange complexe
- l'identification, pour chaque séquence cible i potentiellement contenue dans l'échantillon, d'une collection de Nᵢ sondes possibles parfaitement hybridables avec la cible considérée et dont la taille est comprise dans l'intervalle de taille choisi et le Tm est compris dans les valeurs choisies,
- la sélection au sein de la susdite collection de Nᵢ sondes, d'une population de nᵢ séquences polynucléotidiques pouvant se lier par hybridation à la cible moléculaire constituée par la séquence i considérée et dont la liaison ainsi formée est spécifique, excluant dès lors une hybridation avec les autres séquences cibles possibles du mélange,
- au sein de cette population de nᵢ sondes putatives pour la constitution de sondes, l'attribution à chaque polynucléotide, d'une masse différente de la masse des autres polynucléotides de la population, le cas échéant en modifiant des nucléotides qu'il contient, par exemple par méthylation ou éthylation d'un ou plusieurs résidus cytosine, pour obtenir une collection de polynucléotides ayant tous une masse différente et connue pour chaque séquence, la comparaison étant répétée de façon récursive à chaque modification de séquences,
- l'identification d'une collection de Zᵢ combinaisons de i sondes primaires pour chaque séquence i, chacune de ces combinaisons comprenant une unique sonde primaire de chaque population de nᵢ sondes examinée ; chacune des sondes primaires de la population de nᵢ sondes étant comparée aux sondes primaires des combinaisons précédemment établies, et lorsque les masses de ces sondes primaires sont trop proches l'une de l'autre, la modification de leur séquence, la comparaison étant répétée de façon récursive à chaque modification de séquence,
- le classement des Zᵢ combinaisons de i sondes primaires par ordre croissant de la somme des masses des i sondes primaires qui composent la combinaison et par ordre croissant du nombre de groupements de modification,
- l'identification d'une collection de Zᵢ combinaisons de i sondes primaires pour les i séquences examinées,
- la synthèse des sondes primaires du jeu de sondes ainsi défini.

Lorsque les cibles de l'échantillon ne sont pas des acides nucléiques, la procédure de préparation des polynucléotides contenus dans les sondes primaires est aménagée par rapport à la description ci-dessus, dans la mesure où il n'est pas nécessaire de prendre en considération une réaction spécifique d'hybridation des sondes avec les cibles.

Lorsque la masse n'est pas un critère discriminant pour la détection des polynucléotides des sondes primaires, la discrimination peut se faire par la taille et/ou par la séquence. Quoi qu'il en soit des caractéristiques de taille et de masse des polynucléotides des sondes primaires, la séquence des polynucléotides de chaque type de sonde primaire dans le cas où les cibles sont des acides nucléiques doit être différente de toutes les autres séquences des autres types de sondes primaires, pour permettre la liaison spécifique à une cible à l'exclusion des autres. La spécificité ainsi définie de la séquence du polynucléotide peut selon un mode de réalisation de l'invention constituer le moyen de détection de la sonde primaire et donc de la cible.

Lorsque la détection des sondes primaires des complexes cible-sonde fait appel à la détection des séquences des polynucléotides, on a recours à l'utilisation d'un jeu de sondes secondaires comme décrit plus Ioin.La figure 21 représente un exemple de stratégie pour l'obtention d'un mélange de 1000 sondes primaires différentiées par leur masse. Le choix de la masse de chaque sonde primaire se fait en fonction de la séquence, de la taille et / ou des modifications des bases des acides nucléiques, par exemple par ajout de groupements méthyl sur les cytosines. Le jeu de sondes primaires peut être prévu pour être analysé en spectromètre de masse MALDI-TOFF avec une résolution de 13 daltons. L'altération de la masse d'une sonde primaire peut être obtenue par la substitution de plusieurs atomes par des isotope lourds.

Une approche similaire peut être envisagée pour produire un jeu sondes primaires avec des critères chromatographiques spécifiques à chaque sonde primaire, en considérant la taille des sondes primaires, et la position des méthylations et des substitutions effectuées.
Pour l'utilisation dans le cadre de l'invention, le jeu de sondes primaires comprend un excès de chaque type de sondes primaires par rapport à la quantité de cibles qu'il peut reconnaître dans le mélange complexe. En outre, le jeu de sondes primaires est utilisé sous la forme d'un mélange stoechiométrique des sondes primaires.

Pour respecter la stoechiométrie du mélange de sondes, les sondes sont préparées de façon à ce que leurs parties polynucléotidiques ne puissent pas hybrider entre elles, du moins ne puissent pas former des hybrides stables sonde-sonde.

Les inventeurs ont donc montré que des sondes non activement marquées (par une molécule repérable en tant que telle) peuvent être utilisées en mélange, pour obtenir une empreinte caractéristique des cibles moléculaires hétérogènes du mélange complexe à analyser et reproductible, ladite empreinte étant constituée par les sondes ayant formé des complexes sonde-cible en solution ou par la partie polynucléotidique de ces dernières, et étant soumise à une mesure quantitative, pour en déduire la quantité de cibles contenues dans le mélange.

Lesdites sondes (ou leur partie polynucléotidique) peuvent en outre être identifiées et quantifiées individuellement après avoir formé des complexes sonde-cible en solution après mise en contact avec le mélange de cibles. Cette identification comprend l'identification de leur partie polynucléotidique par la détection d'un effet dû à l'enchaînement de nucléotides du polynucléotide, à sa taille ou à sa masse.

La demande décrit donc un jeu de sondes primaires en mélange stoechiométrique convenant pour l'analyse des cibles moléculaires dans un mélange complexe comprenant une population de sondes primaires de types différents, en solution, dans laquelle chaque type de sonde primaire composant le jeu est différent des autres types de sondes au niveau de l'enchainement des nucléotides du polynucléotide qu'elle comprend et/ou selon des modes de réalisation particuliers de l'invention est différent par la masse dudit polynucléotide ou par sa taille ou par ces deux derniers critères, susceptibles de se lier par liaison spécifique à un type de cibles moléculaires à analyser, lorsque lesdites sondes primaires et lesdites cibles moléculaires sont mises en contact et amenées à réagir en solution.

Dans un mode de réalisation décrit dans la demande, le jeu de sondes primaires comprend au moins 10 sondes primaires, par exemple au moins 50, en particulier au moins 100, notamment au moins 200, avantageusement au moins 1000 ou au moins 10 000 ou 20 000 sondes.

L'invention a pour objet un procédé d'analyse de cibles moléculaires contenues dans un mélange complexe comprenant :
a. la mise en contact en solution du mélange de cibles moléculaires à analyser avec un jeu de sondes primaires de types différents, chaque type de sonde primaire composant le jeu étant susceptible de se lier par liaison spécifique à un unique type de cible moléculaire du mélange complexe, dans des conditions permettant la liaison spécifique entre lesdites cibles moléculaires et lesdites sondes primaires, pour former un complexe sonde primaire-cible en solution, chaque sonde primaire étant constituée par un polynucléotide ou comprenant une partie formant un polynucléotide dont la séquence nucléotidique est connue et est, pour chaque type de sonde primaire, différente de toutes les séquences nucléotidiques des polynucléotides des autres types de sonde primaire,
b. éventuellement l'élimination des sondes primaires non spécifiquement liées avec une cible moléculaire,
c. la séparation des cibles moléculaires et des sondes primaires liées par liaison spécifique dans ledit complexe sonde primaire-cible, de façon à récupérer les sondes primaires représentant une empreinte des cibles moléculaires à analyser,
d. l'analyse des sondes primaires éluées à l'étape c, comportant l'identification et la quantification du polynucléotide de chaque sonde primaire éluée, en particulier par la détection d'un effet dû à la séquence nucléotidique, à la taille ou à la masse de ce polynucléotide.

Le procédé selon l'invention analyse les cibles moléculaires par l'intermédiaire de sondes primaires. Comme indiqué ci-dessus, les sondes primaires, sans être constituées nécessairement de façon exclusive par des acides nucléiques, comprennent toutefois une partie polynucléotidique. Cette partie polynucléotidique doit pouvoir être détectée, éventuellement par des sondes secondaires de détection par exemple pour des polynucléotides de tailles homogènes voire identiques, ou par d'autres moyens, par exemple en repérant les différences entre les tailles ou les masses ou d'autres propriétés physico-chimiques connues des polynucléotides selon leur structure. Les sondes primaires ayant effectivement formé une liaison spécifique avec les cibles moléculaires représentent un sous groupe des sondes primaires du jeu de sondes mis en oeuvre initialement, qui constitue une empreinte des cibles moléculaires à détecter contenues dans le mélange.

Dans un mode de réalisation de l'analyse d'un mélange complexe d'acides nucléiques, (à la différence des cibles moléculaires à analyser, dont la taille et/ou la séquence peuvent être indéterminées, voire inconnues), les sondes primaires peuvent être choisies de façon à avoir toutes une taille homogène, par exemple identique ou, lorsque les sondes combinent une partie polypeptidique et une partie polynucléotidique, la taille de cette dernière est homogène, par exemple identique, entre les sondes. L'homme du métier est en mesure de déterminer quelle variation de taille entre les sondes est acceptable, en fonction notamment de la sensibilité des moyens de détection, électriques ou optiques mis en oeuvre. A titre indicatif, on considérera que la taille des sondes est homogène lorsqu'elle varie dans un intervalle de ± x% par rapport à une taille choisie si la mesure du détecteur a une sensibilité de ± x%. En pratique, une variation de taille dans un intervalle d'environ ± 10% serait acceptable.

De plus, la séquence de chaque type de sonde primaire (ou de partie nucléotidique de sonde primaire) est connue et différente des autres. En particulier ces séquences sont linéaires. Les variations de mesure engendrées par chaque sonde peuvent être calibrées en fonction de sa séquence.

Les sondes primaires ou leur partie polynucléotidique, dont la taille et la séquence sont connues ou calibrées qui ont lié spécifiquement des cibles moléculaires du mélange analysé sont dans un second temps séparées de leur cible et récupérées (éluées) pour être analysées, par exemple au moyen de sondes secondaires polynucléotidiques. L'utilisation de sondes secondaires dans une réaction d'hybridation avec les sondes primaires éluées, permet d'identifier et de quantifier lesdites sondes primaires et indirectement d'identifier et de quantifier les cibles moléculaires. Les sondes secondaires en question sont définies de la même façon que les sondes primaires constituées de polynucléotides en considérant dans ce cas que leurs cibles sont les sondes primaires.

La détection des couples formés entre les sondes primaires (ou leur partie polynucléotidique) et les sondes secondaires peut être réalisée sans que les sondes primaires soient marquées activement, comme illustré plus loin.

Lesdites sondes primaires éluées, peuvent être détectées en les faisant circuler au niveau d'une matrice de sondes secondaires de types différents, chaque sonde secondaire possédant une séquence susceptible de retenir un unique type de sonde primaire par liaison spécifique par hybridation moléculaire. Les sondes secondaires utilisées forment un jeu de sondes de taille semblable à la taille du jeu de sondes primaires mis en contact avec le mélange complexe.

La présente invention permet donc, dans le cadre de l'analyse d'un mélange complexe d'acides nucléiques, lorsque l'on utilise à la fois un jeu de sondes primaires et un jeu des sondes secondaires, mises en contact sur une puce, de réaliser des mesures ne dépendant que de l'abondance de chaque type de sonde et d'éviter les artéfacts de mesure dus à la présence de cibles moléculaires de tailles, de formes et de séquences différentes.

Dans le cadre de l'analyse d'un mélange complexe de cibles moléculaires qui ne seraient pas des molécules d'acides nucléique, par exemple des protéines ou tout autre molécule capable de former un complexe de type sonde-cible spécifique (par exemple des sucres, des lipides,...), la présente demande décrit un jeu de sondes primaires constitué de telle sorte qu'il représente une gamme de sondes dont la partie polynucléotidique se distingue pour chaque sonde par une séquence différente et une taille homogène, voire identique dans le jeu de sondes, ou au contraire chaque partie polynucléotidique se distingue des autres en ce qu'elle a une masse différente ou une taille différente. Les parties polynucléotidiques des sondes sont séparées des complexes sonde-cible formés dans le mélange en solution, pour être analysées par exemple par hybridation avec des sondes secondaires, par spectrométrie de masse, par chromatographie, par filtration ou par électrophorèse.

Ce qui a été décrit ci-dessus concernant les séquences de nucléotides des sondes primaires polynucléotidiques s'applique expressément aux parties polynucléotidiques des sondes primaires lorsqu'elles comprennent aussi une partie polypeptidique, sauf pour ce qui concerne les étapes faisant intervenir les acides nucléiques cibles.

Dans le cadre des définitions qui précèdent, la présente invention concerne donc un procédé d'analyse de cibles moléculaires, contenues dans un mélange complexe comprenant :
a. la mise en contact en solution du mélange complexe susceptible de comprendre les cibles moléculaires à analyser avec un jeu de sondes primaires de types différents, chaque type de sonde primaire composant le jeu étant susceptible de se lier par liaison spécifique à un unique type de cible parmi les cibles moléculaires du mélange complexe, dans des conditions permettant la liaison spécifique entre lesdites cibles moléculaires et lesdites sondes primaires, pour former un complexe sonde primaire-cible en solution, chaque sonde primaire étant constituée par un polynucléotide ou comprenant une partie formant un polynucléotide dont la séquence nucléotidique est connue et est, pour chaque type de sonde primaire, différente de toutes les séquences nucléotidiques des polynucléotides des autres types de sonde primaire,
b. éventuellement l'élimination des sondes primaires non spécifiquement liées avec une cible moléculaire,
c. la séparation des complexes cibles moléculaires-sondes primaires formés par liaison spécifique, de façon à récupérer les sondes primaires représentant une empreinte des cibles moléculaires contenues dans le mélange reconnues par lesdites sondes primaires,
d. l'analyse des sondes primaires éluées à l'étape c, comportant l'identification et la quantification du polynucléotide de chaque sonde primaire éluée, en particulier par la détection d'un effet dû à la séquence nucléotidique, à la taille ou à la masse de ce polynucléotide.

Avantageusement, le procédé de l'invention permet l'analyse quantitative des sondes primaires éluées à l'étape c.

La quantification de ces sondes primaires (ou de leurs parties polynucléotidiques) séparées des cibles moléculaires permet l'analyse quantitative des cibles moléculaires qui sont en quantité proportionnelle à celle des sondes primaires séparées.

Par l'expression «mélange complexe», il faut comprendre au sens de l'invention une solution comprenant un grand nombre de molécules de structures (notamment de séquences) différentes, en particulier un mélange de plus de 10, en particulier de plus de 50 plus de 100 ou plus de 1000, voire plus de 10 000 ou de 20 000 molécules ayant des structures différentes. Le dispositif de l'invention est plus préférentiellement destiné à l'analyse de molécules biologiques (ou biomolécules telles que des acides nucléiques ou des protéines), contenues notamment dans un échantillon d'origine biologique. En particulier le mélange complexe est un transcriptome ou une représentation d'un transcriptome (sous forme d'ADNc) ou est un protéome.

L'invention permet aussi l'analyse d'un mélange complexe comprenant à la fois des acides nucléiques cibles et des cibles polypeptidiques. Dans ce cas particulier, les deux populations de cibles doivent pouvoir être distinguées l'une de l'autre dans le cadre de l'analyse. Cette distinction peut être obtenue par le choix d'un jeu de sondes primaires propre à chaque population.

Plus particulièrement, l'échantillon fournissant le mélange complexe peut être issu d'un prélèvement de tissu ou d'un fluide biologique, tel que le sang, le sérum, le plasma, le liquide céphalo-rachidien, l'urine ou la salive. Le prélèvement peut être réalisé sur un animal (en particulier un mammifère et de préférence chez l'homme). Le prélèvement peut en particulier être réalisé chez un individu sain ou un patient atteint d'une pathologie. La pathologie peut notamment être un cancer, une pathologie neuro-dégénérative, une pathologie infectieuse et en particulier une pathologie virale, bactérienne ou parasitaire.

L'échantillon peut également contenir un extrait tissulaire ou un extrait cellulaire, issu de cellules eucaryotes ou procaryotes, de bactéries, de champignons ou de levures, notamment de cellules en cultures ou de cellules prélevées dans l'environnement extérieur. L'échantillon peut aussi être obtenu à partir d'un prélèvement fait sur un végétal. Il peut aussi s'agir d'un prélèvement effectué sur un produit agro-alimentaire, notamment sur des aliments cuisinés, ou à partir de graines, de fruits ou de céréales.

Par l'expression « cibles moléculaires », il faut notamment entendre des biopolymères, tels que des molécules d'ADN, d'ARN, en particulier ADN ou ARN génomique, ADNc ou ARNm, siRNA... des peptides ou encore des protéines susceptibles d'être reconnus et spécifiquement liés aux sondes primaires de l'invention.

Pour la réalisation de l'étape a) conduisant à la liaison spécifique des cibles moléculaires et des sondes primaires, différentes conditions sont envisageables.

Dans le procédé de l'invention cette étape est réalisée en solution.

Dans un mode de réalisation de ce procédé, les cibles moléculaires à détecter peuvent être fixées à des particules mises en contact avec le mélange en solution. Des particules magnétiques peuvent par exemple être utilisées. On peut aussi avoir recours à des billes magnétiques recouvertes d'ITO ou de poly-imide. La totalité des cibles peut être fixée. Alternativement on peut ne fixer que certains types de cibles pour une analyse plus spécifique du mélange. Selon un autre mode de réalisation, on peut utiliser un mélange de particules différentes où chaque type de particules est destiné à la fixation d'un type déterminé de cibles moléculaires.

Alternativement, la demande décrit des modes de réalisation où les cibles moléculaires du mélange complexe à analyser sont fixées sur un support. Le jeu de sondes primaires est alors amené au contact dudit support pour la réalisation de l'étape a).

L'étape a) est réalisée au moyen d'un mélange d'un excès de sondes primaires avec les cibles moléculaires. Par « excès de sondes », on entend une quantité de sondes supérieure ou égale à la quantité supposée de cibles moléculaires. L'excès de sondes primaires utilisées doit permettre de saturer les cibles reconnues et liées spécifiquement par ces sondes.

Pour réaliser l'étape a), les sondes sont en mélange stoechiométrique.

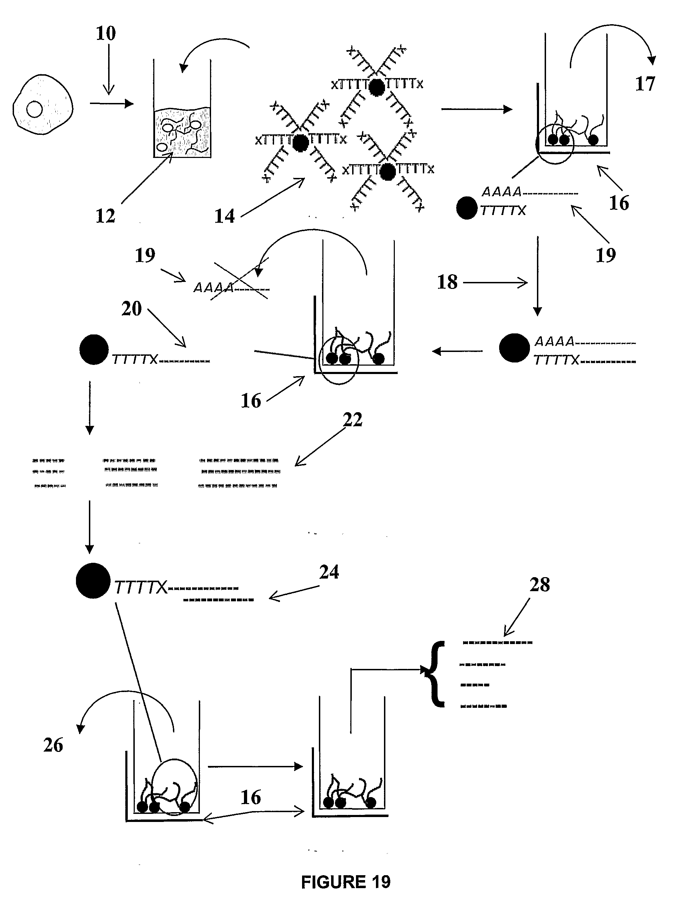

Dans un mode de réalisation particulier décrit dans la demande, la réaction d'hybridation des sondes du jeu de sondes primaires est réalisée en appliquant des conditions thermodynamiques, par exemple dans un gradient de température et le cas échéant an appliquant des paliers de refroidissement et de réchauffement, comme illustré plus loin dans la description de la figure 19.

L'élimination à l'étape b), des sondes primaires n'ayant pas formé des complexes sonde-cible peut être une récupération de ces sondes pour les enlever du milieu de la réaction. Cette étape b) peut être déplacée et effectuée seulement avant la mise en contact des sondes primaires séparées des complexes sonde-cible, avec les moyens de détection. Par exemple, les sondes n'ayant pas formé de complexes sonde-cible doivent être séparées des autres sondes avant la mise en contact de ces dernières avec des sondes secondaires qui seraient utilisées pour détecter les sondes primaires de l'empreinte moléculaire.

L'étape c. comprend la séparation des complexes cible moléculaire-sonde primaire. Par la notion de « séparation », il faut entendre :
- Soit que l'on sépare des doubles brins d'acide nucléique (ADN-ADN, ADN-ARN...) formés par hybridation des cibles moléculaires et des sondes primaires;
- Soit que l'on sépare les complexes spécifiques formés entre des cibles moléculaires de nature polypeptidique et les sondes primaires (comprenant par exemple des anticorps ou fragments d'anticorps ou récepteurs et une partie polynucléotidique). Ultérieurement, la partie polynucléotidique des sondes primaires devra être séparée du reste de la sonde primaire.
- Soit que l'on sépare la partie polynucléotidique des sondes primaires spécifiquement liées aux cibles moléculaires.
Par « séparation », on entend l'opération consistant à rompre la liaison spécifique établie entre la cible et la sonde ou à rompre la liaison établie entre la partie polynucléotidique de la sonde et le reste du complexe cible-sonde. Dans le cas particulier de molécules d'acide nucléique, la séparation correspond à la dénaturation contrôlée de l'hybride formé entre deux brins complémentaires d'ADN, d'ADN/ARN..., encore appelée déshybridation.

Dans le cas de cibles moléculaires de type protéique si la séparation intervient entre les deux entités du complexe cible-sonde, la séparation est par exemple une rupture du complexe antigène/ anticorps (ou fragment d'anticorps comportant le site de liaison antigénique) ou ligand/ récepteur formé par liaison spécifique.

Le terme « liaison spécifique » en référence à la liaison d'une sonde avec une cible ou d'une sonde avec une sonde, signifie que la sonde se lie avec une cible ou une sonde particulière mais ne se lie pas de manière significative avec les autres cibles ou sondes, et plus particulièrement avec les autres cibles ou sondes présentes dans le mélange complexe.

Par l'expression « sondes de types différents », il faut notamment entendre des molécules ayant des séquences de polynucléotides différentes et, lorsque la sonde comprend en outre une partie non polynucléotidique, en particulier une partie polypeptidique, cette partie est différente pour chaque type de sonde et spécifique d'un seul type de cible et, de plus chaque partie polynucléotidique est différente des parties polynucléotidiques des autres sondes et répond aux définitions données dans la présente demande. Chaque type de sonde composant le jeu de sondes primaires est spécifique d'un type de cible unique parmi celles analysées.

La récupération des sondes primaires après leur liaison aux cibles moléculaires pendant l'étape c) peut être obtenue en immobilisant initialement les cibles sur des particules, par exemple des particules magnétiques. Dans le cas d'une fixation des cibles à des particules magnétiques on applique un champ magnétique après l'étape de liaison sondes primaires-cibles, afin de récupérer lesdites sondes primaires.

La récupération des sondes primaires après leur liaison aux cibles moléculaires pendant l'étape c) peut être obtenue en réalisant une centrifugation pour récupérer les sondes primaires, après avoir séparé lesdites sondes des entités « cibles-particules».

Dans un autre mode de réalisation particulier décrit dans la demande, lorsque les sondes primaires représentant une empreinte des cibles moléculaires contenues dans le mélange analysé, sont obtenues après avoir immobilisé initialement les cibles moléculaires sur un support, puis avoir fait circuler les sondes primaires sur le support dans des conditions appropriées pour permettre leur contact et leur liaison spécifique avec les cibles moléculaires, les sondes primaires non hybridées sont éliminées du support puis les complexes « cibles moléculaires-sondes primaires » sont séparés pour recueillir les sondes primaires.

Il est alors possible d'effectuer une quantification des sondes primaires qui ont préalablement été liées spécifiquement aux cibles moléculaires, notamment qui ont été hybridées. Le support utilisé dans cet exemple est notamment une membrane, par exemple fabriquée en nitrocellulose, avec des lames de silane ou de polylysine.

Le procédé peut ainsi être employé dans des applications diverses, notamment dans le diagnostic médical ou le contrôle qualité agro-alimentaire, ou encore toute analyse biologique, dans les domaines notamment de l'écologie, de l'archéologie ou de la criminologie.

A titre d'exemple, le procédé et le dispositif de l'invention permettent l'analyse :
- des molécules d'acide nucléique présentes dans un mélange, par exemple des molécules d'acide nucléique produites (contenues ou transcrites) par une cellule ou un groupe de cellules, identiques ou différentes,
- des protéines contenues dans un mélange, par exemple des protéines produites par une cellule ou un groupe de cellules, identiques ou différentes,
- des acides nucléiques et des protéines contenus dans un mélange, produits par une cellule ou un groupe de cellules, identiques ou différentes.

Des couples sondes-cibles et sondes-sondes préférés, sont notamment les acides nucléiques hybridant avec des séquences complémentaires tels que des molécules d'ARN, en particulier d'ARN messagers, d'ADN ou d'ADNc hybridant avec des sondes d'oligonucléotides spécifiques, des antigènes reconnaissant spécifiquement des sondes constituées d'anticorps monovalents (c'est à dire ayant un seul site de reconnaissance antigénique) ou leurs fragments fonctionnels monovalents (Fab, fragments de digestion à la papaine ou fragments monovalents ayant un site de liaison à un antigène, notamment fragment variable monovalent ...), ou tout couple récepteur-ligand ou vice-versa. Dans ce dernier cas, la sonde primaire comprend également une partie polynucléotidique spécifique pour un type de cible moléculaire donné et susceptible d'hybrider avec les sondes dites secondaires.

L'homme du métier pourra adapter ledit procédé pour l'analyse de tout type de cibles moléculaires dès lors qu'il est possible de leur associer une entité les reconnaissant spécifiquement, au moyen d'un seul site, associé à un polynucléotide de la sonde moléculaire.

Dans un mode de réalisation particulier du procédé ainsi défini, les cibles moléculaires recherchées dans le mélange complexe sont des acides nucléiques.

Dans un mode de réalisation particulier de l'invention, les cibles moléculaires du mélange sont des acides nucléiques représentatifs d'un transcriptome.

Par « représentatif d'un transcriptome », il faut comprendre que les acides nucléiques à analyser comprenant les cibles moléculaires ont des séquences identiques ou complémentaires des ARNm (ou à l'ensemble des ARN) d'une cellule d'un échantillon biologique à analyser. Elles sont notamment obtenues par rétrotranscription et peuvent être des ADNc. Ces séquences s'hybrident spécifiquement dans des conditions stringentes, avec des ARN messagers (ou le complémentaire), produits de la transcription des séquences d'acides nucléiques du génome d'une cellule donnée ou d'un ensemble de cellules, et sont dans des proportions équivalentes à celles des produits de transcription obtenus dans des conditions particulières pour ladite cellule ou ensemble de cellules.
Une méthode pour la préparation d'acides nucléiques représentatifs d'un transcriptome consiste à utiliser des amorces polyT pour réaliser une rétro-transcription des ARNm d'une cellule. Afin d'éviter la présence de queues polyA de grandes tailles, la rétrotranscription de l'échantillon d'ARNm messagers à analyser peut être effectuée à partir d'amorces, telles que 5'(T)ⱼX3' (où j est le nombre de résidus T, j étant supérieur à 1) par exemple avec 5'(T)₁₉X3' où X peut être A, C ou G. La rétrotranscription s'effectue ainsi à partir du premier nucléotide différent de A rencontré après la queue polyA. Pour obtenir un échantillon d'ADNc représentatif du génome fixé sur les particules, les amorces décrites ci-dessus sont préalablement fixées sur lesdites particules, avant la rétrotranscription. Les produits de rétrotranscription peuvent êtres également greffés après la rétrotranscription sur le support. Le greffage peut être assuré par des couplages biotine-avidine en utilisant des amorces 5'(T)ⱼX3' par exemple 5'(T)₁₉X3', biotinylées en 5' (il est également possible d'utiliser un complexation chimique entre les particules et les cibles).

Selon un autre mode de réalisation décrit dans la demande les amorces comprenant la séquence polyT sont des amorces mixtes Peptide Nucleic Acid-Acide Nucléique (PNA-Acide Nucléique).

La figure 19 illustre un exemple de réalisation du procédé selon l'invention dans lequel les cibles moléculaires du mélange sont des acides nucléiques représentatifs d'un transcriptome (ARNm), ces cibles moléculaires étant obtenues par une étape de lyse cellulaire 10.

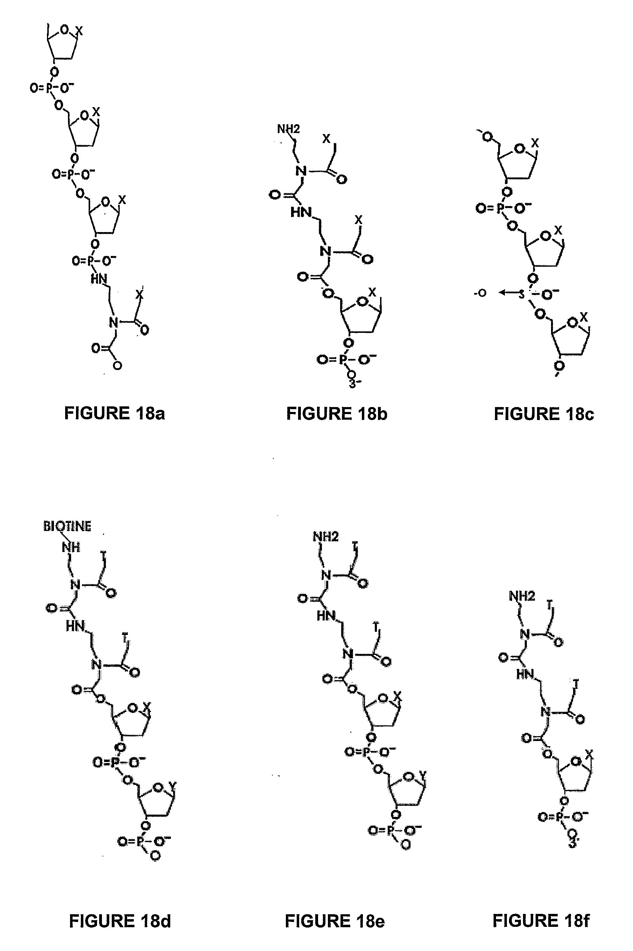

Ces cibles moléculaires 12 sont mélangées à des amorces PNA mixtes 14 Peptide Nucleic Acid-Acide nucléique fixées sur des particules magnétiques. Des amorces du type (PNApolyT)X où X est une base choisie parmi A, G, et C. De telles amorces sont représentées aux figures 18d, 18e, et 18f. Le lien entre le PNA et l'acide nucléique est obtenu par une liaison ester entre l'atome d'oxygène du Phosphore 5' de l'acide nucléique et le groupement COOH terminal du PNA. La fixation des amorces sur les particules magnétiques est obtenue par exemple par la fixation d'une biotine sur le groupement NH2 terminal du PNA par exemple et par une interaction avec une avidine ou une streptavidine fixée sur les particules magnétiques pour donner une association avidine (ou streptavidine) / biotine.

Ces molécules ayant une très grande affinité pour les acides nucléiques, piégent toutes les queues polyA des ARN messagers du mélange de cibles. Les particules magnétiques sont alors immobilisées sur un support à l'aide d'un champ magnétique appliqué par un aimant 16 ou analogue pour pouvoir retirer les cibles moléculaires 17 qui ne sont pas retenues par des amorces.

La rétrotranscription 18 des ARNm peut alors être effectuée à partir d'amorces, telles que NH2-(PNA-polyT)(X)3' par exemple NH2-(PNA-T)₁₉(X)3' (où X peut être un résidu de nucléotide A, C ou G). Une autre construction, plus efficace pour la rétrotranscription, consiste en l'utilisation d'un NH2-(PNA-polyT)XY3', par exemple NH2-(PNA-T)₁₉X(Y)₂3' (où Y représente n'importe lequel des nucléotides A,T,G,C). La rétrotranscription s'effectue ainsi à partir du premier nucléotide différent de A rencontré après la queue polyA.

Les complexes formés sont ensuite dénaturés et les particules magnétiques sont à nouveau immobilisées par un champ magnétique de manière à pouvoir retirer les molécules d'ARN messagers 19. On obtient ainsi un échantillon 20 d'ADNc représentatif du transcriptome fixé sur les particules.

Alternativement, l'utilisation des PNA pour lier les ARN permet de s'affranchir de l'étape de rétrotranscription (étapes 18 et 19 sur la figure) et on hybride alors les sondes directement avec l'ARN qui se trouve être fixé sur les PNA.

L'échantillon 20 est mis en contact avec un jeu de sondes primaires 22 qui sont des polynucléotides dont les séquences, les masses et les tailles respectives sont connues. Les séquences des polynucléotides sont toutes différentes, leurs masses peuvent être identiques ou différentes, et leurs tailles peuvent également être identiques ou différentes.

Chacun des polynucléotides portés par les particules magnétiques se lie spécifiquement par hybridation à un type de sonde primaire pour former un complexe particule-amorce/ADNc-sonde primaire 24. L'hybridation est réalisée dans des conditions thermodynamiques déterminées pour que la taille des sondes primaires n'influe pas sur l'hybridation. Pour cela, la température du milieu est abaissée progressivement , suivant un gradient lent de température ou par exemple en incluant des paliers de refroidissement et de réchauffement successifs, par exemple la température est abaissée depuis une température de 70° jusqu'à une température de 40°c avec des paliers (de durée déterminée pouvant être d'environ 10 secondes à plusieurs minutes ou beaucoup plus, jusqu'à plusieurs heures) de refroidissement et de réchauffement successifs. A titre d'exemple, la température de 70°C peut être abaissée de 3°C et maintenue pendant une temps t pour former une palier de refroidissement puis la température 67°C est augmentée de 1,5°C et maintenue pendant un temps t pour former une palier de réchauffement. Ces étapes sont répétées jusqu'à ce que la température du milieu atteigne 40°C.

Les sondes primaires 26 qui ne sont pas spécifiquement liées à un ADNc sont retirées du mélange comme précédemment décrit. Ces complexes sont ensuite dénaturés puis les sondes primaires 28 qui représentent une empreinte des ADNc sont récupérées pour être analysées.

Dans le cas où les sondes primaires sont de même taille et de même masse, l'analyse peut être effectuée par hybridation des sondes primaires à des sondes secondaires immobilisées sur un support puis détection des sondes primaires par mesure de variation d'impédance, par exemple.

Si les sondes primaires ont des tailles et/ou des masses différentes, l'analyse peut être réalisée par électrophorèse capillaire, par chromatographie, ou par spectrométrie de masse.

Dans un mode de réalisation particulier, chaque type de sonde primaire est formé par un polynucléotide (ou une partie polynucléotidique) capable de s'hybrider avec un acide nucléique particulier mais résistant aux actions des nucléases.

Ce polynucléotide peut comprendre ou consister à une molécule d'acide nucléique, une molécule de PNA (Peptide Nucleic Acid), ou un composé mixte acide nucléique / PNA (figures 18a et 18b), la jonction entre les deux molécules étant par exemple obtenue par une liaison PNA cooP(o3) Acide nucléique. Le polynucléotide peut également comprendre un acide nucléique dont la séquence a été modifiée, le premier ou le dernier résidu étant par exemple attaché par une liaison thio-diester, ou une liaison phospho-thioate, à la place d'une liaison phospho-diester (figure 18c).

La séquence du polynucléotide de chaque type de sonde primaire est spécifique d'une séquence d'acide nucléique présente dans le mélange de cibles moléculaires à analyser (ou, lorsque les cibles ne sont pas des acides nucléiques, elle peut être destinée à être reconnue spécifiquement par une sonde secondaire). La taille et/ou la masse des acides nucléiques présents dans le mélange de cibles moléculaires pourront être déterminées à partir de la connaissance des séquences et des tailles des polynucléotides qui auront hybridé aux cibles moléculaires.

Les sondes primaires peuvent également être fixées à des particules magnétiques, comme précédemment décrit. Dans le cas où les polynucléotides sont formés de composés mixtes acide nucléique / PNA, l'extrémité PNA ou thiol restera avantageusement libre de manière à protéger le polymère de toute dégradation enzymatique. La quantité de polynucléotides fixés sur des particules magnétiques peut être supérieure à la quantité supposée de cibles moléculaires présente dans le mélange complexe. Les séquences des polynucléotides sont préférentiellement équimolaires.

Les sondes primaires peuvent être constituées de PNA identifiables par leur masse, leur taille ou par des modifications chimiques (méthylation, éthylation, de leurs bases).

Le mélange de cibles moléculaires à analyser est mis en contact avec les particules fonctionnalisées par les sondes primaires de façon à ce que les cibles moléculaires s'hybrident avec les parties complémentaires des sondes primaires. Selon un mode de réalisation particulier de l'invention, les complexes particule/sonde primaire/cible sont ensuite traités par des « endo » et / ou des « exo » nucléases qui ne dégradent que les portions simple brin des acides nucléiques, telles que les nucléase NF sp. Toutes les séquences d'acide nucléique simple brin sont alors dégradées. Les complexes particule/sonde primaire/cible sont alors isolés des enzymes et rincés (les enzymes peuvent être inactivées par la chaleur ou un inactivateur), puis les complexes sont dénaturés pour libérer en solution les cibles moléculaires, chaque cible moléculaire étant restreinte à la taille de la sonde primaire à laquelle elle s'est hybridée.

Chaque cible moléculaire est alors identifiable par sa taille et / ou sa masse. Si une sonde primaire a subi une mutation autorisant quand même son hybridation avec une cible moléculaire (et à condition que la mutation ne corresponde pas à une extrémité de la sonde), la masse observée pour cette cible moléculaire est modifiée en conséquence ce qui permet d'identifier la ou les mutations. Le mélange complexe de cibles moléculaires peut être analysé en spectrométrie de masse ou en chromatographie pour identifier et quantifier chacune des cibles moléculaires.

Les particules magnétiques fonctionnalisées peuvent être à nouveau utilisées pour l'analyse d'un autre mélange complexe de cibles moléculaires.

Ce principe peut être décliné avec des sondes primaires constituées de simples séquences d'acide nucléique, mais le système (particule-acide nucléique) est dans ce cas plus fragile et ne peut être utilisé qu'une seule fois.

Lorsque les cibles moléculaires sont des acides nucléiques, chaque type de sonde primaire composant le jeu de sondes primaires est spécifique et complémentaire d'un type de cible parmi les cibles moléculaires à analyser.

Dans le cadre du procédé selon l'invention, appliqué à des cibles moléculaires qui sont des acides nucléiques, les sondes primaires apportées sous forme de mélange stoechiométrique, s'hybrident dans des conditions stringentes aux molécules cibles d'acide nucléique. Ces conditions sont indépendantes de la taille des sondes.

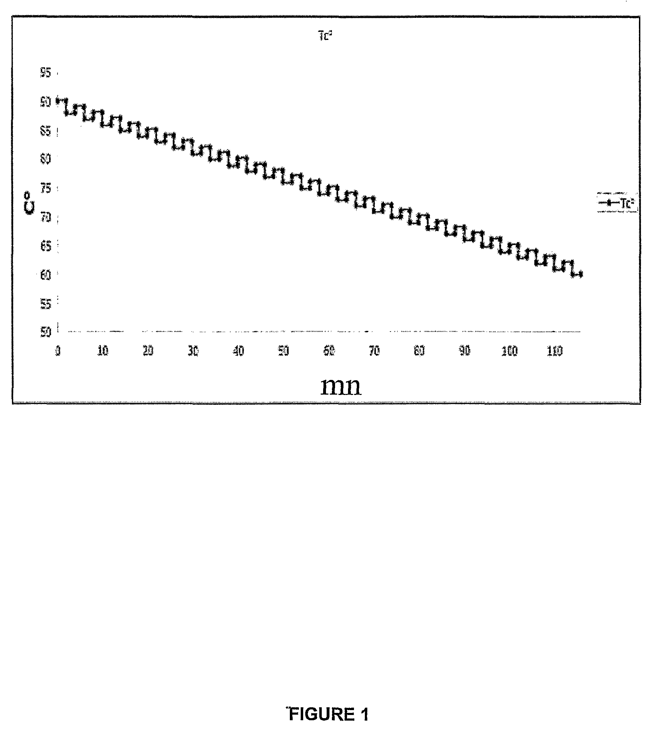

L'hybridation est réalisée dans des conditions thermodynamiques, c'est-à-dire avec un gradient de température allant de 90° à 60°c, avec des paliers de refroidissements et de réchauffements successifs (figure 1).

Pour minimiser les hybridations non spécifiques, lorsque les cibles et les sondes sont des molécules d'acide nucléique, les cibles et sondes sont hybridées en présence de petits polymères nucléotidiques (X)n où X représente A, T, G ou C et n varie de 3 à 7 nucléotides, formant toutes les combinaisons de séquences possibles de n nucléotides, ajoutés au mélange.

Les petits polymères hybrident aux séquences sondes et/ou cibles dans un premier temps. Les sondes reconnaissant une cible spécifique déplacent ensuite les petits polymères pour former des hybrides avec lesdites cibles.

Dans un exemple spécifique, les sondes du jeu de sondes primaires ont toutes une taille homogène, en particulier identique, et ont chacune une séquence déterminée. Les variations de mesure engendrées par chaque sonde sont donc calibrées.

Par exemple, toutes les sondes du jeu de sondes primaires ont une taille identique choisie pour comporter de 20 à 150 nucléotides, par exemple 30, 40, 50 nucléotides ou toute taille comprise dans les intervalles formés par ces limites.

Dans le cadre de l'analyse selon l'étape d) la détection des sondes primaires éluées après avoir formé des complexes sonde-cible par hybridation des séquences nucléotidiques peut être réalisée après la mise en oeuvre des étapes suivantes :
e. la mise en contact des sondes primaires séparées et récupérées (éluées) à l'étape c) avec des sondes secondaires polynucléotidiques de types différents, chaque type de sonde secondaire étant susceptible de se lier par hybridation spécifique à un type de sonde primaire,
f.. la détermination des cibles moléculaires à partir de la détection, et/ou de la récupération et/ou de l'analyse des sondes primaires hybridées aux sondes secondaires.

Ces étapes peuvent être notamment mises en oeuvre au moyen d'une matrice de sondes organisées en spots, telle que décrite par exemple dans l'électro-puce 2D décrite ci-après.

La séparation par déshybridation, des sondes primaires et des cibles moléculaires d'acide nucléique peut être obtenue par élévation de la température.

L'étape de détection et/ou de récupération et/ou d'analyse des sondes primaires récupérées après leur liaison spécifique avec les cibles moléculaires, peut être réalisée en faisant circuler les sondes primaires au contact des sondes secondaires immobilisées sur un support (par exemple une puce), par diffusion simple des sondes primaires ou au moyen d'une circulation active, par exemple par application de potentiels électriques. L'application de potentiels électriques est réalisée par au moins un réseau d'électrodes.

Lorsque la détection met en jeu l'utilisation de sondes secondaires, on recherche les hybrides formés entre les sondes primaires et les sondes secondaires, par exemple par la mesure de la variation d'impédance liée à la liaison, notamment à l'hybridation des sondes primaires et secondaires.
La mesure de variation d'impédance peut être effectuée au moyen d'un dispositif comprenant une puce (électro-puce 2D) telle que décrite ci-après.

Dans le cas des molécules d'acide nucléique, en particulier d'ADN, il est souhaitable de contraindre au maximum la conformation des molécules, afin de minimiser les artéfacts de mesure dus à la courbure de l'acide nucléique, notamment de l'ADN et aux hybridations intramoléculaires, qui entraînent des variations d'impédance du même ordre que pour une hybridation. Une solution pour contraindre l'ADN consiste à l'adsorber sur une électrode d'ITO par un peignage moléculaire. En particulier les sondes d'acide nucléique, notamment d'ADN, sont traitées comme indiqué ci-dessus.

Pour faciliter le peignage moléculaire des acides nucléiques et leur adsorption à la surface des électrodes, les électrodes d'ITO (ou réalisées dans un autre matériau conducteur) peuvent être recouvertes d'une couche de polyimide (par exemple Kapton®) (de 10 à 100nm environ d'épaisseur). La couche de Kapton® peut éventuellement être striée pour mieux orienter les molécules d'acides nucléiques.

Les molécules étirées sur l'électrode ne peuvent plus se couder ou s'hybrider contre elles-mêmes. En revanche, elles gardent la faculté de s'hybrider avec une séquence complémentaire en solution.

Une autre solution, consiste à tendre la molécule sonde (sonde secondaire) entre une électrode du jeu fonctionnalisé et une électrode du jeu non fonctionnalisé, en la fixant par ses extrémités aux électrodes. Les oligonucléotides sondes utilisés, sont fonctionnalisés aux deux extrémités 5' et 3', la fonction choisie en 5' étant différente de celle en 3'. Les deux types de fonction ont une activation et/ou une catalyse différente. Il est par exemple choisi des fonctions Hs, NH2 en 5' avec une activation chimique ou lumineuse et une fonction pyrole en 3' avec une catalyse électrique. La distance entre les jeux d'électrodes fonctionnalisées et non fonctionnalisées est choisie telle que, les molécules sont étirées, sans pouvoir adopter de courbure particulière ou réaliser une hybridation intramoléculaire.

Une alternative consiste à fonctionnaliser l'extrémité 5' de la sonde pour la greffer sur l'électrode fonctionnalisée et à leur ajouter une courte séquence (10 à 20 bases) en 3' (attache 3'). La séquence de l'attache 3' est choisie spécifiquement, pour qu'il n'y ait pas hybridation avec les cibles. Une séquence complémentaire à l'attache 3' est greffée sur l'électrode non fonctionnalisée en vis-à-vis. Les sondes sont accrochées à l'électrode du jeu fonctionnalisé par leur extrémité 5' grâce à une liaison chimique et à l'électrode non fonctionnalisée par l'extrémité 3' grâce au duplex, de 10 à 20 paires de bases, formé entre l'attache et sa séquence complémentaire greffée sur l'électrode. Les sondes sont ainsi tendues entre les deux électrodes ce qui minimise les hybridations intra-moléculaires et la courbure.

La puce à électrodes avec les sondes greffées peut être hybridée passivement par diffusion simple des sondes cibles (sondes primaires éluées formant l'empreinte moléculaire des cibles moléculaires du mélange) au niveau de chaque spot, comme cela est réalisé pour les bio puces actuelles. Il sera toutefois préféré une hybridation active.

Une fois la puce hybridée, la variation d'impédance de chaque spot permet de déterminer la quantité de cibles fixées. L'intersection entre une électrode supérieure (fonctionnalisée) et la projection (dans le plan supérieur) d'une électrode inférieure (non-fonctionnalisée) est unique et correspond à un seul spot. La mesure d'impédance est réalisée spot à spot en appliquant successivement une différence de potentiel électrique et un courant électrique à tous les couples possibles d'électrodes formés par une électrode inférieure et une électrode supérieure.

Des défauts locaux de structure dans les matériaux peuvent entraîner des résistances sporadiques d'une électrode à l'autre, causant des artéfacts sur la lecture de l'impédance. Par ailleurs, la fonctionnalisation des électrodes pour greffer les molécules d'ADN peut perturber la mesure. L'utilisation des alliages d'oxyde d'étain et de zinc tels que : ITO, ATO, FTO, ZNO (éventuellement recouverts d'une couche de polyimide ou Kapton®), permet de minimiser ces problèmes. En effet, les méthodes de dépôt de ces alliages sont très standardisées et reproductibles, ce qui permet d'obtenir des électrodes avec très peu de défauts.

Les molécules libres sont éliminées grâce à l'action des champs électriques produits par les systèmes d'électrodes. L'ensemble des mesures peut être fait au cours de réactions d'hybridation ou de complexation grâce au jeu d'électrodes qui permettent de manipuler l'ensemble des molécules chargées.

Par cette approche, Il est possible d'évaluer le nombre de molécules de sondes secondaires composant le spot et le nombre de sondes primaires qui vient se lier spécifiquement, en particulier s'hybrider. Ces deux mesures permettent d'établir les concentrations réelles des cibles moléculaires que l'on avait initialement en solution. En effet, la taille des sondes primaires et secondaires étant strictement calibrée et identique, leur séquence étant connue, Il devient possible de normaliser la mesure effectuée, par exemple par la mesure de fluorescence, afin qu'elle soit quantitative.

Il est possible de standardiser les mesures en réalisant une puce présentant des sondes secondaires de taille identique, éventuellement des sondes PNA. La taille des sondes primaires sera normalisée pour correspondre strictement à celle des sondes secondaires après hybridation sur la puce, par un traitement avec une nucléase dégradant les acides nucléiques simple brin, comme décrit précédemment.

Les systèmes sont réalisés avec des électrodes fonctionnalisées en matériaux transparents tels que ITO. Ceci permet d'éviter la fluorescence parasite provenant des électrodes en or. Parmi les matériaux possibles pour réaliser l'ensemble de la connectique on citera les alliages transparents dans le visible tels que, ITO (oxyde d'indium et oxyde d'étain), ATO, FTO, ZNO ou tout autre alliage équivalent.... Ces alliages étant très électrophiles, ils devront subir une procédure d'isolation (exemple 1).

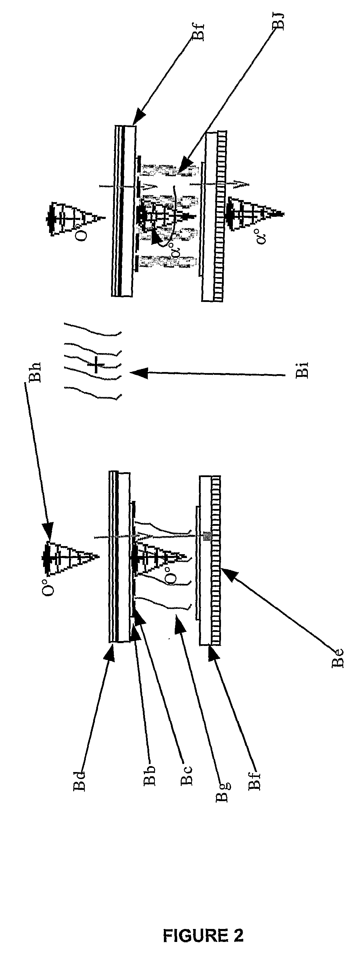

Un autre mode de détection des hybrides sonde primaire-sonde secondaire formés consiste à utiliser des filtres de lumière polarisée placés de part et d'autre de la puce comprenant les hybrides (figure 2).

Un duplex ADN organisé en hélice B ou en hélice A est un objet chiral qui a la propriété de dévier le plan de polarisation (oscillation) de la lumière. L'angle α de déviation de la lumière dépend essentiellement du nombre de paires de bases constituant la double-hélice d'ADN. La déviation d'un angle α est perdue quand la double hélice est détruite donc quand le double brin d'ADN se déshybride.

Cette propriété peut être utilisée pour quantifier le taux d'hybridation de chaque spot de sonde dans les dispositifs d'électro-puces (Electro_Chip) décrits plus loin.

Les électrodes des électro-puces en ITO sont transparentes, et peuvent être réalisées sur des lames de verre, de quartz ou de tout autre matériau transparent.

D'une manière générale, une électro-puces est constituée d'un plan transparent sur lequel sont déposées des électrodes en ITO éventuellement recouvertes d'une couche de poly-imide. Sur chaque électrode en ITO sont greffées des sondes organisées en spots, telles que chaque spot se trouve dans le vide d'un capillaire du réseau de capillaires associés. En vis-à-vis de chaque électrode greffée de spots se trouve une électrode déposée sur un autre support transparent. Un rayon de lumière polarisé avec un angle relatif à 0° peut donc traverser le premier support transparent et son électrode, pour arriver au niveau d'un spot. La lumière subit alors une rotation d'un angle de α° par les molécules d'ADN double brin (les molécules hybridées) et ressort à travers l'électrode et le plan transparent en vis-à-vis. La quantité de lumière subissant une déviation de α° est strictement proportionnelle au nombre d'hybrides formant des doubles hélices présentes dans le spot, et donc strictement proportionnelle à la quantité de cibles hybridées sur les sondes.

La lumière déviée de l'angle a α° est analysée en sortie après qu'elle ait traversé la deuxième électrode et le deuxième plan transparent.

Pour obtenir de la lumière polarisée à 0° (origine arbitraire qui peut être modifiée pour une autre origine déterminée), un filtre polarisé à 0° est disposé sur la face externe du support transparent des électrodes greffées. Un second filtre polarisé orienté à α° est disposé sur la face extérieure de l'autre support transparent d'électrodes en vis-à-vis (cf. schéma). L'émission de lumière est obtenue grâce à un laser dans le visible ou à toute autre source de lumière blanche.

D'une manière générale, toute lumière pour laquelle le montage est transparent, et que les sondes et les cibles n'absorbent pas peut être utilisée. Ce procédé de détection peut être appliqué à des puces classiques à acide nucléique en les confinant entre deux filtres plans polarisants dont les plans de polarisation sont décalés entre eux d'un angle α°.

Les deux filtres polarisants peuvent être mobiles l'un par rapport à l'autre afin d'augmenter l'efficacité de la détection, en modifiant l'angle entre les deux plans de polarisation. Cette modification d'angle permet de mesurer différentes déviations de la lumière et ainsi d'évaluer l'homogénéité des séquences des cibles (sonde primaire-sonde secondaire) hybridées sur un spot. Il est ainsi possible de déterminer le polymorphisme des cibles.

En effet, des différences de séquence entre la cible (ici sonde primaire) et la sonde (ici sonde secondaire) entraînent des mésappariements qui plient par endroits la double hélice formée, modifiant ainsi la chiralité de la molécule. De ce fait, l'angle de déviation de la lumière est modifié en fonction de chaque chiralité. La mesure à chaque angle informe sur chacune des populations.

Dans un autre exemple de réalisation spécifique, la détection de la liaison par hybridation entre les sondes primaires et les sondes secondaires peut être faite par détection de la fluorescence d'un ligand des hybrides double brin formés entre les sondes primaires et les sondes secondaires ou d'un ligand des acides nucléiques simple brin. On utilise au moins un détecteur pour mesurer l'intensité de fluorescence liée à l'hybridation des sondes primaires et secondaires.

Sans que les sondes primaires soient marquées, les taux de complexes sonde primaire / sonde secondaire formés sur la puce peuvent être évalués en utilisant des ligands fluorescents. Pour les puces à ADN, on peut citer les acridines et notamment l'acridine orange qui est un intercalant de l'ADN chargé positivement. L'acridine orange permet de marquer différemment les ADN simples brin ou le duplex double brins.

Dans un autre exemple spécifique, la détection des sondes primaires hybridées avec les sondes secondaires est faite par SPR adapté à la puce électro-puce type 2D décrite ci-après. On utilise au moins un détecteur pour mesurer par SPR l'hybridation des sondes primaires et secondaires.

Les cibles constituées par les complexes sonde primaire/sonde secondaire peuvent être quantifiées directement sur le réseau d'électrodes fonctionnalisées. Un prisme en trièdre est adapté au dos de chaque électrode ligne du réseau d'électrodes fonctionnalisées pour pouvoir réaliser la mesure de SPR.

Un autre aspect décrit dans la demande concerne la réalisation du procédé décrit ci-dessus, pour l'analyse, et la quantification de cibles moléculaires d'un mélange complexe lorsque ces cibles sont des polypeptides ou d'autres cibles pouvant former des complexes spécifiques de type récepteur-ligand.

Les modes de réalisation des étapes a) et b) du procédé décrit plus haut en particulier pour la détection de cibles d'acides nucléiques sont en principe transposables. Ainsi, les cibles polypeptidiques du mélange complexe à analyser peuvent être fixées sur des particules (magnétiques ou non, d'un ou plusieurs types), et être mises en contact avec les sondes primaires en solution. Par exemple on aura recours à des billes magnétiques recouvertes d'ITO. Si des protéines de différents types sont analysées on peut les fixer sur différents types de particules.

Comme indiqué plus haut, le jeu de sondes primaires utilisé met en oeuvre des sondes comprenant une partie polypeptidique et une partie polynucléotidique. La quantification des cibles moléculaires spécifiquement liées à des sondes du jeu de sondes primaires est réalisée après récupération des parties polynucléotidiques des sondes des complexes sonde-cible.

Les parties polypeptidiques et les parties polynucléotidiques de chaque type de sonde étant spécifiques d'un type de cible uniquement, l'identification et la quantification des parties polynucléotidiques des sondes signe la présence et la quantité des cibles moléculaires.

Suivant les modalités décrites plus haut, les parties polynucléotidiques des sondes ont des masses et/ou des tailles différentes et des séquences identiques ou différentes ou, des séquences différentes et des tailles homogènes voire identiques entre les sondes. Les différences en question permettent de choisir un système de détection approprié. Ainsi, les parties polynucléotidiques peuvent être détectées par exemple par tout moyen capable de révéler leurs différences de séquences (hybridation avec des sondes secondaires) de taille (électrophorèse, chromatographie) ou de masse (spectrométrie de masse).

Lorsque les cibles sont de nature protéique, les sondes primaires sont des composés ayant une capacité à se lier spécifiquement à ces cibles, par exemple des anticorps ou des fragments fonctionnels d'anticorps comprenant ou constitués par le site de liaison aux antigènes, ou des récepteurs ayant une affinité pour les protéines cibles.

Dans un exemple spécifique, les sondes primaires comprennent une partie constituée par un polynucléotide fixé sur la partie des susdits composés ayant une affinité spécifique pour les protéines cibles. Ce polynucléotide constitue un marqueur au sens d'un identifiant (encore appelé tag) spécifique de chaque composé. De tels polynucléotides, sont illustrés dans les exemples. Leur utilisation permet de détecter les sondes primaires séparées après qu'elles aient lié les protéines.

Dans cet aspect de l'invention consistant à détecter des cibles moléculaires polypeptidiques, par exemple dans un protéome, l'étape a) du procédé selon l'invention comprend par exemple la mise en contact du mélange susceptible de comprendre des protéines à analyser conformément à la présente description, avec un jeu de sondes primaires de types différents, constitué par un jeu d'anticorps monovalents ou de fragments monovalents d'anticorps comprenant ou constitués d'un seul site de liaison antigénique par exemple des fragments Fab ou toute molécule formée à partir de tout ou partie des chaines variables dès lors qu'elle comprend un seul site de reconnaissance d'un antigène) liés chacun avec une séquence d'acide nucléique spécifique appelée séquence tag, chaque type d'anticorps ou de fragment d'anticorps composant le jeu étant susceptible de se lier par liaison spécifique à un seul type de protéines à analyser, dans des conditions permettant la liaison spécifique entre lesdites protéines et lesdits anticorps ou fragments d'anticorps.

Lorsque les cibles sont de nature protéique, la séparation à l'étape c) des sondes primaires et des cibles des complexes de liaison formés peut être obtenue par des méthodes enzymatiques connues de l'homme du métier.
Dans un exemple spécifique, l'étape c) du procédé selon l'invention comprend la séparation des protéines et des anticorps ou des fragments d'anticorps liés par liaison spécifique, puis la séparation de chaque séquence tag de l'anticorps ou fragment d'anticorps qui lui correspond, de façon à récupérer le jeu de séquences tag représentant une empreinte des cibles retenues parmi les protéines à analyser.

L'étape d) du procédé selon l'invention comprend la détermination des protéines à partir de la détection, et/ou de la récupération et/ou de l'analyse des séquences tag discriminables par leur séquence les tags ayant alors une taille identique, ou au contraire par leurs tailles et/ou leurs masses différentes.

Dans un exemple spécifique, lorsque les tags ont des séquences différentes mais des tailles identiques, leur détection est réalisée selon les mêmes moyens que ceux décrits plus haut pour les cibles moléculaires constituées d'acides nucléiques. En particulier on fait appel à l'utilisation de sondes secondaires spécifiques des tags.

Les caractéristiques des tags sont alors définies comme décrit ci-dessus pour les sondes primaires constituées de polynucléotides destinés à hybrider avec des cibles nucléotidiques.

Lorsque le jeu de sondes primaires est constitué de sondes dont les parties polynucléotidiques sont différentes par la masse, alors le jeu peut être constitué de telle sorte qu'il comprenne plus de 10, en particulier de plus de 50 plus de 100 ou plus de 1000, voire plus de 10 000 ou de 20 000 sondes dont les polynucléotides tag ont tous des masses différentes, par exemple 30, 40, 50, 100, 500, 1000 2000, 5000 ou plus, ou un nombre de sondes compris dans un intervalle formé par les valeurs comprises entre deux de ces limites.

La détection des tags (une sonde étant identifiée par un tag et un seul) peut être réalisée par tout système de détection de masse par exemple spectrométrie de masse.

Dans un autre exemple spécifique, on analyse les séquences tag (une sonde étant identifiée par un tag et un seul) au moyen de leur différence de taille. On peut alors avoir recours à une détection par électrophorèse ou par chromatographie.

Ces séquences tag peuvent en outre être chimiquement modifiées par des groupements méthyl ou éthyl ajoutés sur les bases des résidus, de manière à ce qu'il soit possible d'identifier les sondes primaires par leurs masses ou par leurs modifications chimiques, conformément à ce qui a été décrit plus haut.

Il est possible par exemple d'identifier ces séquences tag et en général les polynucléotides des sondes primaires par leurs temps de rétention en chromatographie en phase gazeuse ou liquide et de les quantifier par absorbance optique, fluorescence naturelle des acides nucléiques, ou en dosant le phosphore présent dans un plasma généré en sortie du chromatographe (par fluorescence du phosphore ou spectrométrie de masse).

Le jeu de sondes primaires est alors constitué de telle sorte qu'il y ait plus de 10, en particulier de plus de 50 plus de 100 ou plus de 1000, voire plus de 10 000 ou de 20 000 sondes dont les polynucléotides tag ont tous des tailles différentes, par exemple 30, 40, 50, 100, 500, 1000 2000, 5000 ou plus, ou un nombre de sondes compris dans un intervalle formé par les valeurs comprises entre deux de ces limites.

L'invention a aussi pour objet un dispositif pour la mise en oeuvre du procédé décrit.

Un dispositif selon l'invention comprend par exemple :
- éventuellement un ensemble de particules, par exemple de particules magnétiques, pouvant fixer de manière forte les cibles moléculaires d'acides nucléiques et/ou de protéines du mélange à analyser ;
- un jeu de sondes primaires de types différents conforme à la description donnée dans la présente demande, sous forme d'un mélange stoechiométrique, en solution, lesdites sondes étant constituées par un polynucléotide ou comprenant un polynucléotide spécifique de chaque sonde, chaque type de sonde primaire composant le jeu est susceptible de se lier par liaison spécifique à un unique type de cible moléculaires à analyser, lorsque lesdites sondes primaires et lesdites cibles moléculaires sont mises en contact en solution, la séquence nucléotidique du polynucléotide de chaque type de sonde étant connue et étant différente des séquences nucléotidiques des polynucléotides des autres types de sonde primaire, et le cas échéant,
- des moyens pour séparer et des moyens pour récupérer les sondes primaires et les cibles moléculaires liées par liaison spécifique, de façon à obtenir un jeu de sondes primaires représentant une empreinte des cibles moléculaires à analyser et/ou le cas échéant,
- un jeu de sondes secondaires capables de reconnaître spécifiquement les parties polynucléotidiques des sondes primaires du jeu de sondes primaires et/ou le cas échéant,
- une puce (par exemple de type électro-puce 2D) à oligonucléotides où les sondes oligonucléotidiques (composant le jeu de sondes secondaires) de chaque spot comprennent une séquence complémentaire d'un type de séquence de sonde primaire ou de chaque tag.

Dans le cadre de l'analyse d'acides nucléiques, les moyens pour séparer, notamment déshybrider les sondes primaires et les cibles moléculaires peuvent être des moyens capables d'augmenter la température, de manière à séparer les deux brins de l'hybride.
Dans le cadre de l'analyse de protéines, les moyens pour séparer les sondes primaires des cibles moléculaires peuvent être notamment des réactifs pour réaliser des séparations enzymatiques.

Les moyens pour récupérer les sondes primaires séparées des cibles moléculaires peuvent comprendre des particules magnétiques sur lesquelles sont immobilisées les cibles et un moyen capable de générer un champ magnétique au sein de la solution comprenant lesdites sondes primaires et lesdites cibles.

Les moyens pour récupérer les sondes primaires séparées des cibles moléculaires peuvent comprendre des particules sur lesquelles sont immobilisées les cibles et un moyen de provoquer une centrifugation au sein de la solution comprenant lesdites sondes primaires et lesdites cibles.

Le procédé peut être décliné pour être utilisé pour le dosage de différentes protéines et de leurs modifications dans un mélange provenant par exemple d'un extrait cellulaire.

Lorsque les cibles moléculaires sont des protéines, le dispositif comprend à titre illustratif:
1) Un ensemble de particules (notamment magnétiquesou une surface) pouvant fixer de manière forte les protéines du mélange à analyser comprenant les cibles. Typiquement on citera les billes ou les membranes de polystyrène, nylon, nitrocellulose...
2) Un mélange stoechiométrique de sondes primaires constituées par exemple d'anticorps monovalents ou de fragments d'anticorps comprenant un seul site de fixation de l'antigène, où chaque type d'anticorps est lié de façon forte (par exemple covalente) avec une séquence spécifique d'acide nucléique ou polynucléotide (la séquence tag). La séquence tag se décompose par exemple en une ou deux séquences génériques susceptibles d'être coupées spécifiquement et en une séquence unique et spécifique pour chaque type d'anticorps ou de fragments d'anticorps ci-dessus décrits ; et éventuellement,
3) Une puce à oligonucléotides, où les sondes oligonucléotidiques (composant le jeu de sondes secondaires) de chaque spot sont constituées d'une séquence complémentaire de la partie spécifique de la séquence tag d'un des types d'anticorps du mélange stoechiométrique d'anticorps.

Pour une analyse quantitative, il est indispensable que chaque sonde comprenne un seul site de fixation à une cible et un seul polynucléotide tag. Les anticorps ou leurs fragments tels que les fragments Fab conviennent puisqu'ils ont un site de fixation qui peut être associé à un tag.

À titre d'exemple, à partir d'un extrait cellulaire, les protéines sont fixées sur des particules magnétiques de polystyrène. Le polystyrène a la faculté d'adsorber de façon durable les protéines, notamment les protéines hydrophobes. Un excès de billes est utilisé par rapport à la concentration des protéines pour éviter toute saturation des billes, limitant ainsi les encombrements stériques. Selon l'étude souhaitée, les protéines sont ou non dénaturées. Les billes et les protéines fixées sont alors :
1) précipitées par un champ magnétique,
2) isolées du surnageant,
3) rincées et reprises dans un tampon salin.

Les billes sont ensuite saturées par des protéines inertes pour le système étudié. Par exemple, l'albumine sérique de bovin (pour les études sur des molécules cibles qui ne sont pas d'origine bovine), des protéines de petites tailles exogènes à l'espèce étudiée (comme l'inhibiteur de Kunixt) ou encore des acides aminés à chaîne aliphatique (comme la leucine) sont utilisés. Les étapes 1 à 3 sont de nouveau réalisées. Puis l'ensemble, billes saturées - protéines fixées est mis à complexer avec le mélange équimolaire d'anticorps munis des séquences tag. Les anticorps se fixent spécifiquement sur leurs protéines cibles immobilisées sur les billes. La quantité de chaque type d'anticorps fixé est proportionnelle à la quantité de chaque type de protéines adsorbées sur les billes.

Pour diminuer l'encombrement stérique, mais avant tout pour permettre la quantification, les anticorps doivent être monovalents où doivent être substitués par des fragments monovalents d'anticorps (ou hémi-anticorps) par exemple obtenus par une digestion enzymatique, notamment par la papaïne, ou encore des fragments synthétiques ou recombinants. Chaque hémi-anticorps est « tagué » par une séquence d'acide nucléique. Les étapes 1 à 3 sont de nouveau réalisées. Les anticorps (ou hémi anticorps) qui ont réagi sont donc isolés et séparés de ceux qui n'ont pas réagi. Le tag d'acides nucléiques est coupé grâce à la séquence de coupure introduite. Il peut s'agir par exemple d'une séquence palindromique ou de la séquence cible d'un abzyme... Dans le cas d'une séquence palindromique, deux solutions particulières peuvent être citées :
- La séquence palindromique est introduite entre l'anticorps et la séquence tag spécifique. Pour séparer la séquence tag de l'anticorps, il suffit d'introduire dans le milieu une séquence complémentaire de la séquence de coupure avec l'enzyme de restriction correspondante, ce qui permet de séparer l'anticorps de la séquence tag spécifique.
- Deux séquences de coupure sont introduites respectivement entre l'anticorps et la séquence tag spécifique, et à la fin de la séquence tag, de telle sorte que ces deux séquences soient complémentaires l'une de l'autre. En s'hybridant, elles forment le site de coupure spécifique pour une enzyme qui est ultérieurement introduite dans le milieu. Les séquences tags spécifiques sont alors libérées en solution.

Une fois séparées des anticorps, les séquences tags fournissent un mélange d'oligonucléotides en solution dont les quantités sont proportionnelles à celles des types des anticorps retenus sur les billes, et donc proportionnelles aux différentes protéines et à leurs modifications présentes dans l'extrait cellulaire analysé. Le mélange de nucléotides obtenu peut être analysé sur une puce à ADN classique ou une puce telle que décrite précédemment.

Dans le cas où le jeu de sondes primaires (comprenant une partie polypeptidique et un tag polynucléotidique) comprend un grand nombre de types différents de sondes primaires, il est nécessaire que les séquences tag soient de grande taille et comportent des modifications chimiques pour pouvoir les différencier. Cependant, les grandes tailles des séquences tag et leurs modifications peuvent gêner les liaisons entre les sondes primaires et les cibles moléculaires en masquant les sites de fixation. De plus, il est très difficile de greffer des séquences nucléotidiques de plus de 20 bases sur un anticorps ou un fragment Fab d'anticorps avec un bon rendement.

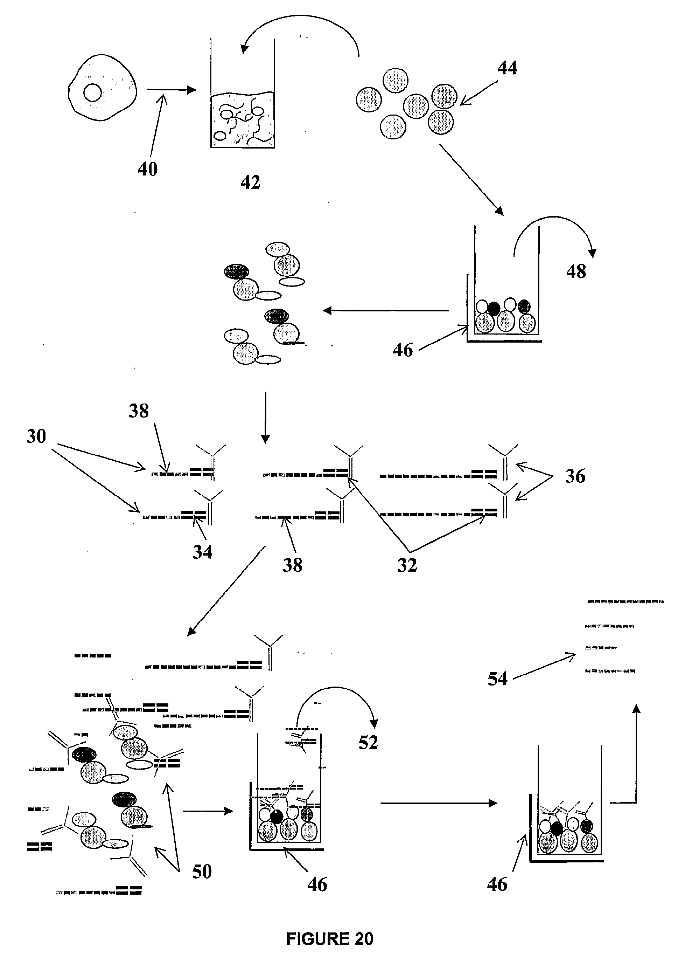

Pour contourner ce problème et comme représenté en figure 20, les séquences tag comprennent environ entre 10 et 20 bases (de préférence 18), ce qui permet de générer entre 4¹⁰ et 4²⁰ séquences différentes. Pour reconnaître les tag, on réalise un mélange de sondes équimolaires appelées « sonde - report » dans lequel chaque sonde - report est constituée d'une part de la séquence complémentaire au tag d'un type de sonde primaire, et d'autre part d'une séquence de taille variable ne reconnaissant aucune séquence tag. Chaque sonde - report est définie de telle sorte qu'elle est reconnue de manière univoque par sa taille et / ou sa masse, et / ou par ses propriétés chromatographiques, comme décrit précédemment.

La figure 20 illustre un exemple de réalisation du procédé selon l'invention dans lequel les cibles moléculaires du mélange sont des protéines représentatives d'un protéome, ces cibles moléculaires étant obtenues par une étape de lyse cellulaire 40.

Ces cibles moléculaires 42 sont mélangées à des particules 44 capables de fixer les cibles moléculaires comme précédemment décrit. Les particules sont immobilisées sur un support à l'aide d'un champ magnétique appliqué par un aimant 46 ou analogue pour pouvoir retirer les cibles moléculaires 48 qui ne se sont pas fixées sur une particule.

Les cibles moléculaires 42 sont ensuite mélangées aux sondes primaires 36 de manière à ce que les cibles moléculaires se lient spécifiquement aux sondes primaires pour former des complexes particule/cible moléculaire/sonde primaire/sonde report 50.

Après l'élimination des sondes primaires 52 non spécifiquement liées à une cible moléculaire, les complexes particule/cible moléculaire/sonde primaire/sonde - report sont isolés et dénaturés et les sonde report 54 passées en solution sont isolées des complexes particule/cible moléculaire/sonde primaire. La composition en sonde report de la solution représente une empreinte des cibles moléculaires retenues sur les particules, et la composition et la quantification du mélange sera réalisée par exemple par spectrométrie de masse, chromatographie, lecture sur puce, etc.

En variante, au lieu de mettre les sondes report en contact avec les sondes primaires, celles-ci peuvent être mises en contact avec les complexes particule/cible moléculaire/sonde primaire de sorte que les sonde - report s'hybrident avec les tags correspondants des sondes primaires et les saturent.

Les tags peuvent avantageusement être des molécules de PNA dont les séquences sont connues et qui sont attachées à leurs extrémités NH₂ aux extrémités COOH de fragments Fab d'anticorps par des liaisons peptidiques.
L'invention porte également sur un dispositif pour l'exécution de l'étape d) d'analyse des sondes primaires recueillies à l'étape c) du procédé d'analyse de cibles moléculaires selon l'invention, suivant ses différents modes de réalisation décrits dans les pages précédentes, comprenant :
- un réseau de capillaires permettant la circulation de sondes primaires,
- une matrice de sondes secondaires organisées en spots, la matrice étant disposée de telle sorte qu'elle est en contact avec le réseau de capillaires,
- un réseau d'électrodes, dites fonctionnalisées, sur lequel sont fixées des sondes secondaires, ce réseau étant disposé de telle sorte que chaque ligne de spot de la matrice de spots est fixée sur une des électrodes fonctionnalisées dudit réseau, et
- un réseau d'électrodes dites non fonctionnalisées, ce réseau étant disposé de telle sorte que le réseau de capillaires se situe entre les deux réseaux d'électrodes.

Le dispositif est mis en oeuvre après avoir effectué les étapes de liaison spécifique des cibles moléculaires avec les sondes primaires et après avoir séparé les sondes primaires dans les complexes formés réalisant l'empreinte des cibles moléculaires.

Un tel dispositif est par exemple une puce dite électro-puce 2D (Electro-chip2D), tel que décrit ci-après, dans lequel la puce comprend une matrice bi-dimensionnelle de sondes biologiques (jeu de sondes secondaires) organisées en spots, associée à au moins un jeu d'électrodes. La matrice est contrainte dans un réseau de capillaires parallèles connectés entre eux par deux réservoirs (un à chacune de leurs extrémités).

Par le terme « capillaire », il faut comprendre tout canal approprié pour permettre la circulation de fluides, d'un diamètre inférieur à 1 millimètre, de préférence compris entre 1 et 100 µm.

Les réseaux de capillaires sont par exemple creusés ou moulés dans des matériaux tels que la silice, le quartz, les plastiques (plexiglas par exemple), le PDMS, selon des techniques d'érosion à l'acide pour la silice ou d'usinage au laser pour les plastiques, connues de l'homme du métier.

Dans un mode de réalisation préféré, chaque réseau de capillaires est creusé dans l'épaisseur d'une plaque d'un matériau approprié.

De manière générale, les réseaux de capillaires peuvent être remplis de gel tel qu'un gel de polyacrylamide ou tout autre gel permettant de réguler et de contrôler la diffusion des sondes primaires lors de leur migration, notamment les gels liquides utilisés pour les électrophorèses capillaires.

Les sondes primaires peuvent être contenues dans une solution ou dans un fluide, ledit fluide ou ladite solution circulant dans les capillaires.

Dans un exemple spécifique, le dispositif comprend un capillaire transversal, dit canal transversal, dans lequel sont introduites les sondes primaires qui se sont hybridées avec les cibles, d'un diamètre de préférence compris entre 2 et 1000 µm. Le canal transversal supérieur est connecté au réseau de capillaires.

Le dispositif comprend aussi une matrice de sondes secondaires organisées en spots, où chaque spot est constitué d'un type de sonde moléculaire, par exemple un polymère d'acide nucléique dont la séquence est strictement complémentaire de la séquence d'une des sondes primaires contenues dans le jeu de sondes primaires.

La matrice de sondes secondaires peut être posée ou fixée sur le réseau de capillaires.
Le premier jeu d'électrodes fonctionnalisées possède des électrodes greffées de sondes secondaires organisées en spots, chaque sonde secondaire étant susceptible de retenir une sonde primaire spécifique, par liaison spécifique sonde/sonde.

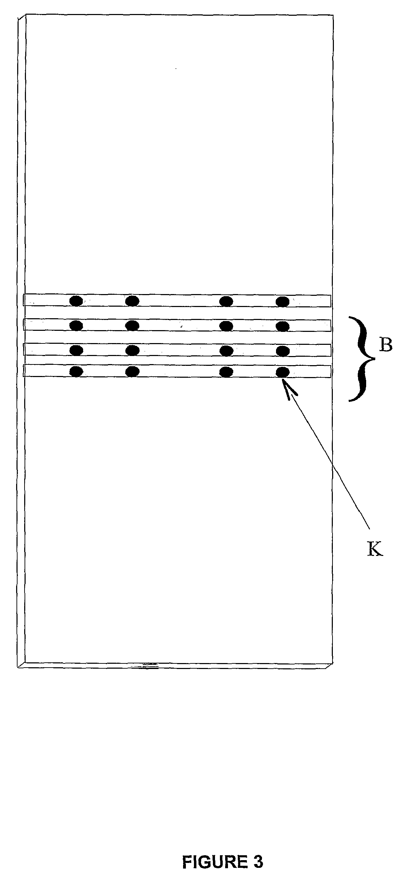

Chaque ligne de spots de la matrice est déposée sur une surface d'or ou d'ITO (ou tout autre métal ou alliage adéquat) délimitant une électrode, l'ensemble de la matrice étant alors constitué de n électrodes correspondant au nombre n de lignes, chaque électrode ligne comportant P spots de sondes greffés (figure 3).

L'ITO étant très électrophile, il est nécessaire d'isoler par un procédé d'encapsulation les parties des électrodes qui n'ont pas reçu de greffage de sondes, afin d'éviter tout accrochage non spécifique de cibles ou de sondes primaires. Par exemple, un film de poly-pyrrole peut être utilisé. Le film est réalisé en mettant les électrodes greffées sous tension en présence d'une solution de pyrrole. Le pyrrole polymérise spontanément sous l'action du courant et isole les parties libres des électrodes. Les électrodes peuvent également être saturées par un petit oligonucléotide qui ne peut pas s'hybrider de manière stable avec les cibles, par exemple un trimer ATA ou TAT...

Les dépôts de sondes (spot ou unité d'hybridation) sont effectués selon le cas sur les électrodes ou entre les électrodes.

Les électrodes sont gravées par couche mince sur un matériau isolant comme le verre, le poly-imide (par exemple le Kapton®), l'oxyde d'alumine.

Toute méthode appropriée pour accrocher les sondes secondaires peut être utilisée. A titre d'exemples, on citera en particulier le couplage non covalent de type biotine-avidine entre des sondes moléculaires couplées à la biotine et des billes fonctionnalisées avec l'avidine, telles que celles commercialisées par « DYNAL » (Dynal distributors worldwide, copyright 1996 Dynal AS - Techinical Handbook second edition).

D'une manière générale, tous les types de couplages, par exemple les liaisons chimiques, les interactions fortes, décrits pour les colonnes de chromatographie peuvent éventuellement convenir.

Par exemple, les interactions utilisées couramment dans les puces à ADN pour fixer les sondes d'acide nucléique, ou les puces à protéines pour fixer les sondes de polypeptides peuvent également être adaptées (interaction électrostatique lysine/acide nucléique, fixation silane, polymérisation du pyrole sur la surface de la loge, etc...), de même que les méthodes de synthèse *in situ* sur le support. Il pourra également être envisagé l'utilisation de nylon ou de la nitro-cellulose pour fixer les sondes de façon irréversible sur le support. La fixation peut être directe ou se faire par l'intermédiaire d'un pontage tel qu'un pont psoralène entre une particule de nylon et la sonde secondaire.

L'immobilisation des sondes secondaires sur l'électrode doit être suffisamment forte pour résister aux différents traitements appliqués et aux éventuels champs électriques utilisés pour manipuler les cibles.

Dans un exemple spécifique, la fixation des sondes secondaires sur les électrodes résiste aux différents traitements dénaturants les plus utilisés, ce qui permet de régénérer la puce après usage.

Le dispositif selon l'invention comprend en outre un réseau d'électrodes dites non fonctionnalisées, ce réseau étant disposé de telle sorte que le réseau de capillaires se situe entre les deux réseaux d'électrodes.

Ces électrodes sont dites non fonctionnalisées parce qu'elles ne sont pas greffées avec des sondes.

Dans un exemple spécifique, le dispositif permettant l'analyse des cibles moléculaires mesure les variations d'impédance liées à l'hybridation des sondes primaires et secondaires.

Dans un exemple spécifique, le dispositif mesure les variations de polarisation de la lumière liée à l'hybridation des sondes primaires et secondaires.

Dans un exemple spécifique, le dispositif comprend en outre un détecteur qui mesure l'intensité de fluorescence liée à l'hybridation des sondes primaires et secondaires.

Dans un exemple spécifique, le dispositif mesure par SPR l'hybridation des sondes primaires et secondaires.

Les électrodes peuvent être gravées en couche mince sur un matériau isolant.

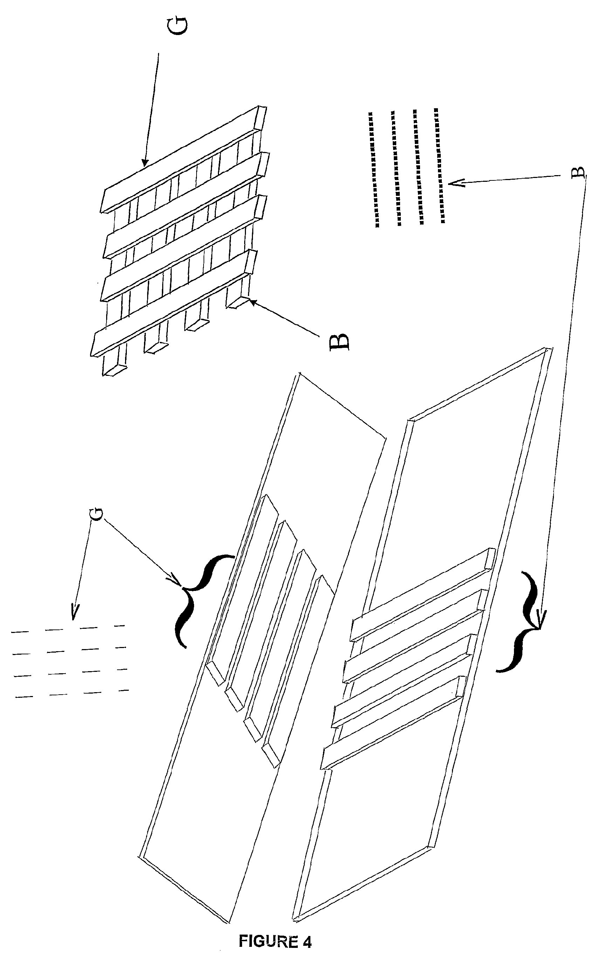

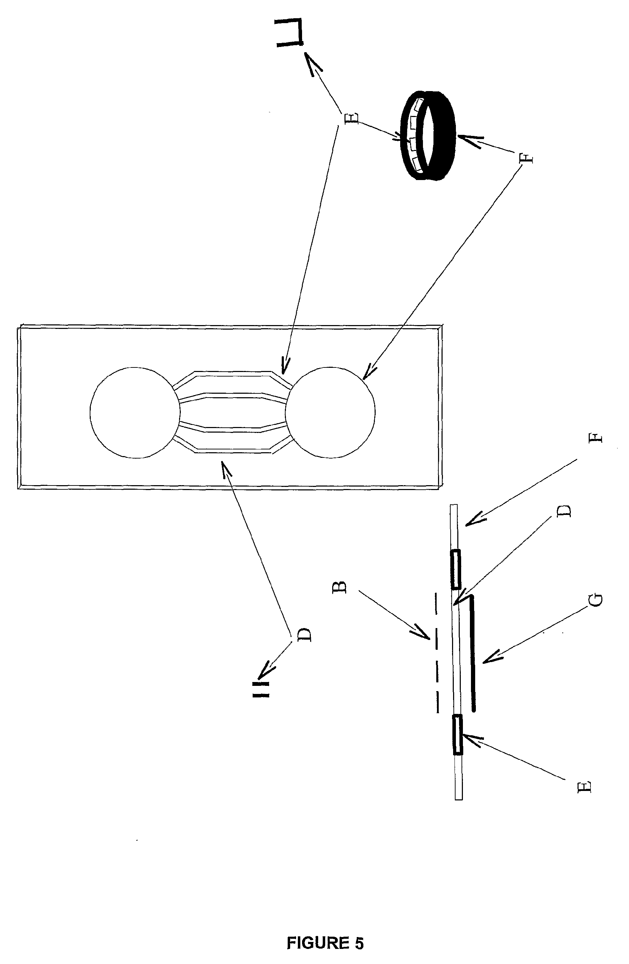

Les deux jeux d'électrodes sont disposés en vis-à-vis de part et d'autre d'un réseau de P capillaires parallèles, de telle sorte qu'il y ait un jeu d'électrodes au-dessus et l'autre en dessous (figure 4 et 5).

Dans un exemple spécifique, le jeu d'électrodes fonctionnalisées est situé au-dessus du réseau de capillaires et le jeu d'électrodes non fonctionnalisées est situé en dessous du réseau de capillaires.

Dans un exemple spécifique, le jeu d'électrodes fonctionnalisées est situé en dessous du réseau de capillaires et le jeu d'électrodes non fonctionnalisées est situé au-dessus du réseau de capillaires.

Chaque capillaire est perpendiculaire aux n électrodes du jeu d'électrodes fonctionnalisées et aux n électrodes du jeu d'électrodes non fonctionnalisées (figure 5, 6 et 7).

Chaque spot des électrodes fonctionnalisées est dans chaque capillaire du réseau de capillaires.

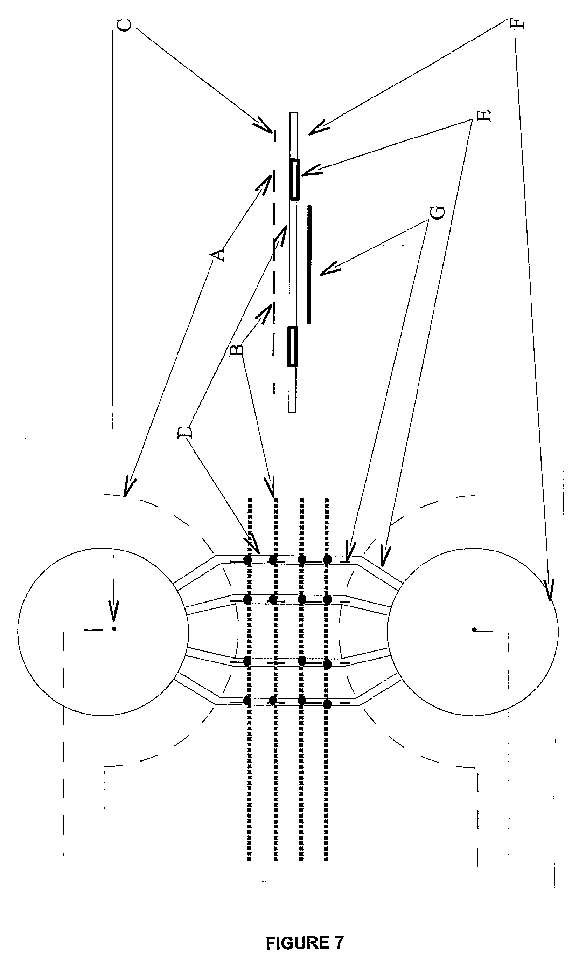

La construction est réalisée de manière telle que, le premier spot des n électrodes fonctionnalisées soit dans le premier capillaire du réseau de capillaires, le deuxième spot des n électrodes fonctionnalisées soit dans le deuxième capillaire du réseau de capillaires et ainsi de suite (figure 7). Un couple d'électrodes est constitué d'une électrode greffée de sondes organisées en spots au-dessus du réseau de capillaires et d'une électrode non fonctionnalisée en vis-à-vis en dessous du réseau de capillaires. Les électrodes peuvent être d'une très faible épaisseur pour permettre la détection par SPR.

A chaque extrémité du réseau de capillaires, les capillaires convergent vers un réservoir circulaire. Le réservoir comporte une électrode (électrode de réservoir), dans le même plan que celui du jeu d'électrodes fonctionnalisées (figure 7). Dans un exemple spécifique, cette électrode est circulaire. Dans un exemple spécifique, cette électrode est située au centre du plafond de chaque réservoir.

Le dispositif selon l'invention peut comprendre en outre, une première et une deuxième électrodes de liaison supplémentaires situées respectivement entre la première électrode de réservoir et la première électrode fonctionnalisée, et entre la deuxième électrode de réservoir et la dernière électrode fonctionnalisée, de telle sorte que les distances les plus courtes entre chaque électrode de liaison et l'électrode de réservoir correspondante soient identiques en tout point des électrodes.

L'électrode de liaison peut être courbe, sa courbure étant définie de façon telle que, les distances entre l'électrode de liaison et le centre du réservoir (électrode de réservoir) soient identiques en tout point de l'électrode.

Le second réservoir peut être formé par au moins un canal transversal, dit canal transversal inférieur, qui est relié en amont à l'ensemble des capillaires du réseau de capillaires et en aval au détecteur. Les sondes primaires qui se sont hybridées aux sondes secondaires peuvent être séparées et mises en circulation jusqu'au détecteur, en particulier par le canal transversal inférieur.

Dans le cadre d'une puce dans laquelle les sondes secondaires sont mises en circulation jusqu'au détecteur afin d'être analysées, il est nécessaire d'alterner une électrode fonctionnalisée et une électrode non fonctionnalisée dans la construction du réseau d'électrodes.

Le dispositif selon l'invention permet une liaison spécifique active, en particulier une hybridation active des sondes primaires qui se sont liées spécifiquement, notamment hybridées aux cibles moléculaires.

Dans un exemple spécifique, cette hybridation des sondes primaires circulant à travers la matrice de sondes secondaires est réalisée comme suit :
1) l'éluat des sondes primaires à analyser est introduit dans le premier réservoir. Un potentiel électrique est appliqué entre la première électrode courbe (positif) et l'électrode du premier réservoir (négatif). Les sondes primaires migrent de façon équimolaire dans chacun des capillaires et se concentrent au niveau de l'électrode courbe,
2) le potentiel entre l'électrode de réservoir et l'électrode courbe est coupé et un potentiel électrique est appliqué entre la première électrode fonctionnalisée (+) et l'électrode courbe (-). Les sondes de chaque capillaire migrent alors au niveau de la première électrode fonctionnalisée où les sondes secondaires complémentaires des spots de la première électrode (un spot par capillaire) peuvent s'hybrider (l'hybridation est accélérée par le champ électrique). La concentration au niveau de chaque spot est maximale (elle ne dépend que du nombre de capillaires et non plus du volume total des canalisations). Afin de confiner les sondes primaires au niveau du spot, la deuxième électrode ligne fonctionnalisée peut être portée à un potentiel négatif, ce qui conduit à obtenir une distribution de charges (- + -) où la charge + est centrée sur le premier spot de chaque capillaire.
3) pour améliorer l'hybridation, les potentiels électriques peuvent être discontinus, le laps de temps sans potentiel correspondant à un temps de relaxation, pendant lequel les sondes primaires peuvent s'hybrider sans contrainte. Pour augmenter le mélange des sondes primaires et ainsi favoriser l'hybridation des sondes primaires présentes en faible nombre, durant le temps de relaxation un potentiel discontinu et alternatif peut-être établi entre la première électrode ligne fonctionnalisée et les électrodes non fonctionnalisées au-dessus et en dessous du réseau de capillaires (ceci améliore encore la spécificité d'hybridation).

Une fois les sondes primaires hybridées au niveau de la première ligne de spots, les potentiels électriques sont décalés d'une ligne. En notant 0 la position de l'électrode courbe et respectivement 1, 2 et 3 les positions de la première, seconde et troisième électrodes fonctionnalisées greffées de sondes, la cinématique de potentiel électrique appliquée peut être décrite de la façon suivante :
4) la deuxième électrode fonctionnalisée est mise à un potentiel positif la distribution de charges est alors (0 -, 1 +, 2 +) (éventuellement la troisième électrode est mise à un potentiel négatif) avec une distribution de charge (0 -, 1 +, 2 +, 3 -);
5) la première électrode fonctionnalisée est mise à un potentiel négatif. La distribution de charges est alors (0- 1-, 2+) éventuellement (0-, 1-, 2+, 3-).
6) L'électrode courbe est mise à 0 pour une distribution de charges (1-, 2 +) éventuellement (1-, 2+, 3-).
7) un potentiel électrique discontinu et alternatif est appliqué entre le jeu d'électrodes non fonctionnalisées et l'électrode 2 fonctionnalisée.

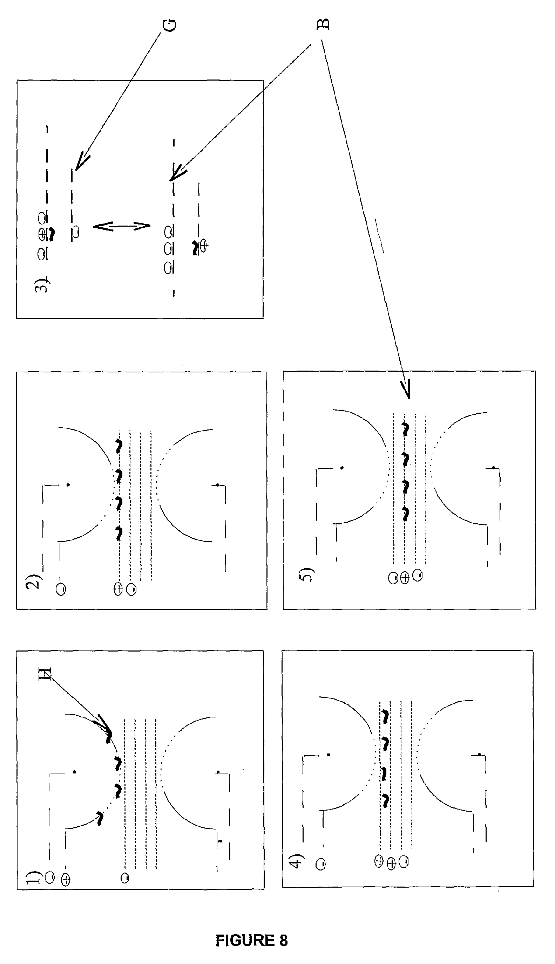

L'ensemble des sondes non hybridées au niveau de la première ligne de spots, va migrer au niveau de la seconde ligne de spots (figure 8). L'ensemble de la cinématique de migration, relaxation, mélange (correspondant aux étapes 4-7 ci-dessus) est appliqué de proche en proche pour hybrider tous les spots de toutes les lignes qui ont des sondes primaires complémentaires dans l'échantillon analysé. Une fois arrivées dans le deuxième réservoir, les sondes primaires qui avaient migré dans des capillaires différents se mélangent de nouveau. Il est alors possible d'effectuer les séquences de potentiel électrique dans l'autre sens pour parcourir le réseau de capillaires en sens inverse. Les allers et retours des sondes primaires entre les deux réservoirs à travers les capillaires permettent d'augmenter la sensibilité de détection du dispositif.

En règle générale, il est nécessaire d'appliquer des champs électriques de l'ordre de 160 mV /mm pour déplacer efficacement les molécules. La distance entre l'électrode courbe et l'électrode de réservoir étant importante, les potentiels appliqués à ces électrodes pour respecter le champ sont de l'ordre du volt et non plus de celui du mm volt. Pour éviter que les électrodes courbes et de réservoir ne brûlent, il est préférable de les réaliser en or.

Dans un exemple spécifique, l'un des réseaux d'électrodes est formé par une superposition de deux jeux d'électrodes perpendiculaires dans deux plans différents, l'autre réseau d'électrodes étant alors relié à la masse. Les spots sont greffés sur les électrodes du jeu inférieur à l'intersection de la projection des électrodes du jeu supérieur dans le plan des électrodes inférieures.

Dans un exemple spécifique, le réseau d'électrodes fonctionnalisées est formé par un quadrillage de type (lignes/colonnes) d'électrodes dans lequel, à chaque intersection entre une électrode ligne et une électrode colonne, une électrode spot sera reliée à une ligne et une colonne par l'intermédiaire d'un transistor à effet de champ.

La disposition perpendiculaire entre les deux jeux superposés d'électrodes, permet théoriquement de réaliser la mesure. Toutefois, l'utilisation d'un courant alternatif ou discontinu et le fait qu'une électrode connecte plusieurs spots entraîne des problèmes de capacité électrique parasite qui perturbe la mesure. En effet, il devient difficile, voire impossible, de mesurer correctement le courant et les tensions électriques pour définir l'impédance de chaque spot. Pour pallier ce problème il est nécessaire d'introduire des interrupteurs qui permettront d'établir une tension et un courant spot à spot.

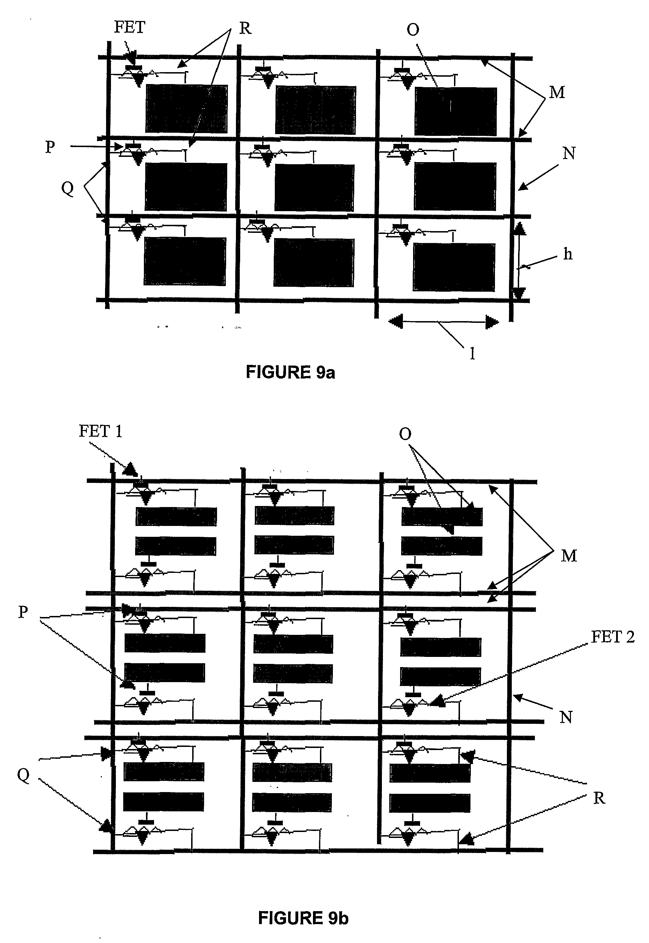

Pour réaliser les interrupteurs compatibles avec la dimension des biopuces, le jeu d'électrodes fonctionnalisées est remplacé par un quadrillage d'électrodes dans un même plan, constitué d'un premier ensemble d'électrodes (horizontales ou lignes) isolé et perpendiculaire à un deuxième ensemble d'électrodes (verticales ou colonnes). La maille du quadrillage délimite un espace où est disposée une petite électrode (électrode spot) de 10 à 500 µm de côté selon la taille du quadrillage. Typiquement, chaque maille du quadrillage a une hauteur h de 150µm environ et une largeur I de 500µm environ. Au niveau de chaque maille du quadrillage sont disposés un ou deux transistors à effet de champ, tels que la grille du transistor soit reliée à l'électrode horizontale d'un côté de la maille, la borne entrante (source) du transistor à l'électrode verticale d'un côté de la maille et la borne sortante (drain) du transistor à l'électrode spot (figure 9a).

Dans la variante représentée en figure 9b, chaque maille du quadrillage comporte deux électrodes spots O dont chacune est associée à un transistor FET 1 et FET 2, le deuxième transistor FET 2 jouant le rôle de détecteur. La grille P du premier transistor est reliée à une première électrode ligne M et la grille P du deuxième transistor est reliée à la seconde électrode spot. Les sources Q des deux transistors sont reliées à une même électrode colonne N d'un côté de la maille. Le drain R du premier transistor FET1 est relié à la première électrode spot et le drain R du deuxième transistor est relié à une seconde électrode ligne M indépendante de tout autre transistor.

Dans cette configuration, les électrodes lignes du quadrillage sont doublées. La première des électrodes lignes M connecte les grilles P des premiers transistors FET 1 d'une ligne du quadrillage alors que la deuxième électrode ligne M connecte les drains R des transistors FET 2 de cette ligne du quadrillage.

Après hybridation chaque transistor FET 1 est utilisé pour mettre sous tension l'électrode spot à laquelle il est relié. Un courant électrique et/ou un potentiel électrique alternatif ou continu est imposé à la source Q du transistor FET 1. Le potentiel électrique ou la variation de potentiel électrique imposé va induire des courants et des potentiels localement au niveau des molécules d'acides nucléiques greffées sur les électrodes spots. En effet, les molécules greffées sur les deux électrodes spots d'une maille du quadrillage se comportent comme de petites capacités électriques. Les variations de courant et de potentiel au niveau de la grille P du transistor FET 2 vont modifier l'état de fermeture de ce transistor entraînant des variations de courant et de potentiel proportionnelles entre la source Q et le drain R du transistor FET 2. Ces variations dépendent de la séquence, de la taille et de l'état d'hybridation des molécules greffées sur les électrodes spots. Notamment, ces variations dépendent de l'état simple ou double brin des acides nucléiques et permettent donc de quantifier le taux d'hybridation au niveau de chaque électrode spot.

L'utilisation de deux électrodes spots dans une maille du quadrillage rend le signal mesuré et la sensibilité de cette mesure fortement dépendants de la forme de ces électrodes spots. La mesure peut être améliorée en utilisant des électrodes spots en spirales emboîtées ou en introduisant sur la plaque supérieure une contre-électrode formée d'électrodes spots isolées, images des électrodes spots de la première matrice.

Une autre solution consiste à séparer ce quadrillage à deux transistors par maille en deux quadrillages à un seul transistor par maille, ces quadrillages étant situés de part et d'autre du réseau de capillaires.

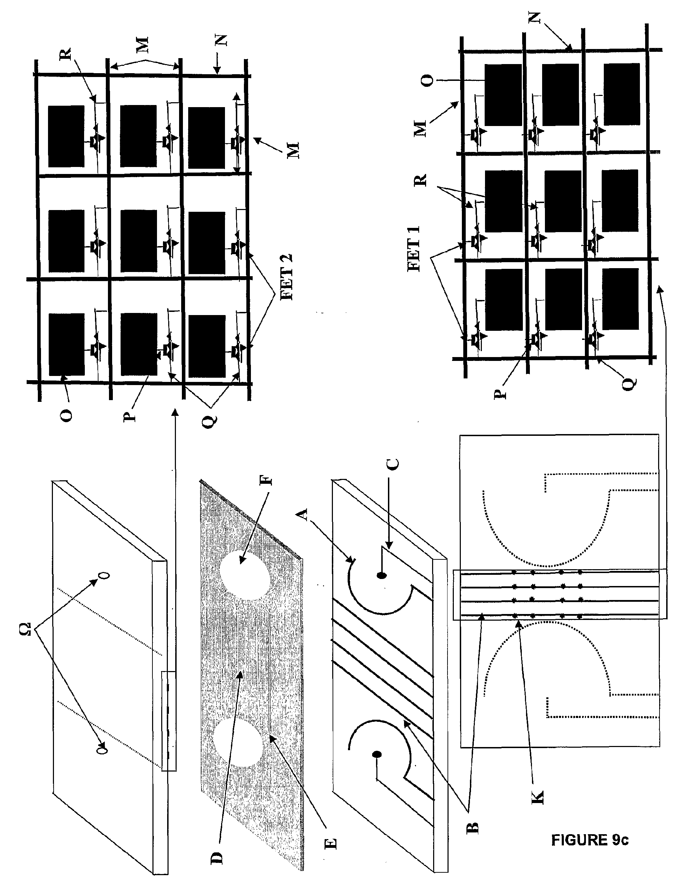

Le premier quadrillage dit de contrôle représenté dans le coin inférieur droit de la figure 9c est identique à celui de la figure 9a. Il s'étend au-dessous du réseau de capillaires et permet d'alimenter sélectivement chaque électrode spot. Les électrodes colonnes N et horizontales M sont isolées électriquement et les électrodes spots O sont recouvertes d'un film de poly-imide de 10 à 40nm d'épaisseur pour le greffage de molécules.

Les mesures électriques sont effectuées par un second quadrillage représenté dans le coin supérieur droit de la figure 9c qui s'étend au dessus du réseau de capillaires en vis-à-vis du premier quadrillage. Ce quadrillage permet de contrôler les champs électriques de migration et de confinement des sondes introduites dans le réseau de capillaire pour l'hybridation. Pour cela, les électrodes colonnes N (ou inversement horizontales M) ainsi que les électrodes spots de ce quadrillage peuvent ne pas être isolées électriquement. Pour chaque transistor FET 2, la source Q est reliée à une électrode colonne, le drain R est reliée à une électrode ligne, et la grille est reliée à une électrode spot. Les électrodes lignes M sont recouvertes par les parois des capillaires (ou inversement les électrodes colonnes N).

Une fois la puce hybridée, chaque électrode spot O du quadrillage de contrôle (figure 9c) est mise sous tension séquentiellement (champ et courant alternatifs). Le courant entre la source Q et le drain R du transistor FET 1 d'une électrode spot est fonction en intensité, tension, fréquence et déphasage du niveau de l'état d'hybridation des sondes greffées sur l'électrode spot. Le courant et la tension produit au niveau de l'électrode spot du quadrillage de contrôle ouvrent de manière proportionnelle la grille P du transistor FET 2 de l'électrode spot située en vis-à-vis dans le quadrillage de détection, ce qui induit un courant et une tension mesurables entre la source Q et le drain R du transistor FET 2 de cette électrode spot.

En résumé, un quadrillage est obtenu tel que les mailles, définies par les électrodes horizontales et verticales, sont occupées par de petites électrodes spot. Les électrodes spot sont reliées à deux des quatre côtés de la maille par un ou deux transistors à effet de champ. Les sondes moléculaires sont greffées au niveau de chaque électrode spot. Les électrodes du jeu supérieur sont disposées en vis-à-vis de chaque colonne d' « électrodes spot » et parallèlement aux électrodes verticales du quadrillage inférieur. Chaque électrode du jeu inférieur est reliée à la masse. Le jeu d'électrodes non fonctionnalisées peut être remplacé par une plaque unique reliée à la masse, ou une matrice de détection telle que décrite ci-dessus ou une matrice d'électrodes spots isolées.

En plaçant les électrodes horizontales sous tension, les grilles de tous les transistors sont alimentées, ce qui coupe le courant entre l'entrée et la sortie de tous les transistors ; les électrodes spot sont donc isolées. En coupant le courant au niveau d'une seule électrode horizontale et en appliquant le courant et un champ électrique discontinu sur une électrode verticale unique, seul le transistor à l'intersection des deux électrodes laisse passer le courant entre leur entrée et leur sortie. Un spot unique est mis sous tension, la variation d'impédance au niveau de l'électrode spot peut donc être déterminée sans être parasitée par les autres spots.

Dans un mode de réalisation particulier, le fonctionnement de la grille est inversé selon le transistor, c'est-à-dire le courant ne passe entre la source et le drain que si la grille est sous tension. Dans ces conditions, les lignes sont contrôlées en mettant les électrodes de grilles sous tension.

Dans un exemple spécifique, l'étape c) du procédé selon l'invention est réalisée au sein du réservoir du réseau de capillaires du dispositif selon l'invention.

L'ensemble « billes magnétiques - sondes primaires - cibles moléculaires » est dénaturé de manière contrôlée, dans le volume souhaité, afin de récupérer l'élua contenant les sondes primaires qui se sont auparavant liées spécifiquement, en particulier qui se sont hybridées.

Il est alors possible d'introduire un champ magnétique dans un réservoir qui va recevoir les complexes cibles/sondes primaires fixées sur les billes. Il peut s'agir soit d'un micro aimant statique fixé sur la paroi du réservoir opposé à l'électrode de réservoir, soit d'un solénoïde obtenu par dépôt de couche mince sur la même paroi (le champ magnétique étant produit par un courant alternatif dans le solénoïde).

Le champ magnétique permet de maintenir l'ensemble particule-cible moléculaire dans le réservoir. La température du système est augmentée afin de séparer, par exemple déshybrider, les sondes primaires et les cibles. La température du système est régulée globalement par un bain-marie ou dans une chambre calorimétrique et éventuellement, une régulation locale est obtenue par des résistances électriques introduites dans les réservoirs.

Le solénoïde a un double emploi de générateur de champ magnétique et de résistance chauffante.

Une fois les sondes primaires dénaturées et en solution dans le réservoir, elles se déplacent dans les capillaires et se lient, notamment s'hybrident sur les spots.

Il convient d'adapter les températures du système de thermostat pour permettre l'hybridation des sondes primaires sur les spots. Une dénaturation chimique des sondes primaires sur les billes peut être réalisée. Dans ces conditions, il est nécessaire d'enlever les billes grâce à l'aimant après dénaturation des hybrides et migration des sondes primaires au niveau de l'électrode courbe de réservoir. Pour permettre l'hybridation, le milieu doit être neutralisé.

La présente invention porte également sur un jeu de sondes primaires représentant une réplique des cibles moléculaires à analyser comprenant :
- un jeu de sondes primaires de types différents,tel que décrit plus haut,
- des moyens pour séparer et des moyens pour récupérer les sondes primaires et les cibles moléculaires liées par liaison spécifique, de façon à obtenir un jeu de sondes primaires représentant une empreinte des cibles moléculaires à analyser.

Sous réserve de leur compatibilité technique, les caractéristiques décrites dans la présente demande ci-dessus peuvent être combinées également avec différentes caractéristiques décrites dans les exemples, de façon à définir d'autres modes de réalisation particuliers de l'invention.

### LEGENDE DES FIGURES

La figure 1 illustre un exemple de variation de température appliquée au cours de la complexation (hybridation) sonde cible pour rendre la reconnaissance plus spécifique entre les sondes et les cibles.
La figure 2 illustre un détecteur à lumière polarisée.
   Coupe au niveau d'une électrode spot d'une Electro-Chip_2D.
   Pour le greffage des sondes on utilise un guide de greffage pour guider et orienter le greffage sur l'électrode.
   Le guide de greffage est obtenu soit par frottement du matériau constituant le guide avec un velours, soit par un moulage du matériau qui constituera le guide avec de courts fragments ADN simple brin déposés et peignés sur l'électrode spot. Les guides sont alors obtenus après abrasion du matériau et/ou élimination des fragments d'ADN.
La figure 3 illustre un exemple d'un jeu d'électrodes fonctionnalisées et greffées de sondes. Les électrodes en or ou en ITO sont gravées sur une lame de verre ; les dépôts de sondes (spot ou unité d'hybridation) sont effectués sur les électrodes.
La figure 4 illustre un assemblage des couples d'électrodes lignes. Les couples d'électrodes lignes sont obtenus en disposant deux lames gravées d'électrodes en vis-à-vis. Une des lames est fonctionnalisée, l'autre ne l'est pas.
La figure 5 illustre un réseau de capillaires à intercaler entre les deux jeux d'électrodes fonctionnalisées et non fonctionnalisées.

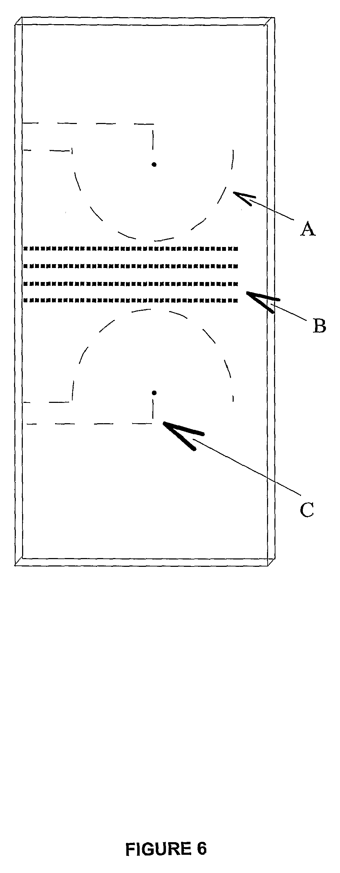

La figure 6 illustre un jeu d'électrodes fonctionnalisées, complété par les électrodes courbes de liaison et les électrodes de réservoir.
La figure 7 illustre un assemblage des deux jeux d'électrodes fonctionnalisées et non fonctionnalisés autour du réseau de capillaires.
La figure 8 illustre une séquence de charges appliquées aux électrodes pour faire migrer séquentiellement les cibles d'une électrode à l'autre, donc d'un spot à l'autre.
La figure 9a illustre un quadrillage remplaçant le jeu d'électrodes fonctionnalisées pour les mesures d'impédance en courant discontinu.
La figure 9b illustre un autre quadrillage remplaçant le jeu d'électrodes fonctionnalisées pour les mesures d'impédance en courant discontinu.
La figure 9c illustre des quadrillages destinés à être situés respectivement au dessus et au dessous d'un réseau de capillaires

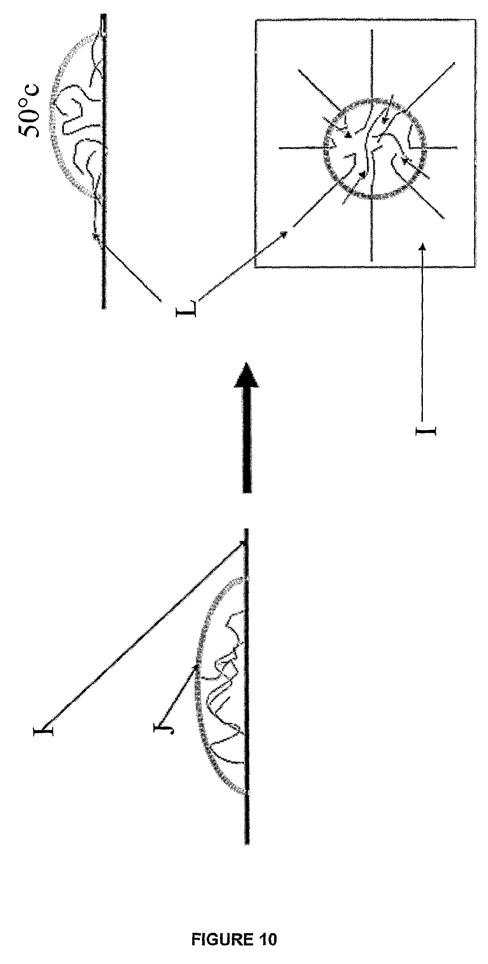

La figure 10 illustre l'accrochage des brins d'ADN à la paroi par évaporation. En s'évaporant, la goutte déposée va étirer vers le centre du spot.

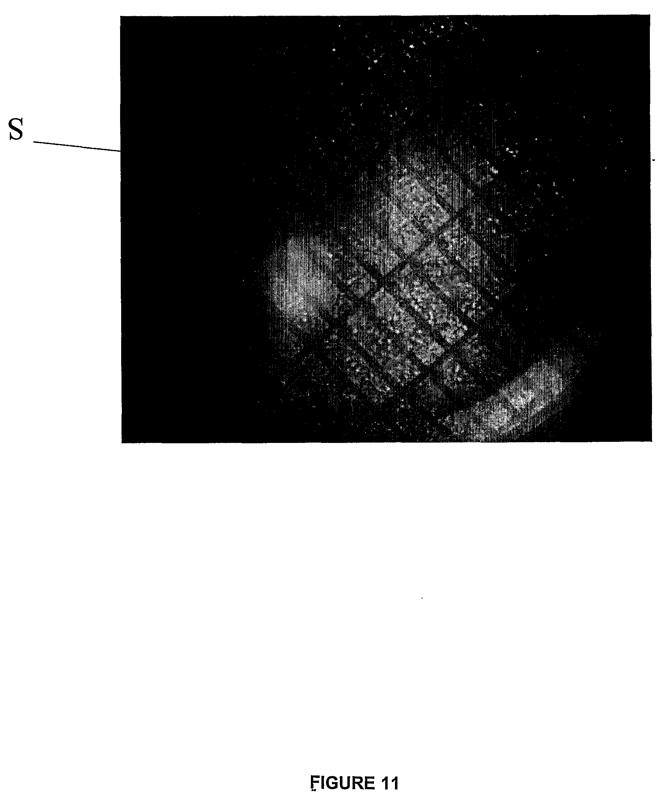

La figure 11 illustre une électrode en ITO recouverte de sondes ADN simple brin étiré.

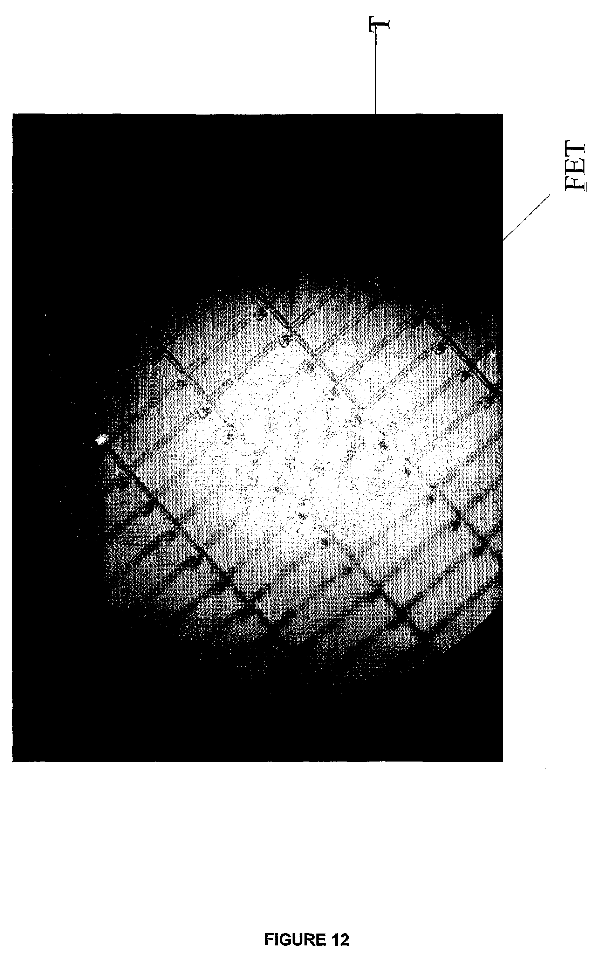

La figure 12 illustre une électrode en ITO hybridée avec un brin d'ADN complémentaire.

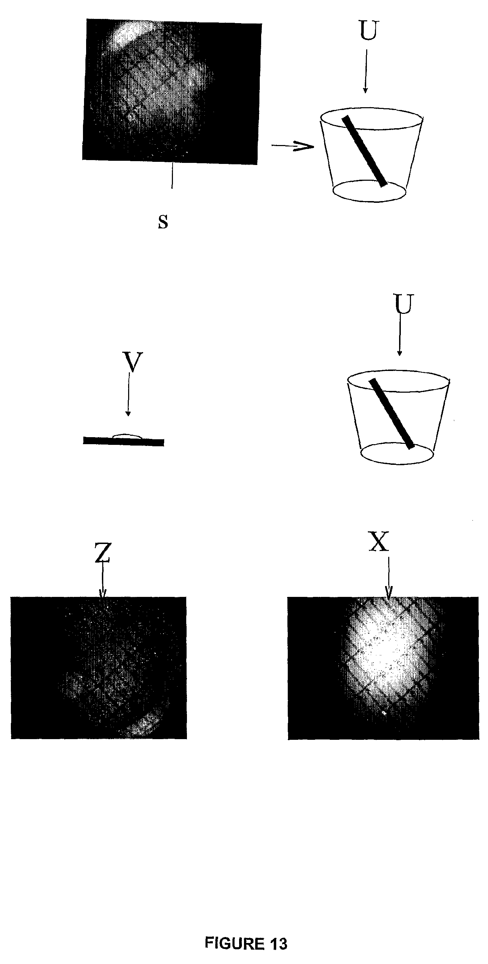

La figure 13 illustre différents traitements dénaturants. La fixation des nucléotides sur les électrodes d'ITO résiste aux différents traitements dénaturants les plus utilisés (NA OH 0.1M, SDS 10% , 95°c ...), ce qui permet de régénérer la puce après usage.

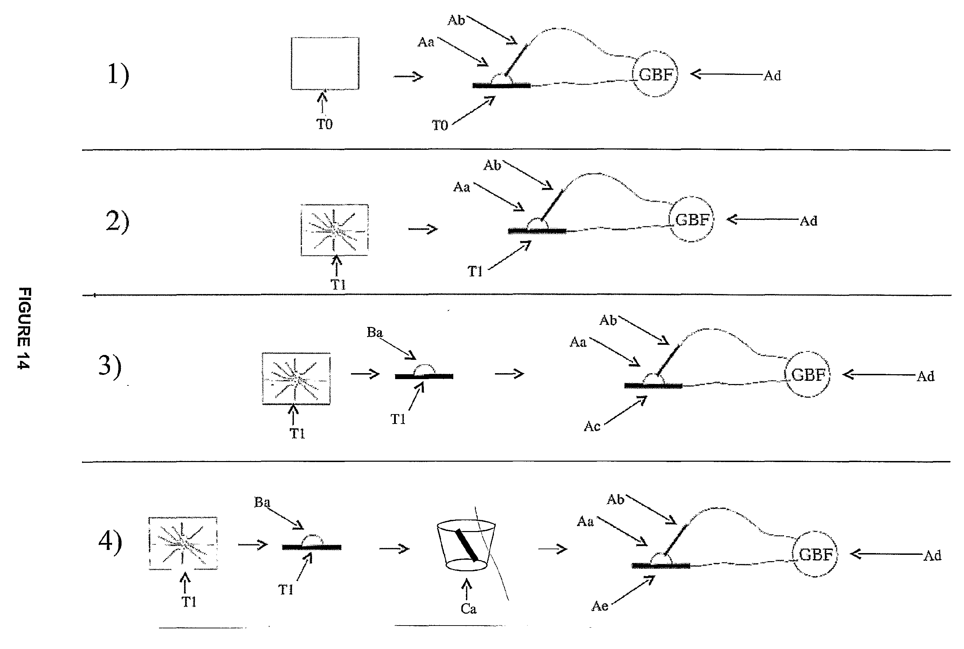

La figure 14 illustre la réalisation de mesures. Une mesure d'impédance est réalisée, d'une part sur une électrode spot nue (figure 14.1) et d'autre part sur une électrode fonctionnalisée avec un simple brin ADN de 118 bases et obtenue par peignage d'un dépôt de 0.1 µl d'une solution d'ADN 1 M (figure 14.2). Il apparaît que l'impédance est plus élevée pour l'électrode recouverte d'un ADN simple brin que pour une électrode recouverte d'un duplex d'ADN double brin (figure 14.3 et 14.4).

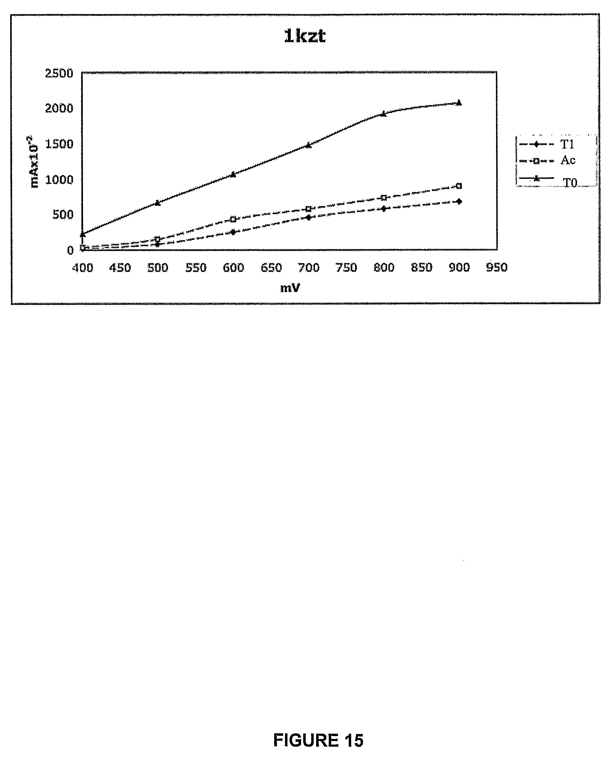

La figure 15 illustre la mesure des courants en fonction des tensions appliquées aux électrodes spots.

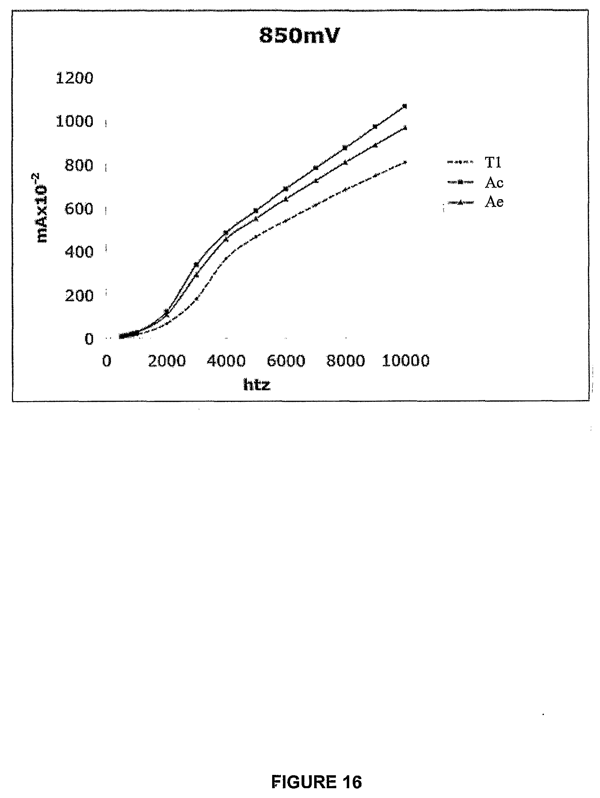

La figure 16 illustre la mesure des courants à une tension de 850 mV en fonction de la fréquence des tensions.

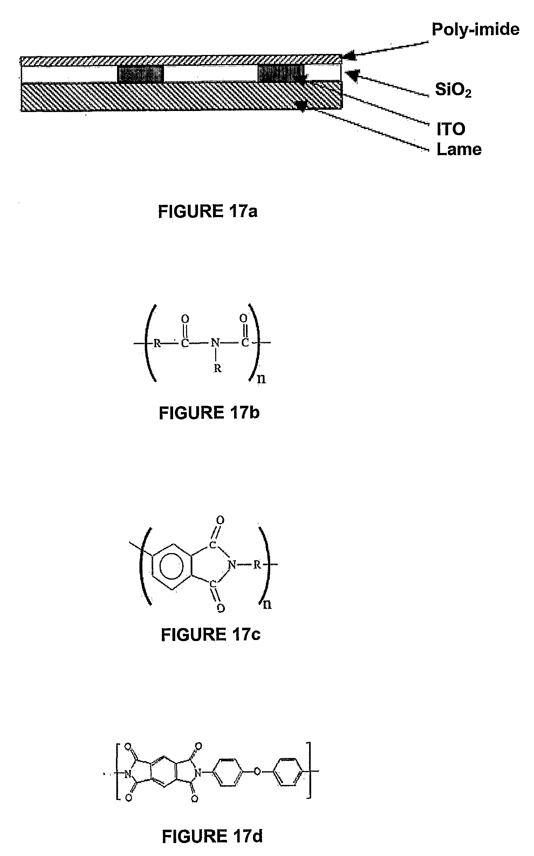

La figure 17a est une vue schématique en coupe d'un support de fixation de sondes moléculaires.
Les figures 17b à 17d sont des formules de polyimides
Les figures 18a, 18b et 18c représentent des composés mixtes Acide nucléique / Peptide Nucleic Acid utilisés comme sondes primaires.
Les figures 18d, 18e et 18f représentent des amorces du type (PNA)polyT-Acide nucléique.
La figure 19 illustre un exemple de réalisation du procédé selon l'invention.
La figure 20 illustre un autre exemple de réalisation du procédé selon l'invention.
La figure 21 représente les étapes d'un algorithme pour la préparation d'un jeu de sondes primaires.

### EXEMPLES :

L'électro-puce 2D (Electro-Chip 2D) permet d'identifier et de quantifier, sans marquage préalable, les séquences des bio-polymères constituant les sondes primaires éluées. Le dispositif se compose d'une matrice bi-dimensionnelle de sondes biologiques (jeu de sondes secondaires) organisées en spot, associée à deux jeux d'électrodes particuliers. La matrice est contrainte dans un réseau de capillaires parallèles connectés entre eux par deux réservoirs (un à chacune de leurs extrémités).

Le réseau de capillaires et le dispositif d'électrodes permet d'acheminer les cibles (sondes primaires éluées selon le procédé de l'invention) au niveau de chaque ligne de spot et de les concentrer à ce niveau grâce à une succession de champs électriques de confinements (contrôlés par le réseau d'électrodes).

Ce dispositif permet la réduction du volume total de réaction en empêchant la diffusion passive des sondes.

Ceci permet donc de concentrer successivement la totalité des cibles au niveau de chaque ligne de spot de la matrice. La quantité limite de matériel nécessaire pour réaliser une expérience ne dépend alors que de la concentration locale des cibles au niveau d'une ligne de spots et non plus du volume total du réseau de capillaire. Ceci permet de diminution de matériel à utiliser, proportionnelle au rapport entre le volume de réaction d'une puce traditionnelle et le volume occupé par les sondes confinées au niveau d'une ligne de la matrice (jusqu'à 10⁸ en théorie).

La quantification des sondes hybridées est faite par la mesure d'impédance du spot. De ce fait, il n'est plus nécessaire de marquer les cibles. La technologie de fabrication couplée à la géométrie particulière choisie pour les électrodes, permet d'augmenter la densité des spots pour passer d'un maximum de 800 à 20 000 - 30 000 spot sur une surface de moins de 18 cm². De plus, les artéfacts de mesure dus aux changements de conformation des sondes sont minimisés par les contraintes stériques imposées à celles-ci lors de leur greffage sur la surface des électrodes. Pour réaliser le greffage contraint, nous avons mis au point un protocole de greffage sur des alliages métallique transparent qui ne nécessitent pas de fonctionnalisation des sondes.

### Exemple 1 : Fixation des acides nucléiques sur des électrodes en ITO.

L'immobilisation des sondes moléculaires sur leur support doit être suffisamment forte pour résister aux différents traitements de dénaturation des acides nucléiques et aux éventuels champs électriques utilisés pour manipuler les sondes primaires. Dans le cadre de la mesure du taux d'hybridation des acides nucléiques par l'impédance, deux principaux problèmes ont été rencontrés, en plus de la géométrie des électrodes :
1) Des défauts locaux de structure dans les matériaux peuvent entraîner des résistances sporadiques d'une électrode à l'autre, causant des artéfacts sur la lecture de l'impédance,
2) La fonctionnalisation des électrodes pour greffer les molécules d'ADN peut perturber la mesure.

À ces problèmes, viennent s'ajouter les changements d'impédance en fonction de la conformation et de la taille des molécules d'acides nucléiques.

L'utilisation des alliages d'oxyde d'étain ou de zinc tel que : ITO, ATO, FTO, ZNO (éventuellement recouverts de poly-imide par exemple de Kapton®), permet de minimiser ces problèmes.

En effet, les méthodes de dépôt de ces alliages sont très standardisées et reproductibles, ce qui permet d'obtenir des électrodes avec très peu de défauts. Ces matériaux sont transparents et permettent des mesures optiques.

Enfin, la fonction phosphate PO₃²⁻ est adsorbée à la surface des électrodes telles que l'ITO (éventuellement recouvertes de poly-imide par exemple de Kapton®), de manière quasi irréversible. Les acides nucléiques étant naturellement riches en phosphate, ils sont adsorbés sur un dépôt d'ITO (éventuellement recouvert de poly-imide par exemple de Kapton®). De même, les protéines couplées à des fonctions phosphate peuvent être fixées sur de tels supports ou directement si elles possèdent des groupements électrophiles. Pour ce faire, les protéines telles que les anticorps, peuvent être couplées à des molécules comme l'éthanolamine phosphorique, qui établit une fonction peptidique avec les protéines (éventuellement en présence de glutaraldéhyde) et qui est retenue sur les alliages d'ITO grâce à la fonction phosphate.

Un des protocoles possibles pour la fixation des sondes secondaires sur des électrodes d'ITO est le suivant.

Les électrodes d'ITO sont :
1) Plongées 20 minutes dans une solution d'ammoniaque et de peroxyde d'hydrogène.
2) Rincées à l'eau distillée.
3) Rincées avec du propan-2-ol.
4) Les sondes sont déposées en spot sur les électrodes d'ITO, en atmosphère humide.
5) La lame est ensuite mise dans une étuve à 50°C pour réaliser un peignage moléculaire de l'ADN.

En s'évaporant, la goutte déposée va étirer vers le centre du spot les brins d'ADN accrochés à la paroi tel que décrit à la figure 10.

L'ITO étant très électrophile, il est nécessaire d'isoler par un procédé d'encapsulation les parties des électrodes qui n'ont pas reçu de greffage de sondes, afin d'éviter tout accrochage non spécifique de cibles ou de sondes primaires. Par exemple, un film de poly-pyrrole peut être utilisé. Le film est réalisé en mettant les électrodes greffées sous tension en présence d'une solution de pyrrole. Le pyrrole polymérise spontanément sous l'action du courant et isole les parties libres des électrodes. Les électrodes peuvent également êtres saturées par un petit oligonucléotide qui ne peut pas s'hybrider de manière stable avec les cibles, par exemple un trimer ATA ou TAT... Un réseau d'électrodes fonctionnalisées par de l'ADN simple brin est obtenu comme le montre la figure 11.

Ce réseau est capable d'hybrider de façon spécifique les sondes primaires des ARNm ou des ADNc tel que montré figure 12.

La fixation des nucléotides sur les électrodes d'ITO résiste aux différents traitements dénaturants les plus utilisés (NA OH 0.1M, SDS 10%, 95°c ...), ce qui permet de régénérer la puce après usage (figure 13).

Une alternative est l'utilisation de sondes secondaires fonctionnalisées au pyrrole pour réaliser le greffage des sondes.

Il est possible d'utiliser directement des plaques ou des lames de microscope entièrement recouvertes d'ITO et / ou de poly-imide (Kapton®) pour réaliser des matrices de sondes (nucléotide ou protéique) par dépôt direct selon le même procédé décrit pour les dépôts sur électrodes. Les puces obtenues sont des réseaux conventionnels utilisés de façon classique.

Dans certaines conditions, il est avantageux de séparer les électrodes métalliques en ITO déposées sur une lame par du SiO₂ (une couche moléculaire au minimum) pour isoler électriquement les électrodes, puis de recouvrir les électrodes d'une couche de poly-imide (une couche moléculaire au minimum de 10 à 40nm d'épaisseur environ) pour le greffage des sondes moléculaires. Le support ainsi obtenu représenté en figure 17a est du type verre / SiO₂ / poly-imide et verre / ITO / poly-imide et permet à la fois le greffage de sondes moléculaires sur le support et la réalisation de mesures de courant et de résistance du type capacitif pour la détection des complexes formés sur le support.

Des exemples de formules de polyimide sont représentés aux figures 17b, 17c et 17d. La figure 17b représente un polyimide linéaire et les figures 17 c et 17d représentent des polymides aromatiques héterocycliques, la formule de la figure 17d représentant le Kapton®.

### Exemple 2 : Mesure d'impédance

Une mesure d'impédance est réalisée, d'une part sur une électrode spot nue (figure 14.1) et d'autre part sur une électrode fonctionnalisée avec un simple brin ADN de 118 bases et obtenues par peignage d'un dépôt de 0.1 µl d'une solution d'ADN 1 M (figure 14.2).

Il apparaît que l'impédance est plus élevée pour l'électrode recouverte d'un ADN simple brin que pour une électrode recouverte d'un duplex d'ADN double brin (figure 14.3, 14.4 ; figure 15). La présence des acides nucléiques qui ne se sont pas hybridés ne modifie que très peu l'impédance mesurée sur l'électrode spot. L'impédance est essentiellement due aux molécules fixées à la surface d'une électrode d'ITO : l'impédance est plus forte pour les simples brins que pour les doubles brins (figure 16). Il est donc possible de réaliser des mesures dynamiques pour voir la cinétique d'hybridation.

Les différences d'impédances mesurées entre l'électrode spot nue, l'électrode spot recouverte d'ADN simple brin et l'électrode spot recouverte de duplex ADN/ADN dépend de la fréquence des champs (tension) et des courant électriques appliqués, ces différences permettent de quantifier le taux d'hybridation (figure 16).

### Exemple 3 : Procédé de séparation et d'analyse appliqué aux protéines

Le procédé peut être décliné pour être utilisé pour le dosage de différentes protéines et de leurs modifications dans un mélange provenant par exemple d'un extrait cellulaire.

### E.1 Le procédé nécessite :

4) Un ensemble de particules magnétiques (ou une surface) pouvant fixer de manière forte les protéines. Typiquement nous pouvons citer les billes ou les membranes de polystyrène, nylon, nitro-cellulose...
5) Un mélange stoechiométrique d'anticorps, où chaque type d'anticorps est lié de façon forte (covalente) avec une séquence spécifique d'acide nucléique: la séquence tag. La séquence tag se décompose en une ou deux séquences génériques susceptibles d'être coupées spécifiquement et en une séquence unique et spécifique pour chaque type d'anticorps. La séquence nucléotidique spécifique de l'anticorps signe ce dernier de manière non-équivoque comme un code barres.
6) Une puce à oligonucléotides, où les sondes de chaque spot sont constituées d'une séquence complémentaire à la partie spécifique de la séquence tag d'un des types d'anticorps du mélange stoechiométrique d'anticorps. Typiquement, il pourra s'agir des puces à impédance décrites ci-dessus ou tout autre puce telle que l'ensemble des spots de la puce correspondent à l'ensemble des séquences tag utilisées (un spot par séquence tag).

### E.2 Principe de fonctionnement.

À partir d'un extrait cellulaire, les protéines sont fixées sur des particules magnétiques de polystyrène. Le polystyrène a la faculté d'adsorber de façon durable les protéines. Un excès de billes est utilisé par rapport à la concentration des protéines pour éviter toute saturation des billes, limitant ainsi les encombrements stériques. Selon l'étude souhaitée, les protéines sont ou non dénaturées. Les billes et les protéines fixées sont alors :
4) précipitées par un champ magnétique,
5) isolées du surnageant,
6) rincées et reprises dans un tampon salin.

Les billes sont ensuite saturées par des protéines inertes pour le système étudié. Par exemple, l'albumine sérique de bovin (pour les études non bovines), des protéines de petites tailles exogènes à l'espèce étudiée comme l'inhibiteur de Kunixt ou encore des acides aminés à chaîne aliphatique comme la leucine sont utilisés. Les étapes 1 à 3 sont de nouveau réalisées. Puis l'ensemble, billes saturées - protéines fixées est mis à complexer avec le mélange équimolaire d'anticorps tagués. Les anticorps (monovalents) se fixent spécifiquement sur leurs protéines cibles immobilisées sur les billes. La quantité de chaque type d'anticorps fixé est proportionnelle à la quantité de chaque type de protéines adsorbées sur les billes. Pour diminuer l'encombrement stérique, les anticorps peuvent êtres substitués par des fragments d'anticorps (ou hémi-anticorps) obtenus par une digestion par la papaïne. Chaque hémi-anticorps est « tagué » par une séquence d'acide nucléique. Les étapes 1 à 3 sont de nouveau réalisées. Les anticorps (ou hémi anticorps) qui ont réagi sont donc isolés et séparés de ceux qui n'ont pas réagi. Le tag d'acides nucléiques est coupé grâce à la séquence de coupure introduite. Il peut s'agir par exemple d'une séquence palindromique ou de la séquence cible d'un abzyme... Dans le cas d'une séquence palindromique, deux solutions particulières peuvent être citées :
- La séquence palindromique est introduite entre l'anticorps et la séquence tag spécifique. Pour séparer la séquence tag de l'anticorps, il suffit d'introduire dans le milieu une séquence complémentaire de la séquence de coupure avec l'enzyme de restriction correspondante, ce qui permet de séparer l'anticorps de la séquence tag spécifique.
- Deux séquences de coupure sont introduites respectivement entre l'anticorps et la séquence tag spécifique, et à la fin de la séquence tag, de telle sorte que ces deux séquences soient complémentaires l'une de l'autre. En s'hybridant, elles forment le site de coupure spécifique pour une enzyme qui est ultérieurement introduite dans le milieu. Les séquences tags spécifiques sont alors libérées en solution.

Une fois séparées des anticorps, les séquences tags fournissent un mélange d'oligonucléotides en solution dont les quantités sont proportionnelles à celles des types des anticorps retenus sur les billes, et donc proportionnelles aux différentes protéines et à leurs modifications présentes dans l'extrait cellulaire analysé. Le mélange de nucléotides obtenu peut être analysé sur une puce à ADN classique ou une puce telle que décrite précédemment.

Il est possible avec ce procédé de réaliser une étude différentielle entre les protéines de deux extraits cellulaires différents (sans avoir à marquer directement les protéines). Les tags des anticorps du premier extrait sont marqués avec des marqueurs fluorescents d'une couleur (par exemple cy3) et les tag des anticorps du deuxième extrait sont marqués avec des marqueurs fluorescents d'une autre couleur (par exemple cy5). Chacun des extraits est traité comme décrit ci-dessus, puis les mélanges de tags représentatifs recueillis pour chaque extrait sont réunis volume à volume et hybridés sur une puce. La lecture de la puce pour chaque couleur fournit le différentiel entre les protéines des deux extraits.

### E.3 Marquage des anticorps

Les anticorps sont marqués par des tags d'acide nucléique.

Par exemple :
- α=5'NH2(x)n(y)m3' (éventuellement 5'NH2(zi)p(x)n(y)m3')
- β=5'NH2(x)n(y)m(x)n3'.(éventuellement5'NH2(zi)p(x)n(y)m(x)n(zj)p3)

La fonction amine NH2 permet de fixer la séquence nucléotidique sur l'anticorps avec un agent pontant tel que le glutaraldéhyde et/ou l'éthanolamine... Le polymère d'acide nucléique peut éventuellement être directement être fixé sur l'anticorps grâce à sa fonction amine.

(x)n est une séquence de n nucléotides capable de former un palindrome tel que :
(x)n =5'CCCGGG3' coupé par l'enzyme de restriction Srf1
(x)n =5'TACGTA3' coupé par l'enzyme de restriction SmaB I
(x)n=5'AATAAT3' coupé par l'enzyme de restriction Sspl
(x)n=5'TTTAAA3' coupé par l'enzyme de restriction Dra I...

De manière générale, toutes les enzymes de restriction à coupure franche conviennent. Les sites de restriction à plus faible Tm sont toutefois préférés.

Deux solutions sont possibles :
- α) La formation du palindrome de coupure est réalisée entre une séquence intra tag et une séquence libre (x)n (éventuellement (x)n(zj)n) ajoutée au système en même temps que l'enzyme de coupure, pour former le palindrome de coupure,
- P) Le palindrome est intra moléculaire, la séquence (x)n est alors introduite au début et à la fin du tag.

La séquence (y)m est une séquence d'acide nucléique spécifique à un type d'anticorps donné (comprenant de 7 à 100 nucléotides). Elle agit comme un code barre moléculaire et permet de compter le nombre et le type des anticorps qui se sont complexés.

Les séquences z minimisent les arrangements intempestifs entre séquences palindromiques intra ou inter tag. Par exemple, les séquences z sont définies telles que:
(zi)p=5'CACACACA3'
(zj)p=5'TGTGTGTG3'.
L'environnement des séquences palindromiques est rendu asymétrique par les séquences zi et zj, ce qui augmente donc la spécificité des coupures.

Une alternative à l'utilisation d'un site de restriction pour séparer le tag de l'anticorps consiste à utiliser un tag d'acide nucléique double brin complémentaire fixé à l'anticorps par l'une des deux extrémités d'un des brins. Une fois les complexes anticorps/protéines fixés aux billes et isolés, la dénaturation des tags fournit un mélange d'oligonucléitides libres en solution, la quantité d'oligonucléotides étant proportionnel aux anticorps retenus sur les billes. Il suffit alors d'analyser ce mélange sur une puce, tel que d'écrit ci-dessus.

### Exemple 4 : procédé mixte d'identification et de quantification protéine/ARNm

Il est possible de réaliser comme décrit en E et A un isolement successif des protéines et des ARNm d'une cellule et d'obtenir deux mélanges de polymères d'acides nucléiques, sonde primaire et tag, respectivement représentatif des ARNm et des protéines produits dans une cellule. Ces mélanges sont hybridés sur une puce (éventuellement deux puces : une pour les ARNm et une autre pour les protéines) permettant d'évaluer en une analyse pour un même lot cellulaire les ARNm et les protéines.
A = électrodes courbes de liaison (par exemple des électrodes transparentes en ITO gravées sur une lame de verre).
B= électrodes lignes fonctionnalisées (par exemple des électrodes transparentes en ITO gravées sur une lame de verre).
Bb = électrode spot fonctionnalisée.
Bc = guide ou masque de greffage des sondes
Bd = filtre polarisant à 0°.
Be = filtre polarisant à α°.
Bf = support transparent des électrodes.
Bg = sonde ADN simple brin greffée.
Bh = lumière polarisée dans un plan unique.
Bi = cibles moléculaires ADN à analyser.
Bj = ADN double brin.
C= électrode réservoir (par exemple des électrodes transparentes en ITO gravées sur une lame de verre).
D= capillaire sans plancher ni plafond (par exemple creusé dans du Kapton®) : plan médian.
E= capillaire sans plafond avec plancher (par exemple creusé dans du Kapton® ou PBMS) : plan médian.
F= réservoir sans plancher ni plafond (par exemple creusé dans du Kapton®) : plan médian,
G= électrodes lignes non fonctionnalisées (par exemple des électrodes transparentes en ITO gravées sur une lame de verre).
I= électrodes en ITO.
J= goutte de solution d'ADN.
K = spots de sondes : unité d'hybridation.
L= brin d'ADN étiré en train d'être absorbé sur l'électrode en ITO.
M= électrode horizontale de grille
N= électrode verticale de source.
O= électrode spot.
P= grille.
Q= source.
R= drain.
S= électrode en ITO recouverte de sondes ADN simple brin étiré (mis en évidence avec l'acridine orange, la coloration rouge orange caractéristique d'un simple brin).
T= électrode spot en ITO fonctionnalisée avec un oligonucléotide de 118 bases (S). L'électrode est hybridé avec un brin d'ADN complémentaire (mis en évidence avec l'acridine orange, la coloration verte caractéristique d'un duplex double brin ADN/ADN).
U= lavage dans une solution NaOH 0.1M.
V= hybridation avec le brin complémentaire.
X= électrode spot fonctionnalisée avec un simple brin lavée avec NaOH 0.1M puis hybridée avec un brin ADN complémentaire (mis en évidence avec l'acridine orange, la coloration verte caractéristique d'un duplex double brin ADN/ADN).
Z= électrode spot après déshybridation dans une solution NaOH 0.1 M (mis en évidence avec l'acridine orange, la coloration rouge orange caractéristique d'un simple brin.
TO= électrode en ITO nue.
T1= électrode en ITO fonctionnalisée avec un oligonucléotide de 118 bases.
Aa= tampon d'électrophorèse.
Ab= électrode à la surface de la goutte de tampon.
Ad= générateur basse fréquence.
Ac= électrode spot après hybridation : recouverte d'ADN double brin.
Ba= solution d'ADN complémentaire des séquences des sondes hybridées (sondes secondaires).
Ca= étape de rinçage.
Ae= électrode spot après hybridation et rinçage.
FET= transistor à effet de champ.
h= hauteur d'une maille du quadrillage.
I= largeur d'une maille du quadrillage.
Ω= Vannes pour l'injection de fluides dans les réservoirs de la puce.

## Revendications

1. Procédé d'analyse de cibles moléculaires contenues dans un mélange complexe comprenant :
a. la mise en contact en solution du mélange de cibles moléculaires à analyser avec un jeu de sondes primaires de types différents, chaque type de sonde primaire composant le jeu étant susceptible de se lier par liaison spécifique à un unique type de cible moléculaire du mélange complexe, dans des conditions permettant la liaison spécifique entre lesdites cibles moléculaires et lesdites sondes primaires, pour former un complexe sonde primaire-cible en solution, chaque sonde primaire étant constituée par un polynucléotide ou comprenant une partie formant un polynucléotide dont la séquence nucléotidique est connue et est, pour chaque type de sonde primaire, différente de toutes les séquences nucléotidiques des polynucléotides des autres types de sonde primaire,
b. éventuellement l'élimination des sondes primaires non spécifiquement liées avec une cible moléculaire,
c. la séparation des cibles moléculaires et des sondes primaires liées par liaison spécifique dans ledit complexe sonde primaire-cible, de façon à récupérer les sondes primaires représentant une empreinte des cibles moléculaires à analyser,
d. l'analyse des sondes primaires éluées à l'étape c, comportant l'identification et la quantification du polynucléotide de chaque sonde primaire éluée, en particulier par la détection d'un effet dû à la séquence nucléotidique, à la taille ou à la masse de ce polynucléotide.

2. Procédé d'analyse de cibles moléculaires selon la revendication 1, dans lequel les cibles moléculaires du mélange sont fixées sur des particules, par exemple des particules magnétiques, avant d'être mises en contact avec le jeu de sondes primaires.

3. Procédé d'analyse de cibles moléculaires selon la revendication 1 ou 2, dans lequel les cibles moléculaires sont des acides nucléiques.

4. Procédé d'analyse de cibles moléculaires selon la revendication 1 ou 2, dans lequel les cibles moléculaires sont des polypeptides ou sont à la fois des acides nucléiques et des polypeptides.

5. Procédé d'analyse de cibles moléculaires selon la revendication 1 ou 2, dans lequel le polynucléotide de chaque sonde primaire est destiné à se lier par une liaison spécifique et au moyen d'un seul site à un unique type de cible moléculaire dans le mélange.

6. Procédé d'analyse de cibles moléculaires selon la revendication 1 ou 2, dans lequel les sondes primaires du jeu de sondes comprennent une partie polypeptidique associée à un polynucléotide, chaque type de sonde primaire étant capable, par le biais de sa partie polypeptidique, de reconnaître et de lier de façon spécifique et au moyen d'un seul site à un type unique de cible moléculaire polypeptidique, la partie polynucléotidique de chaque type de sonde (appelée tag) étant en outre spécifique d'un type unique de cible moléculaire.

7. Procédé d'analyse de cibles moléculaires selon l'une quelconque des revendications précédentes, **caractérisé en ce que** l'étape a) est réalisée par mélange d'un excès de sondes primaires avec les cibles moléculaires.

8. Procédé d'analyse de cibles moléculaires selon l'une quelconque des revendications précédentes, dans lequel l'étape b) d'élimination des sondes primaires non spécifiquement liées avec les cibles moléculaires est effectuée après l'étape de séparation des sondes primaires contenues dans des complexes sonde-cible, préalablement à l'étape d) d'analyse des sondes primaires séparées.

9. Procédé selon l'une quelconque des revendications précédentes, dans lequel la séparation des sondes primaires et des cibles moléculaires pendant l'étape c) est obtenue en immobilisant initialement les cibles sur des particules magnétiques, et en appliquant un champ magnétique pour séparer les entités « cibles-particules magnétiques » des complexes sonde-cible et récupérer les sondes primaires des complexes, après les avoir séparées.

10. Procédé selon l'une quelconque des revendications 1 à 8, dans lequel la séparation des sondes primaires et des cibles moléculaires pendant l'étape c) est obtenue en immobilisant initialement les cibles moléculaires sur des particules, et en réalisant une centrifugation pour récupérer les sondes primaires, après les avoir séparées.

11. Procédé d'analyse de cibles moléculaires selon l'une quelconque des revendications précédentes, **caractérisé en ce que** les polynucléotides des sondes du jeu de sondes primaires ont tous une taille homogène, de préférence identique, et ont chacun une séquence déterminée pour obtenir des sondes primaires différentes les unes des autres.

12. Procédé d'analyse de cibles moléculaires selon la revendication 11, dans lequel l'analyse des polynucléotides des sondes primaires est effectuée au moyen d'un jeu de sondes polynucléotidiques dites secondaires, chaque type de sonde secondaire étant spécifique d'un unique type de sonde primaire.

13. Procédé selon la revendication 12, dans lequel l'analyse des sondes primaires séparées à l'étape c) comprend :
d. la mise en contact des sondes primaires séparées à l'étape c) avec des sondes secondaires de types différents, chaque type de sonde secondaire étant susceptible de se lier par liaison spécifique par hybridation à la partie polynucléotidique des sondes primaires,
e. la détermination des cibles moléculaires à partir de la détection, et/ou de la récupération et/ou de l'analyse de la partie polynucléotidique des sondes primaires hybridées aux sondes secondaires.

14. Procédé selon la revendication 13, **caractérisé en ce que** l'étape e) est réalisée en faisant circuler les sondes primaires sur les sondes secondaires immobilisées sur un support, par diffusion simple des sondes primaires ou par application de potentiels électriques.

15. Procédé selon la revendication 14, **caractérisé en ce que** l'application de potentiels électriques est réalisée par au moins un réseau d'électrodes.

16. Procédé d'analyse de cibles moléculaires selon l'une quelconque des revendications précédentes, **caractérisé en ce que** les polynucléotides des sondes du jeu de sondes primaires sont des Peptide Nucleic Acid (PNA), des composés mixtes acide nucléique / Peptide Nucleic Acid (PNA) ou des acides nucléiques modifiées.

17. Procédé selon l'une quelconque des revendications 13 à 17, **caractérisé en ce que** la liaison spécifique entre les sondes secondaires et les sondes primaires est une hybridation entre des séquences nucléotidiques complémentaires et **en ce que** la séparation des hybrides formés entre des sondes primaires et des sondes secondaires est obtenue par dénaturation contrôlée par élévation de la température.

18. Procédé selon l'une quelconque des revendications 12 à 17, **caractérisé en ce que** l'on utilise au moins un détecteur pour mesurer les variations d'impédance liées à l'hybridation des parties polynucléotidiques des sondes primaires et secondaires.

19. Procédé selon l'une quelconque des revendications 12 à 17, **caractérisé en ce que** l'on utilise au moins un détecteur pour mesurer les variations de lumière polarisée induites par l'hybridation des parties polynucléotidiques des sondes primaires et secondaires.

20. Procédé selon l'une quelconque des revendications 12 à 17, **caractérisé en ce que** l'on utilise au moins un détecteur pour mesurer par SPR l'hybridation des parties polynucléotidiques des sondes primaires et secondaires.

21. Procédé selon l'une quelconque des revendications 1 à 3, 5, et 7 à 20, **caractérisé en ce que** les cibles moléculaires à analyser sont des séquences nucléiques représentatives d'un transcriptome.

22. Procédé selon l'une quelconque des revendications 1, 2, 4, et 6 à 20, **caractérisé en ce que** les cibles moléculaires à analyser constituent un protéome.

23. Procédé selon l'une quelconque des revendications 1, 2, 4, 6 à 20, et 22, **caractérisé en ce que** l'étape a) comprend la mise en contact du mélange susceptible de comprendre des protéines à analyser avec un jeu de sondes primaires de types différents, constituées par un jeu d'anticorps ou de fragments d'anticorps comprenant un site de fixation à un antigène, liés chacun avec une séquence de polynucléotide spécifique (appelée séquence ou polynucléotide tag), chaque type d'anticorps ou de fragment d'anticorps composant le jeu étant susceptible de reconnaître un type unique de protéines à analyser, dans des conditions permettant la liaison spécifique entre lesdites protéines et lesdits anticorps ou fragments d'anticorps.

24. Procédé selon la revendication 23, **caractérisé en ce que** l'étape c) comprend la séparation des protéines et des anticorps ou fragments d'anticorps liées par liaison spécifique, puis la séparation de chaque anticorps ou fragment d'anticorps et de son polynucléotide tag spécifique, de façon à récupérer le jeu de polynucléotide tag représentant une empreinte des protéines à analyser.

25. Procédé selon la revendication 24, **caractérisé en ce que** l'étape d) d'analyse comprend la mise en contact des séquences tag séparées à l'étape c) avec des sondes secondaires de types différents, chaque type de sonde secondaire étant susceptible de se lier par liaison spécifique à un type de polynucléotides tag.

26. Procédé selon la revendication 25, **caractérisé en ce que** l'étape e) comprend la détection quantitative des protéines à partir de la détection, et/ou de la récupération et/ou de l'analyse des polynucléotides tag liés aux sondes secondaires.

27. Procédé selon l'une quelconque des revendications 1 à 11 et 21 à 27, **caractérisé en ce que** les polynucléotides des sondes primaires ont tous une masse différente et **en ce que** l'on utilise au moins un spectromètre de masse pour analyser ces polynucléotides.

28. Procédé selon l'une quelconque des revendications 23 à 26, **caractérisé en ce que** les polynucléotides des sondes primaires ont tous une taille différente et **en ce que** l'on analyse les polynucléotides par électrophorèse, ou chromatographie, ou filtration.

29. Procédé selon la revendication 1, **caractérisé en ce que** l'étape b) d'élimination des sondes primaires est effectuée par un traitement mettant en jeu des nucléases.

30. Procédé selon la revendication 1, **caractérisé en ce que** chaque sonde primaire est constituée par un polynucléotide ou comprend une partie formant un polynucléotide dont la séquence nucléotidique et la masse sont connues et sont, pour chaque type de sonde primaire, différentes de celles des polynucléotides des autres types de sonde primaire.

31. Dispositif pour l'exécution du procédé selon l'une quelconque des revendications 14 à 22, **caractérisé en ce qu'**il comprend :
- un réseau de capillaires (D) permettant la circulation de sondes primaires,
- une matrice de sondes secondaires organisées en spots (K), la matrice étant disposée de telle sorte qu'elle est en contact avec le réseau de capillaires,
- un réseau d'électrodes (B) dites fonctionnalisées, sur lequel sont fixées les sondes secondaires, ce réseau étant disposé de telle sorte que chaque ligne de spot de la matrice de spots est fixée sur une des électrodes fonctionnalisées dudit réseau, et
- un réseau d'électrodes (G) dites non fonctionnalisées, ce réseau étant disposé de telle sorte que le réseau de capillaires se situe entre les deux réseaux d'électrodes.

32. Dispositif selon la revendication 31, **caractérisé en ce qu'**il permet l'analyse des cibles moléculaires par le biais de l'analyse des sondes primaires.

33. Dispositif selon la revendication 32, **caractérisé en ce qu'**il permet la mesure des variations d'impédance liées à l'hybridation des sondes primaires et secondaires.

34. Dispositif selon la revendication 32, **caractérisé en ce qu'**il permet de mesurer par SPR l'hybridation des sondes primaires et secondaires.

35. Dispositif selon la revendication 32, **caractérisé en ce qu'**il permet la mesure de la variation de la lumière polarisée induite par la formation des hybrides sonde primaire-sonde secondaire.

36. Dispositif selon l'une quelconque des revendications 31 à 35, **caractérisé en ce que** les électrodes (B, G) sont gravées en couche mince sur un matériau isolant.

37. Dispositif selon la revendication 31, **caractérisé en ce que** le jeu d'électrodes fonctionnalisées (B) est situé au-dessus du réseau de capillaires (D) et **en ce que** le jeu d'électrodes non fonctionnalisées (G) est situé en dessous du réseau de capillaires.

38. Dispositif selon la revendication 31, **caractérisé en ce que** le jeu d'électrodes fonctionnalisées (B) est situé en dessous du réseau de capillaires (D) et **en ce que** le jeu d'électrodes non fonctionnalisées (G) est situé au-dessus du réseau de capillaires.

39. Dispositif selon l'une quelconque des revendications 31 à 38, **caractérisé en ce que** chaque spot (K) des électrodes fonctionnalisées est dans chaque capillaire du réseau de capillaires (D).

40. Dispositif selon l'une quelconque des revendications 31 à 38, **caractérisé en ce qu'**il comprend en outre un réservoir (F) à chaque extrémité du réseau de capillaires (D).

41. Dispositif selon la revendication 40, **caractérisé en ce que** le réservoir (F) comporte une électrode, dite électrode de réservoir (C).

42. Dispositif selon la revendication 41, **caractérisé en ce que** l'électrode de réservoir (C) contenue dans chaque réservoir (F) est située dans le même plan que les électrodes fonctionnalisées (B).

43. Dispositif selon la revendication 41 ou 42, **caractérisé en ce qu'**il comprend en outre une première et une deuxième électrodes de liaison (A) supplémentaires situées respectivement entre la première électrode de réservoir (F) et la première électrode fonctionnalisée (B), et entre la deuxième électrode de réservoir et la dernière électrode fonctionnalisée, de telle sorte que les distances les plus courtes entre chaque électrode de liaison et l'électrode de réservoir correspondante sont identiques en tout point des électrodes.

44. Dispositif selon l'une quelconque des revendications 40 à 43, **caractérisé en ce que** le second réservoir est formé par au moins un canal transversal, qui est relié en amont à l'ensemble des capillaires du réseau de capillaires (D) et en aval au détecteur.

45. Dispositif selon l'une quelconque des revendications 40 à 44, **caractérisé en ce que** chaque réservoir (F) est creusé dans l'épaisseur d'une plaque d'un matériau approprié.

46. Dispositif selon la revendication 31, **caractérisé en ce que** au moins l'un des réseaux d'électrodes (B, G) est formé par un quadrillage de type lignes, colonnes d'électrodes.

47. Dispositif selon la revendication 46, **caractérisé en ce que** l'autre réseau d'électrodes est relié à la masse.

48. Dispositif selon l'une quelconque des revendications 31 à 47, **caractérisé en ce que** le réseau d'électrodes fonctionnalisées (B) est formé par un quadrillage de type lignes, colonnes d'électrodes, et comprend dans chaque maille du quadrillage au moins une électrode spot (O) reliée à une électrode ligne et à une électrode colonne du quadrillage par l'intermédiaire d'un transistor à effet de champ (FET).

49. Dispositif selon la revendication 48, **caractérisé en ce que** le réseau d'électrodes fonctionnalisées (B) et le réseau d'électrodes non fonctionnalisées (G) sont formés par des quadrillages de types lignes, colonnes d'électrodes s'étendant en vis-à-vis de part et d'autre du réseau de capillaires, chaque quadrillage comportant dans chacune de ses mailles une électrode spot (O) reliée à une électrode ligne et à une électrode colonne du quadrillage par l'intermédiaire d'un transistor à effet de champ (FET).

50. Dispositif selon la revendication 48, **caractérisé en ce que** chaque maille du quadrillage comprend deux électrodes spots (O) qui sont chacune reliée à une électrode ligne et à une électrode colonne du quadrillage par l'intermédiaire d'un transistor à effet de champ (FET).

51. Dispositif pour la mise en oeuvre d'un procédé selon l'une quelconque des revendications 1 à 30, **caractérisé en ce qu'**il comprend:
- éventuellement un ensemble de particules, par exemple de particules magnétiques, pouvant fixer de manière forte les cibles moléculaires d'acides nucléiques et/ou de protéines du mélange à analyser ;
- un jeu de sondes primaires de types différents, en solution, lesdites sondes étant constituées par un polynucléotide ou comprenant un polynucléotide spécifique de chaque sonde, chaque type de sonde primaire composant le jeu étant susceptible de se lier par liaison spécifique à un unique type de cible moléculaires à analyser lorsque lesdites sondes primaires et lesdites cibles moléculaires sont mises en contact en solution, la séquence nucléotidique du polynucléotide de chaque type de sonde primaire étant connue et étant différente des séquences nucléotidiques des polynucléotides des autres types de sonde primaire, et le cas échéant,
- des moyens pour séparer et des moyens pour récupérer les sondes primaires et les cibles moléculaires liées par liaison spécifique, de façon à obtenir un jeu de sondes primaires représentant une empreinte des cibles moléculaires à analyser, et/ou le cas échéant,
- un jeu de sondes secondaires capables de reconnaître spécifiquement les parties polynucléotidiques des sondes primaires du jeu de sondes primaires.

52. Dispositif selon la revendication 51, **caractérisé en ce que** les moyens pour déshybrider les sondes primaires et les cibles moléculaires sont capables d'augmenter la température.

53. Dispositif selon la revendication 51 ou 52, **caractérisé en ce qu'**il comporte en outre des moyens d'analyse quantitative des sondes primaires séparées des cibles moléculaires, par exemple des moyens selon les revendications 29 à 48.

54. Jeu de sondes primaires convenant pour l'analyse des cibles moléculaires dans un mélange complexe, comprenant une population de sondes primaires de types différents, en solution, dans laquelle :
- chaque type de sonde primaire est différent des autres types de sondes primaires du jeu de sondes,
- chaque type de sonde primaire est susceptible de se lier, par liaison spécifique, à un unique type de cible moléculaire à analyser, lorsque lesdites sondes primaires et lesdites cibles moléculaires sont mises en contact,
- chaque type de sonde primaire (i) est un polynucléotide ou (ii) comprend un polynucléotide associé à une partie polypeptidique capable de lier spécifiquement un unique type de cible moléculaire du mélange complexe, chaque polynucléotide ayant une séquence nucléotidique connue et différente de celles des polynucléotides de toutes les autres sondes primaires du jeu de sondes, soit par l'enchaînement des nucléotides qui le compose, soit par sa taille, soit par sa masse, chacun des polynucléotides étant connu respectivement par sa séquence, par sa taille ou par sa masse, au sein du jeu de sondes.

55. Procédé d'analyse selon l'une des revendications 1 à 30 **caractérisé en ce qu'**il comprend, préalablement à l'étape a), des étapes de préparation du jeu de sondes primaires, consistant en :
- la définition d'un intervalle de taille des sondes primaires et la définition de conditions d'hybridation des sondes aux cibles et en particulier de la température de fusion Tm pour l'ensemble des sondes,
- la détermination du nombre i de cibles moléculaires polynucléotidiques de séquences différentes du mélange complexe,
- l'identification, pour chaque séquence cible i potentiellement contenue dans l'échantillon, d'une collection de Nᵢ sondes possibles parfaitement hybridables avec la cible considérée et dont la taille est comprise dans l'intervalle de taille choisi et le Tm est compris dans les valeurs choisies,
- la sélection au sein de la susdite collection de Nᵢ sondes, d'une population de nᵢ séquences polynucléotidiques pouvant se lier par hybridation à la cible moléculaire constituée par la séquence i considérée et dont la liaison ainsi formée est spécifique, excluant dès lors une hybridation avec les autres séquences cibles possibles du mélange,
- au sein de cette population de nᵢ sondes putatives pour la constitution de sondes, l'attribution à chaque polynucléotide, d'une masse différente de la masse des autres polynucléotides de la population, le cas échéant en modifiant des nucléotides qu'il contient, par exemple par méthylation ou éthylation d'un ou plusieurs résidus cytosine, pour obtenir une collection de polynucléotides ayant tous une masse différente et connue pour chaque séquence, la comparaison étant répétée de façon récursive à chaque modification de séquences,
- l'identification d'une collection de Zᵢ combinaisons de i sondes primaires pour chaque séquence i, chacune de ces combinaisons comprenant une unique sonde primaire de chaque population de nᵢ sondes examinée ; chacune des sondes primaires de la population de nᵢ sondes étant comparée aux sondes primaires des combinaisons précédemment établies, et lorsque les masses de ces sondes primaires sont trop proches l'une de l'autre, la modification de leur séquence, la comparaison étant répétée de façon récursive à chaque modification de séquence,
- le classement des Zᵢ combinaisons de i sondes primaires par ordre croissant de la somme des masses des i sondes primaires qui composent la combinaison et par ordre croissant du nombre de groupements de modification,
- l'identification d'une collection de Zᵢ combinaisons de i sondes primaires pour les i séquences examinées,
- la synthèse des sondes primaires du jeu de sondes ainsi défini.

## Patentansprüche

1. Verfahren zur Analyse von molekularen Zielen, die in einer komplexen Mischung enthalten sind, umfassend:
a. In-Kontakt-Bringen in Lösung der Mischung aus molekularen Zielen, die analysiert werden sollen, mit einem Satz von primären Sonden verschiedener Arten, wobei jede Art von Sonde, aus denen der Satz besteht, durch spezifische Verbindung mit einer einzigen Art von molekularem Ziel der komplexen Mischung verbunden werden kann, unter Bedingungen, die die spezifische Verbindung zwischen den molekularen Zielen und den primären Sonden ermöglichen, um einen primäre Sonde-Ziel Komplex in Lösung zu bilden, wobei jede primäre Sonde aus einem Polynukloeotid besteht oder einen Teil umfasst, der ein Polynukleotid bildet, dessen Nukleotidsequenz bekannt ist und, für jede Art von primärer Sonde, verschieden von allen Nukleotidsequenzen der Polynukleotide der anderen Arten von primärer Sonde ist.
b. eventuell die Beseitigung der primären Sonden, die nicht spezifisch mit einem molekularen Ziel verbunden sind,
c. die Trennung der molekularen Ziele und der primären Sonden, verbunden durch spezifische Verbindung in demprimäre Sonde-Ziel Komplex, um die primären Sonden wiederzugewinnen, die einen Abdruck der molekularen Ziele darstellen, die analysiert werden sollen.
d. Analyse der primären Sonden, die in Schritt c eluiert wurden, umfassend die Identifizierung und die Quantifizierung des Polynukleotids jeder eluierten primären Sonde, insbesondere durch den Nachweis der Wirkung auf Grund der Nukleotidsequenz, der Größe oder der Masse dieses Polynukleotids.

2. Verfahren zur Analyse von molekularen Zielen nach Anspruch 1, wobei die molekularen Ziele der Mischung auf Partikeln fixiert sind, z.B. magnetischen Partikeln, bevor sie mit dem Satz von primären Sonden in Kontakt gebracht werden.

3. Verfahren zur Analyse von molekularen Zielen nach Anspruch 1 oder 2, wobei die molekularen Ziele Nukleinsäuren sind.

4. Verfahren zur Analyse von molekularen Zielen nach Anspruch 1 oder 2, wobei die molekularen Ziele Polypeptide sind oder gleichzeitig Nukleinsäuren und Polypeptide sind.

5. Verfahren zur Analyse von molekularen Zielen nach Anspruch 1 oder 2, wobei das Polynukleotid jeder primären Sonde ausgelegt ist, um sich durch eine spezifische Verbindung und mit Hilfe einer einzigen Stelle mit einer einzigen Art von molekularem Ziel in der Mischung zu verbinden.

6. Verfahren zur Analyse von molekularen Zielen nach Anspruch 1 oder 2, wobei die primären Sonden des Satzes von Sonden einen Polypeptidteil umfassen, der mit einem Polynukleotid assoziiert ist, wobei jede Art von primärer Sonde dazu in der Lage ist, durch seinen Polypeptidteil eine einzige Art von Polypeptid-Molekularziel zu erkennen und auf spezifische weise und mit Hilfe einer einziger Stelle damit zu verbinden, wobei der Polynukleotidteil jeder Art von Sonde (genannt Tag) außerdem spezifisch für eine einzige Art von molekularem Ziel ist.

7. Verfahren zur Analyse von molekularen Zielen nach irgendeinem der vorhergehenden Ansprüche, **dadurch gekennzeichnet, dass** Schritt a) durch die Mischung eines Überschusses von primären Sonden mit den molekularen Zielen durchgeführt wird.

8. Verfahren zur Analyse von molekularen Zielen nach irgendeinem der vorhergehenden Ansprüche, **dadurch gekennzeichnet, dass** der Schritt b) des Beseitigens der primären Sonden, die nicht spezifisch mit den molekularen Zielen verbunden sind, nach dem Schritt des Trennens der primären Sonden, die in Sonde-Ziel Komplexen enthalten sind, durchgeführt wird, vor dem Schritt d) des Analysierens der getrennten primären Sonden.

9. Verfahren nach irgendeinem der vorhergehenden Ansprüche, wobei die Trennung der primären Sonden und der molekularen Ziele während Schritt c) durch die anfängliche Immobilisierung der Ziele auf magnetischen Partikeln und durch die Anwendung eines magnetischen Felds , um die "Ziel-magnetische Partikel" Einheinten der Sonde-Ziel Komplexe zu trennen und die primären Sonden der Komplexe wiederzugewinnen, nachdem sie getrennt wurden, erhalten wird.

10. Verfahren nach irgendeinem der Ansprüche 1 bis 8, wobei die Trennung der primären Sonden und der molekularen Ziele während Schritt c) durch die anfängliche Immobilisierung der molekularen Ziele auf Partikeln und durch die Durchführung einer Zentrifugierung, um die primären Sonden wiederzugewinnen, nachdem sie getrennt wurden, erhalten wird.

11. Verfahren zur Analyse von molekularen Zielen nach irgendeinem der vorhergehenden Ansprüche, **dadurch gekennzeichnet, dass** die Polynukleotide der Sonde des Satzes von primären Sonden alle eine homogene Größe aufweisen, vorzugsweise identisch, und jeweils über eine bestimmte Sequenz verfügen, um untereinander verschiedene primäre Sonden zu erhalten.

12. Verfahren zur Analyse von molekularen Zielen nach Anspruch 11, wobei die Analyse der Polynukleotide der primären Sonden mit Hilfe eines Satzes von Polynukleotidsonden, genannt sekundär, durchgeführt wird, wobei jede Art von sekundärer Sonde spezifisch für eine einzige Art von primärer Sonde ist.

13. Verfahren nach Anspruch 12, wobei die Analyse der getrennten primären Sonden in Schritt c) Folgendes umfasst:
d. das In-Kontakt-Bringen der in Schritt c) getrennten primären Sonden mit sekundären Sonden verschiedener Arten, wobei jede Art von sekundärer Sonde sich durch eine spezifische Bindung durch Hybridisierung mit dem Polynukleotidteil der primären Sonden verbinden kann,
e. die Bestimmung der molekularen Ziele aufgrund des Nachweises und/oder die Wiedergewinnung und/oder die Analyse des Polynukleotidteils der mit den sekundären Sonden hybridisierten primären Sonden.

14. Verfahren nach Anspruch 13, **dadurch gekennzeichnet, dass** Schritt e) durchgeführt wird, indem die primären Sonden auf den auf einem Träger immobilisierten sekundären Sonden durch einfache Diffusion der primären Sonden oder durch Anwendung elektrischen Potenziale zirkulieren werden.

15. Verfahren nach Anspruch 14, **dadurch gekennzeichnet, dass** die Anwendung elektrischen Potenziale durch mindestens ein Netz von Elektroden durchgeführt wird.

16. Verfahren zur Analyse von molekularen Zielen nach irgendeinem der vorhergehenden Ansprüche, **dadurch gekennzeichnet, dass** die Polynukleotide der Sonden des Satzes von primären Sonden Peptidnukleinsäure (PNA), gemischte Nukleinsäure / Peptidnukleinsäure (PNA) Verbindungen oder modifizierte Nukleinsäuren sind.

17. Verfahren nach irgendeinem der Ansprüche 13 bis 17, **dadurch gekennzeichnet, dass** die spezifische Verbindung zwischen des sekundären Sonden und den primären Sonden eine Hybridisierung zwischen komplementären Nukleotidsequenzen ist, und dadurch, dass die Trennung des Hybride zwischen primären Sonden und sekundären Sonden durch eine kontrollierte Denaturierung durch Erhöhung der Temperatur erhalten wird.

18. Verfahren nach irgendeinem der Ansprüche 12 bis 17, **dadurch gekennzeichnet, dass** mindestens ein Detektor verwendet wird, um die Variationen der Impedanz zu messen, die mit der Hybridisierung der Polynukleotidteile der primären und sekundären Sonden verbunden sind.

19. Verfahren nach irgendeinem der Ansprüche 12 bis 17, **dadurch gekennzeichnet, dass** mindestens ein Detektor verwendet wird, um die Variationen polarisierten Lichts zu messen, die mit der Hybridisierung der Polynukleotidteile der primären und sekundären Sonden induziert werden.

20. Verfahren nach irgendeinem der Ansprüche 12 bis 17, **dadurch gekennzeichnet, dass** mindestens ein Detektor verwendet wird, um durch SPR die Hybridisierung der Polynukleotidteile der primären und sekundären Sonden zu messen.

21. Verfahren nach irgendeinem der Ansprüche 1 bis 3, 5, und 7 bis 20, **dadurch gekennzeichnet, dass** die molekularen Ziele, die analysiert werden sollen, Nukleinsequenzen sind, die ein Transkriptom darstellen.

22. Verfahren nach irgendeinem der Ansprüche 1, 2, 4 und 6 bis 20, **dadurch gekennzeichnet, dass** die molekularen Ziele, die analysiert werden sollen, ein Proteom darstellen.

23. Verfahren nach irgendeinem der Ansprüche 1, 2, 4, 6 bis 20 und 22, **dadurch gekennzeichnet, dass** Schritt a) Folgendes umfasst: das In-Kontakt-Bringen der Mischung, die Proteine enthalten kann, die analysiert werden sollen, mit einem Satz von primären Sonden verschiedener Arten, die aus einem Satz von Antikörpern oder Fragmenten von Antikörpern umfassend eine Stelle zur Befestigung an ein Antigen bestehen, jeweils verbunden mit einer Sequenz von spezifischen Polynukleotiden (genannt Tag-Sequenz oder -Polynukleotid), wobei jede Art von Antikörpern oder Antikörperfragmenten aus denen der Satz besteht, eine einzige Art von Proteinen, die analysiert werden soll, erkennen kann, unter Bedingungen, die die spezifische Verbindung zwischen den Proteinen und den Antikörpern oder Antikörperfragmenten ermöglichen.

24. Verfahren nach Anspruch 23, **dadurch gekennzeichnet, dass** Schritt c) die Trennung der Proteine und der Antikörper oder Antikörperfragmente umfasst, die durch eine spezifische Verbindung verbunden sind, dann die Trennung jedes Antikörpers oder Antikörperfragments und seines spezifischen Tag-Polynukleotid, um den Satz von Tag-Polynukloetiden wiederzugewinnen, der einen Abdruck der Proteine darstellt, die analysiert werden sollen.

25. Verfahren nach Anspruch 24, **dadurch gekennzeichnet, dass** Schritt d) des Analysierens das In-Kontakt-Bringen der in Schritt c) getrennten Tag-Sequenzen mit sekundären Sonden verschiedener Arten umfasst, wobei jede Art von sekundärer Sonde durch eine spezifische Verbindung mit einer Art von Polynukleotid-Tag verbunden werden kann.

26. Verfahren nach Anspruch 25, **dadurch gekennzeichnet, dass** Schritte e) das quantitative Nachweisen der Proteine auf der Grundlage des Nachweises und/oder der Wiedergewinnung und/oder der Analyse der Tag-Polynukleotide umfasst, die mit den sekundären Sonden verbunden sind.

27. Verfahren nach irgendeinem der Ansprüche 1 bis 11 und 21 bis 27, **dadurch gekennzeichnet, dass** die Polynukleotide der primären Sonden alle eine verschiedene Masse aufweisen und dadurch, dass mindestens ein Massenspektrometer verwendet wird, um diese Polynukleotide zu analysieren.

28. Verfahren nach irgendeinem der Ansprüche 23 bis 26, **dadurch gekennzeichnet, dass** die Polynukleotide der primären Sonden alle eine verschiedene Größe aufweisen und dadurch, dass die Polynukleotide durch Elektrophorese, Chromatographie oder Filtrierung analysiert werden.

29. Verfahren nach Anspruch 1, **dadurch gekennzeichnet, dass** Schritt b) der Beseitigung der primären Sonden durch eine Behandlung durchgeführt wird, die die Nukleasen einsetzt.

30. Verfahren nach Anspruch 1, **dadurch gekennzeichnet, dass** jede primäre Sonde aus einem Polynukleotid besteht oder einen Teil umfasst, der ein Polynukleotid bildet, von dem die Nukleotidsequenz und die Masse bekannt sind und für jede Art von primärer Sonde verschieden von denjenigen der Polynukleotide der anderen Arten von primärer Sonde sind.

31. Vorrichtung zur Durchführung des Verfahrens nach irgendeinem der Ansprüche 14 bis 22, **dadurch gekennzeichnet, dass** es Folgendes umfasst:
- ein Netz von Kapillaren (D), das die Zirkulierung der primären Sonden ermöglicht,
- eine Matrix von sekundären Sonden, die in Flecken (K) organisiert sind, wobei die Matrix derart angeordnet ist, das sie mit dem Netz von Kapillaren in Kontakt steht,
- ein Netz von Elektroden (B), genannt funktionalisiert, auf dem die sekundären Sonden fixiert sind, wobei dieses Netz derart angeordnet ist, dass jede Flecklinie der Matrix von Flecken auf einem der funktionalisierten Elektroden des Netzes fixiert ist; und
- ein Netz von Elektroden (G), genannt nicht funktionalisiert, wobei dieses Netz derart angeordnet ist, dass sich das Netz von Kapillaren zwischen den zwei Netzen von Elektroden befindet.

32. Vorrichtung nach Anspruch 31, **dadurch gekennzeichnet, dass** sie die Analyse der molekularen Ziele mit Hilfe der Analyse der primären Sonden ermöglicht.

33. Vorrichtung nach Anspruch 32, **dadurch gekennzeichnet, dass** sie die Messung der Variationen der Impedanz ermöglicht, die mit der Hybridisierung der primären und sekundären Sonden verbunden sind.

34. Vorrichtung nach Anspruch 32, **dadurch gekennzeichnet, dass** sie ermöglicht, durch SPR die Hybridisierung der primären und sekundären Sonden zu messen.

35. Vorrichtung nach Anspruch 32, **dadurch gekennzeichnet, dass** sie ermöglicht, die Variation des polarisierten Lichts zu messen, die durch die Bildung der primäre Sonde-sekudäre Sonde Hybride induziert wird.

36. Vorrichtung nach irgendeinem der Ansprüche 31 bis 35, **dadurch gekennzeichnet, dass** die Elektroden (B, G) in einer dünnen Schicht auf einem isolierenden Material graviert sind.

37. Vorrichtung nach Anspruch 31, **dadurch gekennzeichnet, dass** sich der Satz von funktionalisierten Elektroden (B) über dem Netz von Kapillaren (D) befindet, und dadurch, dass sich der Satz von nicht funktionalisierten Elektroden (G) unter dem Netz von Kapillaren befindet.

38. Vorrichtung nach Anspruch 31, **dadurch gekennzeichnet, dass** sich der Satz von funktionalisierten Elektroden (B) unter dem Netz von Kapillaren (D) befindet, und dadurch, dass sich der Satz von nicht funktionalisierten Elektroden (G) über dem Netz von Kapillaren befindet.

39. Vorrichtung nach irgendeinem der Ansprüche 31 bis 38, **dadurch gekennzeichnet, dass** sich jeder Fleck (K) der funktionalisierten Elektroden in jedem Kapillar des Netzes von Kapillaren (D) befindet.

40. Verfahren nach irgendeinem der Ansprüche 31 bis 38, **dadurch gekennzeichnet, dass** es außerdem einen Behälter (F) an jedem Ende des Netzes von Kapillaren (D) umfasst.

41. Vorrichtung nach Anspruch 40, **dadurch gekennzeichnet, dass** der Behälter (F) eine Elektrode, genannt Behälterelektrode (C), umfasst.

42. Vorrichtung nach Anspruch 41, **dadurch gekennzeichnet, dass** die Behälterelektrode (C), die in jedem Behälter (f) enthalten ist, sich auf der gleichen Ebene wie die funktionalisierten Elektroden (B) befindet

43. Vorrichtung nach Anspruch 41 oder 42, **dadurch gekennzeichnet, dass** sie außerdem eine zusätzliche erste und zweite Verbindungselektrode (A) umfasst, die sich jeweils zwischen der ersten Behälterelektrode (F) und der ersten funktionalisierten Elektrode (B) und zwischen der zweiten Behälterelektrode und der letzten funktionalisierten Elektrode befindet, so dass die kürzesten Abstände zwischen jeder Verbindungselektrode und der entsprechenden Behälterelektrode an jedem Punkt der Elektroden konstant sind.

44. Vorrichtung nach irgendeinem der Ansprüche 40 bis 43, **dadurch gekennzeichnet, dass** der zweite Behälter durch mindestens einen quer liegenden Kanal gebildet ist, der vorgelagert mit der Gesamtheit der Kapillaren des Kapillarennetzes (D) und nachgelagert mit dem Detektor verbunden ist.

45. Vorrichtung nach irgendeinem der Ansprüche 40 bis 44, **dadurch gekennzeichnet, dass** jeder Behälter (F) in der Dicke einer Platte aus einem geeigneten Material gebohrt ist.

46. Vorrichtung nach Anspruch 31, **dadurch gekennzeichnet, dass** mindestens eines der Elektrodennetze (B, G) durch ein Raster von der Elektroden Linien, -Säulen Art gebildet ist.

47. Vorrichtung nach Anspruch 46, **dadurch gekennzeichnet, dass** das andere Elektrodennetz mit der Masse verbunden ist.

48. Vorrichtung nach irgendeinem der Ansprüche 31 bis 47, **dadurch gekennzeichnet, dass** das Netz von funktionalisierten Elektroden (B) durch ein Raster von der Elektroden Linien, -Säulen Art gebildet ist und in jeder Masche des Rasters mindestens eine Punktelektrode (O) umfasst, die mit einer Linienelektrode und mit einer Säulenelektrode des Rasters mit Hilfe eines Transistors mit Feldeffekt (FET) verbunden sind.

49. Vorrichtung nach Anspruch 48, **dadurch gekennzeichnet, dass** das Netz von funktionalisierten Elektroden (B) und das Netz von nicht funktionalisierten Elektroden (G) aus Rastern von der Elektroden Linien, -Säulen Art gebildet sind, wobei sich Elektrodensäulen sich gegenüber beiden Seiten des Kapillarnetzes erstrecken, wobei jedes Raster in jeder seiner Maschen eine Punktelektrode (O) umfasst, die mit einer Linienelektrode und mit einer Säulenelektrode des Rasters mit Hilfe eines Transistors mit Feldeffekt (FET) verlinkt sind.

50. Vorrichtung nach Anspruch 48, **dadurch gekennzeichnet, dass** jede Masche des Rasters zwei Punktelektroden (O) umfasst, die jeweils mit einer Linienelektrode und mit einer Säulenelektrode des Rasters mit Hilfe eines Transistors mit Feldeffekt (FET) verlinkt sind.

51. Vorrichtung zur Durchführung des Verfahrens nach irgendeinem der Ansprüche 1 bis 30, **dadurch gekennzeichnet, dass** es Folgendes umfasst:
- eventuell eine Einheit von Partikel, z.B. magnetischen Partikeln, die die molekularen Ziele von Nukleinsäuren und/oder von Proteinen des Mischung, die analysiert werden sollen, auf starke Weise fixieren kann,
- einen Satz von primären Sonden verschiedener Art in Lösung, wobei die Sonden aus einem Polynukleotid bestehen oder ein Polynukleotid umfassen, spezifisch jeder Sonde, wobei jede Art von primärer Sonde, aus denen der Satz besteht, durch spezifische Verbindung mit einer einzigen Art von molekularem Ziel, das analysiert werden soll, verbunden werden kann, wenn die primären Sonden und die molekularen Ziele in Lösung miteinander in Kontakt gebracht werden, wobei die Nukleotidsequenz des Polynukleotids jeder Art von primäre Sonde bekannt ist und verschieden von Nukleotidsequenzen der Polynukleotide der anderen Arten von primärer Sonde bekannt ist, und gegebenenfalls
- Mittel zum Trennen und Mittel, um die primären Sonden und die molekularen Ziele wiederzugewinnen, die durch spezifische Verbindung verbunden sind, um einen Satz von primären Sonden zu erhalten, die einen Abdruck der molekularen Ziele, die analysiert werden sollen, darstellen, und/oder gegebenenfalls
- einen Satz von sekundären Sonden, der dazu in der Lage sind, spezifisch die Polynukleotidteile der primären Sonden des Satzes von primären Sonden zu erkennen.

52. Vorrichtung nach Anspruch 51, **dadurch gekennzeichnet, dass** die Mittel, um die primären Sonden und die molekularen Ziel zu dehybridisieren, die Temperatur erhöhen können.

53. Vorrichtung nach Anspruch 51 oder 52, **dadurch gekennzeichnet, dass** es außerdem Mittel zur quantitativen Analyse der von der molekularen Ziele getrennten primären Sonden umfasst, z.B. Mittel nach den Ansprüchen 29 bis 48.

54. Satz von primären Sonden, die für die Analyse der molekularen Ziele in einer komplexen Mischung zweckmäßig ist, umfassend eine Population von verschiedenen Arten von primären Sonden, wobei
- jede Art von primärer Sonde verschieden von den anderen Arten von primären Sonden des Satzes von Sonden ist,
- jede Art von primärer Sonde durch spezifische Verbindung mit einer einzigen Art von molekularem Ziel, das analysiert werden soll, verbunden werden kann, wenn die primären Sonden und die molekularen Ziele miteinander in Kontakt gebracht werden,
- jede Art von primärer Sonde (i) ein Polynukleotid ist oder (ii) ein Polynukleotid umfasst, das mit einem Polypeptidteil assoziiert ist, der in der Lage ist, spezifisch einen einzige Art von molekularem Ziel der komplexen Mischung zu verbinden, wobei jedes Polynukleotid ein Polynukleotidsequenz aufweist die bekannt und verschieden von denjenigen der Polynukleotide von allen anderen primären Sonden des Satzes von Sonden ist, entweder durch die Verkettung der Nukleotide, aus denen es besteht, oder durch seine Größe oder durch seine Masse, wodurch jedes der Polynukleotide entweder durch seine Sequenz, durch seine Größe oder durch seine Masse innerhalb des Satzes von Sonden bekannt ist.

55. Verfahren nach einem der Ansprüche 1 bis 30, **dadurch gekennzeichnet, dass** es, vor Schritt a), Schritte zur Vorbereitung des Satzes von primären Sonden umfasst, bestehend aus:
- der Definition eines Größenintervalls der primären Sonden, und die Definition der Bedingungen der Hybridisierung des Sonden an die Ziele und insbesondere der Fusionstemperatur Tm für die Gesamtheit der Sonden,
- die Bestimmung der Anzahl i der molekularen Polynukleotidziele von verschiedenen Sequenzen der komplexen Mischung,
- die Identifizierung, für jede Zielsequenz i, die potenziell in der Probe enthalten ist, einen Sammlung von Nᵢ möglichen Sonden, die perfekt mit der in Betracht gezogenen Ziele hybridisieren können, und deren die Größe in dem Größenintervalls liegt uned die Tm innerhalb gewählten werten liegt,
- Auswahl, in der Sammlung von Nᵢ Sonden, einer Population von nᵢ Polynukleotidsequenzen die durch Hybridisierung an das molekulare Ziel binden können, das aus der in Betracht gezogenen Sequenz i besteht, und deren so gebildete Bindung spezifisch ist, wobei infolgedessen eine Hybridisierung mit den anderen möglichen Zielsequenzen der Mischung ausgeschlossen ist,
- innerhalb dieser Population von nᵢ vermeintlichen Sonden für den Aufbau von Sonden, Zuweisung an jedes Polynukleotid einer Masse, die verschieden von der Masse der anderen Polynukleotide der Population ist, gegebenenfalls indem Nukleotide, die es enthält, modifiziert werden, z.B. durch Methylierung oder Ethylierung eines oder mehrerer Zytosinrückstände, um eine Sammlung von Polynukleotiden zu erhalten, die alle eine verschiedene und bekannte Masse enthalten, wobei der Vergleich bei jeder Sequenzmodifizierung auf rekursive Weise wiederholt wird,
- Identifizierung einer Sammlung von Zᵢ Kombinationen mit i primären Sonden, für jede Sequenz i, wobei jede dieser Kombinationen eine einzige primäre Sonde jeder Population von nᵢ untersuchten Sonden umfasst; wobei jede der primären Sonden der Population von nᵢ Sonden mit den primären Sonden der zuvor festgesetzten Kombinationen verglichen wird, und wenn die Massen der primären Sonden zu nahe aneinander sind, Modifizierung ihrer Sequenz, wobei der Vergleich bei jeder Sequenzmodifizierung auf rekursive Weise wiederholt wird,
- Klassifizierung der Zᵢ Kombinationen von i primären Sonden in aufsteigender Reihenfolge der Summe der Massen der i primären Sonden, aus denen die Komponente besteht, und in aufsteigender Reihenfolge der Anzahl der Modifizierungsgruppen,
- Identifizierung einer Sammlung von Zᵢ Kombinationen mit i primären Sonden für die untersuchten i Sequenzen,
- die Synthese der primären Sonden des so definierten Satzes von Sonden.

## Claims

1. Method for analysing molecular targets contained in a complex mixture comprising:
a. contacting in solution the mixture of molecular targets to be analysed with a set of primary probes of different types, each type of primary probe forming the set being able to bind by a specific bond a single type of molecular target of the complex mixture, under conditions enabling the specific binding between said molecular targets and said primary probes, to form a primary probe-target complex in solution, each primary probe consisting of a polynucleotide or comprising a portion forming a polynucleotide of which the nucleotide sequence is known and is, for each type of primary probe, different to all the nucleotide sequences of the polynucleotides of the other types of primary probe,
b. optionally removing the primary probes not specifically bound with a molecular target,
c. separating the molecular targets and the primary probes bound by a specific bond in said primary probe-target complex, so as to retrieve the primary probe representing a imprint of the molecular targets to be analysed,
d. analysing the primary probes eluted in step c, including the identification and quantification of the polynucleotide of each primary probe eluted, in particular by detecting an effect due to the nucleotide sequence, the size or the mass of this polynucleotide.

2. Method for analysing molecular targets according to claim 1, wherein the molecular targets of the mixture are fixed onto particles, for example magnetic particles, before being contacted with the set of primary probes.

3. Method for analysing molecular targets according to claim 1 or 2, wherein the molecular targets are nucleic acids.

4. Method for analysing molecular targets according to claim 1 or 2, wherein the molecular targets are polypeptides or are both nucleic acids and polypeptides.

5. Method for analysing molecular targets according to claim 1 or 2, wherein the polynucleotide of each primary probe is intended to be bound by a specific bond and by means of a single site to a single type of molecular target in the mixture.

6. Method for analysing molecular targets according to claim 1 or 2, wherein the primary probes of the set of probes comprise a polypeptide portion associated with a polynucleotide, each type of primary probe being capable, via its polypeptide portion, of recognising and binding specifically and by means of a single site with a single type of polypeptide molecular target, the polynucleotide portion of each type of probe (referred to as a tag) being further specific for a single type of molecular target.

7. Method for analysing molecular targets according to any one of the above claims, **characterised in that** step a) is performed by mixing an excess of primary probes with the molecular targets.

8. Method for analysing molecular targets according to any one of the above claims, wherein step b) of removing the primary probes not specifically bound with the molecular targets is performed after the step of separating the primary probes contained in probe-target complexes, prior to step d) of analysing the separated primary probes.

9. Method according to any one of the above claims, wherein the separation of the primary probes and the molecular targets during step c) is obtained by initially immobilising the targets on magnetic particles, and applying a magnetic field to separate the "target-magnetic particle" entities from the probe-target complexes and retrieve the primary probes from the complexes, after having separated them.

10. Method according to any one of claims 1 to 8, wherein the separation of the primary probes and the molecular targets during step c) is obtained by initially immobilising the molecular targets on particles, and carrying out a centrifugation to retrieve the primary probes, after having separated them.

11. Method for analysing molecular targets according to any one of the above claims, **characterised in that** the polynucleotides of the probes of the set of primary probes all have a homogeneous size, preferably identical, and have each a defined sequence in order to obtain primary probes different from one antother.

12. Method for analysing molecular targets according to claim 11, wherein the analysis of the polynucleotides of the primary probes is performed by means of a set of polynucleotide probes, so-called secondary, each type of secondary probe being specific for a single type of primary probe.

13. Method according to claim 12, wherein the analysis of the primary probes separated in step c) comprises:
d. contacting the primary probes separated in step c) with secondary probes of different types, each type of secondary probe being able to bind by a specific bond by hybridisation with the polynucleotide portion of the primary probes,
e. determining the molecular targets on the basis of the detection, and/or retrieval and/or analysis of the polynucleotide portion of the primary probes hybridised with the secondary probes.

14. Method according to claim 13, **characterised in that** step e) is performed by circulating the primary probes on the secondary probes immobilised on a substrate, by plain diffusion of the primary probes or by applying electrical potentials.

15. Method according to claim 14, **characterised in that** electrical potentials are applied by means of at least one electrode array.

16. Method for analysing molecular targets according to any one of the above claims, **characterised in that** the polynucleotides of the probes of the set of primary probes are Peptide Nucleic Acids (PNA), composite nucleic acid / Peptide Nucleic Acid (PNA) compounds or modified nucleic acids.

17. Method according to any one of claims 13 to 17, **characterised in that** the specific bond between the secondary probes and the primary probes is a hybridisation between complementary nucleotide sequences and **in that** the separation of the hybrids formed between primary probes and secondary probes is obtained by means of controlled denaturing by raising the temperature.

18. Method according to any one of claims 12 to 17, **characterised in that** at least one detector is used for measuring the impedance variations associated with the hybridisation of the polynucleotide portions of the primary and secondary probes.

19. Method according to any one of claims 12 to 17, **characterised in that** at least one detector is used for measuring the polarised light variations induced by the hybridisation of the polynucleotide portions of the primary and secondary probes.

20. Method according to any one of claims 12 to 17, **characterised in that** at least one detector is used for measuring by means of SPR the hybridisation of the polynucleotide portions of the primary and secondary probes.

21. Method according to any one of claims 1 to 3, 5, and 7 to 20, **characterised in that** the molecular targets to be analysed are nucleic sequences representative of a transcriptome.

22. Method according to any one of claims 1, 2, 4, and 6 to 20, **characterised in that** the molecular targets to be analysed form a proteome.

23. Method according to any one of claims 1, 2, 4, 6 to 20, and 22, **characterised in that** step a) includes contacting the mixture which may comprise proteins to be analysed with a set of primary probes of different types, consisting of a set of antibodies or antibody fragments comprising an antigen-binding site, each bound with a specific polynucleotide sequence (referred to as a tag sequence or polynucleotide), each type of antibody or antibody fragment forming the set being able to recognise a single type of proteins to be analysed, under conditions suitable for the specific binding between said proteins and said antibodies or antibody fragments.

24. Method according to claim 23, **characterised in that** step c) includes separating the proteins and the antibodies or antibody fragments bound by a specific bond, then separating each antibody or antibody fragment and its specific tag polynucleotide , so as to retrieve the set of tag polynucleotides representing an imprint of the proteins to be analysed.

25. Method according to claim 24, **characterised in that** the analysis step d) includes contacting the tag sequences separated in step c) with secondary probes of different types, each type of secondary probe being suitable for binding by a specific bond with a type of tag polynucleotide.

26. Method according to claim 25, **characterised in that** step e) includes the quantitative detection of the proteins on the basis of the detection, and/or retrieval and/or analysis of the tag polynucleotides associated with the secondary probes.

27. Method according to any one of claims 1 to 11 and 21 to 27, **characterised in that** the polynucleotides of the primary probes all have a different mass and **in that** at least one mass spectrometer is used for analysing these polynucleotides.

28. Method according to any one of claims 23 to 26, **characterised in that** the polynucleotides of the primary probes all have a different size and **in that** the polynucleotides are analysed by electrophoresis, or chromatography, or filtration.

29. Method according to claim 1, **characterised in that** step b) of removing the primary probes is performed by means of a treatment involving nucleases.

30. Method according to claim 1, **characterised in that** each primary probe consists of a polynucleotide or comprising a portion forming a polynucleotide of which the nucleotide sequence and the mass are known and are, for each type of primary probe, different to those of the polynucleotides of the other types of primary probe.

31. Device for performing the method according to any one of claims 14 to 22, **characterised in that** it comprises:
- an array of capillaries (D) for the circulation of primary probes,
- a grid of secondary probes organised in spots (K), the grid being arranged such that it is in contact with the array of capillaries,
- an array of electrodes, so-called functionalised (B), whereon the secondary probes are fixed, this array being arranged such that each row of spots of the grid of spots is fixed on one of the functionalised electrodes of said array, and
- an array of electrodes, so-called non-functionalised (G), this array being arranged such that the array of capillaries is situated between the two electrode arrays.

32. Device according to claim 31, **characterised in that** it is suitable for analysing the molecular targets by means of analysing the primary probes.

33. Device according to claim 32, **characterised in that** it is suitable for measuring the impedance variations associated with the hybridisation of the primary and secondary probes.

34. Device according to claim 32, **characterised in that** it is suitable for measuring by SPR the hybridisation of the primary and secondary probes.

35. Device according to claim 32, **characterised in that** it is suitable for measuring the polarised light variation induced by the formation of primary probe-secondary probe hybrids.

36. Device according to any one of claims 31 to 35, **characterised in that** the electrodes (B, G) are etched in a thin layer on an insulating material.

37. Device according to claim 31, **characterised in that** the set of functionalised electrodes (B) is situated above the array of capillaries (D) and **in that** the set of non-functionalised electrodes (G) is situated below the array of capillaries.

38. Device according to claim 31, **characterised in that** the set of functionalised electrodes (B) is situated below the array of capillaries (D) and **in that** the set of non-functionalised electrodes (G) is situated above the array of capillaries.

39. Device according to any one of claims 31 to 38, **characterised in that** each spot (K) of the functionalised electrodes is in each capillary of the array of capillaries (D).

40. Device according to any one of claims 31 to 38, **characterised in that** it further comprises a reservoir (F) at each end of the array of capillaries (D).

41. Device according to claim 40, **characterised in that** the reservoir (F) comprises an electrode, referred to as the reservoir electrode (C).

42. Device according to claim 41, **characterised in that** the reservoir electrode (C) contained in each reservoir (F) is situated in the same plane as the functionalised electrodes (B).

43. Device according to claim 41 or 42, **characterised in that** it further comprises a first and a second additional linking electrodes (A) situated respectively between the first reservoir electrode (F) and the first functionalised electrode (B), and between the second reservoir electrode and the last functionalised electrode, such that the shortest distances between each linking electrode and the corresponding reservoir electrode are identical at all points of the electrodes.

44. Device according to any one of claims 40 to 43, **characterised in that** the second reservoir is formed by at least one transverse channel, which is connected upstream to all the capillaries of the array of capillaries (D) and downstream to the detector.

45. Device according to any one of claims 40 to 44, **characterised in that** each reservoir (F) is hollowed in the thickness of a plate of a suitable material.

46. Device according to claim 31, **characterised in that** at least one of the electrode arrays (B, G) is formed by a grid of electrode row, column type.

47. Device according to claim 46, **characterised in that** the other electrode array is grounded.

48. Device according to any one of claims 31 to 47, **characterised in that** the functionalised electrode array (B) is formed by a grid of electrode row, column type, and comprises in each mesh of the grid at least one spot electrode (O) connected to one row electrode and to one column electrode of the grid via a field-effect transistor (FET).

49. Device according to claim 48, **characterised in that** the functionalised electrode array (B) and the non-functionalised electrode array (G) are formed by grids of electrode row, column type extending facing each other on either side of the array of capillaries, each grid comprising in each of its meshes a spot electrode (O) connected to one row electrode and to one column electrode of the grid via a field-effect transistor (FET).

50. Device according to claim 48, **characterised in that** each mesh of the grid comprises two spot electrodes (O) which are each connected to one row electrode and to one column electrode of the grid via a field-effect transistor (FET).

51. Device for implementing a method according to any one of claims 1 to 30, **characterised in that** it comprises:
- optionally a set of particles, for example magnetic particles, suitable for strongly binding with the nucleic acid and/or protein molecular targets of the mixture to be analysed;
- a set of primary probes of different types, in solution, said probes consisting of a polynucleotide or comprising a polynucleotide specific of each probe, each type of primary probe forming the set being able to bind by a specific bond a single type of molecular target to be analysed, when said primary probes and said molecular targets are contacted in solution, the nucleotide sequence of the polynucleotide of each type of primary probe being known and being different to the nucleotide sequences of the polynucleotides of the other types of primary probe, and if applicable,
- means for separating and means for retrieving the primary probes and the molecular targets bound by a specific bond, so as to obtain a set of primary probes representing an imprint of the molecular targets to be analysed, and/or if applicable,
- a set of secondary probes capable of specifically recognising the polynucleotide portions of the primary probes of the set of primary probes.

52. Device according to claim 51, **characterised in that** the means for dehybridising the primary probes and the molecular targets are capable of increasing the temperature.

53. Device according to claim 51 or 52, **characterised in that** it further comprises means for the quantitative analysis of the primary probes separated from the molecular targets, for example means according to claims 29 to 48.

54. Set of primary probes suitable for analysing molecular targets in a complex mixture, comprising a population of primary probes of different types, in solution, wherein:
- each type of primary probe is different to the other types of primary probes of the set of probes,
- each type of primary probe is able to bind, by a specific bond, with a single type of molecular target to be analysed, when said primary probes and said molecular targets are contacted,
- each type of primary probe (i) is a polynucleotide or (ii) comprises a polynucleotide associated with a polypeptide portion capable of binding specifically a single type of molecular target of the complex mixture, each polynucleotide having a known nucleotide sequence different to those of the polynucleotides of all the other primary probes of the set of probes, either by the sequence of the constituent nucleotides, or by its size, or by its mass, each of the polynucleotides being known respectively by its sequence, by its size thereof or by its mass, within the set of probes.

55. Analysis method according to any one of claims 1 to 30 **characterised in that** it comprises, prior to step a), steps for preparing the set of primary probes, consisting of:
- defining a size interval of the primary probes and defining hybridisation conditions of the probes with the targets and particularly the melting temperature Tm for all the probes,
- determining the number i of polynucleotide molecular targets having different sequences of the complex mixture,
- identifying, for each target sequence i potentially contained in the sample, a collection of Nᵢ possible probes that are perfectly hybridisable with the target in question and of which the size is within the selected size interval and the Tm is within the selected values,
- selecting within said collection of Nᵢ probes, a population of nᵢ polynucleotide sequences table to bind by hybridisation with the molecular target consisting of the sequence i in question and of which the bond thus formed is specific, hence excluding hybridisation with the other possible target sequences of the mixture,
- within this population of nᵢ putative probes for the probe composition, assigning to each polynucleotide a different mass to the mass of the other polynucleotides of the population, if applicable by modifying the nucleotides contained therein, for example by methylation or ethylation of one or a plurality of cytosine residues, to obtain a collection of polynucleotides all having a different mass known for each sequence, the comparison being repeated recursively at each sequence modification,
- identifying a collection of Zᵢ combinations of i primary probes for each sequence i, each of these combinations comprising a single primary probe of each population of n; probes under study; each of the primary probes of the population of nᵢ probes being compared to the primary probes of the combinations previously established, and when the masses of these primary probes are too close to one another, modifying their sequence, the comparison being repeated recursively at each sequence modification,
- classifying the Zᵢ combinations of i primary probes in increasing order of the sum of the masses of the i primary probes forming the combination and in increasing order of the number of modification groups,
- identifying a collection of Zᵢ combinations of i primary probes for the i sequences under study,
- synthesising the primary probes of the set of probes defined.
